# EUROPEAN PATENT SPECIFICATION

(11) **EP 3 513 341 B1**
(45) Date of publication and mention of the grant of the patent: **02.09.2026**
(21) Application number: 17783961.0
(22) Date of filing: 14.09.2017
(51) Int. Cl.: G16H 50/20, G16H 10/20, G16H 20/70, G16H 50/30

(54) **SYSTEM AND METHOD FOR DIAGNOSING AND TREATING HEADACHES**
SYSTEM UND VERFAHREN ZUR DIAGNOSE UND BEHANDLUNG VON KOPFSCHMERZEN
SYSTÈME ET PROCÉDÉ DE DIAGNOSTIC ET DE TRAITEMENT DE MAUX DE TÊTE

(30) Priority: 15.09.2016 GB 201615740
(43) Date of publication of application: 24.07.2019
(73) Proprietor: Matharu, Manjit Singh, London, Greater London N20 0XA (GB)
(72) Inventor: Matharu, Manjit Singh, London, Greater London N20 0XA (GB)
(74) Representative: Williams Powell
(86) International application number: PCT/GB2017/052722
(87) International publication number: WO 2018/051103

(56) References cited:
- US-A1- 2003 144 829
- US-A1- 2015 220 694
- R B LIPTON ET AL: "A self-administered screener for migraine in primary care: The ID Migraine validation study", NEUROLOGY, 12 August 2003 (2003-08-12), United States, pages 375 - 382, XP055429965, Retrieved from the Internet <URL:http://www.neurology.org/content/61/3/375.full.pdf#page=1&view=FitH> [retrieved on 20171129], DOI: 10.1212/01.WNL.0000078940.53438.83
- "Cephalalgia", vol. 33, 14 June 2013, SAGE PUBLICATIONS LTD., GB, ISSN: 0333-1024, article HEADACHE CLASSIFICATION COMMITTEE OF THE INTERNATIONAL HEADACHE SOCIETY (IHS): "The International Classification of Headache Disorders, 3rd edition (beta version)", pages: 629 - 808, XP055429896, DOI: 10.1177/0333102413485658

## Description

### Technical Field

The present invention relates to a system for diagnosing, managing and treating headaches.

### Background of the Invention

Headache disorders have a high prevalence and incidence. Headaches are one of the most common reasons for patients to consult with a doctor. The burden of headaches on patients, health services and society as a whole is extremely high. Current treatments are frequently ineffective, poorly tolerated, and remarkably variable in their success from one patient to another. The most common headache drugs have no more than a 40% chance of efficacy, with the result that a doctor has no real indication of whether or not any one drug will work. As a consequence, a patient tends to be treated on a trial and error basis until a solution is found. This can take months and in some circumstances years, during which time the patient continues to suffer. It is not possible for a doctor personally to gather enough data to be able to try to improve the selection of a suitable drug or treatment.

The reason for the difficulties lies in the fact that headaches are richly multifactorial. They can be influenced, for instance, by a myriad of genetic and environmental factors, including lifestyle factors (such as sleep, diet, exercise, work, rest, and so on) that a patient cannot convey synoptically to a doctor. It is therefore very difficult for both the patient and the doctor to know what factor or factors is/are decisive. The only practicable approach has been to change one factor or treatment at a time and observe the effect. Since the effectiveness of any manipulation requires many months to assess, headaches being sufficiently stochastic in their pattern, it can take years before the optimal lifestyle pattern or treatment is identified.

No conventional methodology has been able to address this state of affairs. Patients cannot rapidly discover their optimum by themselves, for it needs inevitably slow iterative changes to evaluate. Equally, a doctor cannot discover the optimum for any one patient from studies of single factors in other patients, for no single factor is ever decisive, it is nearly always a pattern of multiple factors that one has to consider.

To add to these problems, in many cases a patient is unable to convey the right symptoms to the doctor to enable the doctor to be able to assess the nature of the headache or the triggers that may have caused it. This is due in part by the fact that in many cases the patient does not visit a doctor during the course of a headache and by the fact that when in the course of suffering it is difficult for the patient to be able to think sufficiently lucidly to be able to understand the nature or possible causes of the headache.

There have been attempts to assist patients in recording the onset and possible triggers for headaches, typically in the form of diaries that a patient can use. These may include a set of questions the patient should answer, for later assessment by a doctor. While these devices can assist in data logging to some extent, they are necessarily limited by their format and use. In order to be able to provide a proper assessment, they would need to provide a very large number of questions for the patient to answer, but so doing would make them too onerous to complete and as a consequence of limited practical value.

Moreover, in some cases it is difficult for the patient to capture data of the symptoms suffered, particularly when these are severe and can impair the patient's abilities. In such circumstances, it can be difficult to capture important data for diagnosis and for determining the efficacy of a treatment or medicament.

Lipton et al. (2003; Neurology 61, 375-82) discloses a self-administered screener for migraine in primary care.

The Headache Classification Committee of the International Headache Society (2013; Cephalagia 33, 629-808) discloses The International Classification of Headache Disorders.

US 2015/0220694 A1 discloses a headache disease management system and method.

US 2003/0144829 A1 discloses a system and method for sensing and evaluating physiological parameters and modelling an adaptable predictive analysis for symptoms management.

### Summary of the Present Invention

The present invention is defined in the independent claims. The dependent claims define embodiments of the present invention.

The present invention seeks to provide an improved system for diagnosing managing and treating headaches.

According to an aspect of the present invention, there is provided a system for diagnosing and treating headaches as specified in claim 1.

Advantageously, the patient device is a computerised patient device. This may be, for example, a computer, a tablet, a smart telephone or other device having an input, a processor and an output display.

The generic characterisation module and/or the differential diagnosis module are preferably also included in the patient device.

A plurality of said algorithms may use the same input parameters from the generic characterisation module.

The at least one output module is operable to provide a patient indication of one or more of:
(i) headache history;
(ii) headache frequency;
(iii) treatment efficacy;
(iv) trigger frequency.

At least one of the patient indications is preferably in graphical form, for example as a bar or pie chart. At least one of the patient indications may be a graphical display of a person's head, subdivided into head and facial zones.

The patient indication is preferably limited over a time period, which time period is advantageously patient adjustable.

The system advantageously includes at least one sensing device operable to sense one or more patient physiological parameters, such as:
(i) number of steps walked,
(ii) steps climbed,
(iii) distance travelled,
(iv) calories burned,
(v) active time,
(vi) heart rate,
(vii) sleep time,
(viii) sleep quality,
(ix) levels of phone use,
(x) sound levels,
(xi) proportion of time spent indoors versus outdoors,
(xii) blood pressure,
(xiii) breath rate,
(xiv) mood sensor,
(xv) fatigue sensor,
(xvi) oxygen levels,
(xvii) blood sugar levels,
(xvii) temperature,
(xviii) alcohol levels in blood.

The sensing device and type specific module(s) are preferably arranged to communicate with one another automatically. The sensing device includes an attachment element, for instance in the form of a wrist strap or bracelet.

The system may also include a generic headache log module for logging and processing data relating to a non-specific headache type. It may also include a diary log module allowing a patient to input headache symptoms on time period basis.

The system advantageously provides for a patient to select one or more log modules for inputting and analysing headache symptoms. The system is preferably also operable to export data from one log module to another log module for analysis and display of data associated with that another log module.

The system preferably includes at least one graphical user input for inputting data into the log modules. The graphical user input includes at least a graphical display of a person's head, subdivided into head and facial zones.

### Brief Description of the Drawings

Embodiments of the present invention are described below, by way of example only, with reference to the accompanying drawings, in which:
Figure 1 is a schematic diagram of an embodiment of apparatus for implementing the system disclosed herein;
Figures 2 to 32 show various graphical user interfaces produced during the operation of the preferred system disclosed herein; and
Figures 33 to 72 show various graphical user interfaces produced during the operation of the preferred system implemented on a small screen device such as a smart telephone or table.

### Description of the Preferred Embodiments

The embodiments of system disclosed herein have the aim of improving the logging, diagnosis and treatment of headaches and in particular with the intention of reducing the time taken to identify effective treatments, of assisting the patient in understanding the causes and remedies and also of monitoring the history of headaches in order to seek to identify any changes in occurrence, severity or nature that may indicate a possible alternative or additional resolution path.

As will become apparent, the disclosed system can provide enhanced and real time data logging, not possible with prior art systems, as well as patient feedback.

The preferred practical implementations are able to gather substantial amounts of information about patients with headache disorders and then automatically mine that information using non-reductive, high-dimensional multivariate models preferably using machine learning so as to discover patterns of multiple factors that lead to exacerbation or recovery in any individual patient.

Aspects of the present invention relate to a system which provides a user input mechanism able to assist a patient and in particular able to capture substantial detail of a patient's symptoms even when the patient is suffering or temporarily debilitated.

A patient's health can be determined by inherited genetic differences combined with lifestyle and other environmental factors. By combining and analysing information about a patient's genomes with clinical and diagnostic information, and then comparing that with data from others, patterns can be identified. Together, this information can help to determine a patient's individual risk of developing disease, detect illness earlier, provide an accurate diagnosis, and determine the most effective interventions to help improve a patient's health, be lifestyle choices, medicines or other therapeutic interventions.

The preferred system disclosed herein can bring together new approaches such as data and informatics, genome sequencing and imaging data, as well as wearable technology. The interconnections between these factors makes it possible to provide personalised care and in particular can assist in faster and more effective short and long term treatment of recurring headaches.

An element of the system consists of a clinical registry and biorepository with the following elements:
(1) a knowledge database comprising a collection of data from large cohorts of patients with headache disorders in order to improve knowledge about the various aspects of these disorders, preferably including demographics, epidemiology, clinical symptoms and signs and diagnostic pathways, as well as impact of lifestyle factors, and efficacy/adverse effects of treatments used. This can also provide for follow up of patients so as to provide information on long-term prognosis of various headache syndromes;
(2) a diagnosis module able to provide more precise diagnosis, specifically based on knowledge of each individual's complex genetic and molecular profile and informed by clinical and diagnostic information enabling more precise diagnoses to be made. The system is able to reduce diagnostic heterogeneity. Specifically, there is currently considerable diagnostic heterogeneity with significant proportions of patients receiving imprecise diagnoses. Diagnostic homogeneity is enabled by utilising diagnostic algorithms that are then applied uniformly across the patient population;
(3) improved treatment regimens and in particular the ability to collect data on outcomes of various therapies used and provide for comparison between multiple treatments. The system can also provide intelligent treatment pathways informed by individualised assessment of clinical effectiveness and adverse drug reactions, resulting in improved clinical outcomes;
(4) improved healthcare impact by providing data on impact of headache disorders on generic and disease specific health-related quality of life (HRQoL), data on cost-effectiveness of different treatments, information on utility and cost-effectiveness of diagnostic tests, assessment of adherence to diagnostic and treatment guidelines, as well as efficacy and sustainability of service delivery;
(5) providing an improved research tool, in particular:
   (a) improved diagnostic criteria in which phenotypic data can be tested to ascertain the value of individual International Classification of Headache Disorders (ICHD) diagnostic criteria and inform future revisions of the classification to allow for more accurate diagnosis;
   (b) targeted headache disorder prevention: identify predisposing markers or underlying processes to predict future development or worsening of headache disorders and thereby prevent this with appropriate interventions;
   (c) identification of factors that predict progression, regression and treatment response;
   (d) assessment of impact of co-morbidities and endophenotypes on outcomes;
   (e) creation of a biorepository linked to each patient and identify biomarkers (including genetic and metabolic markers) that impact on outcomes. Epigenetic data, genetic data, biochemical data and gene expression data will enable investigators to identify gene mutations and variants, and epigenetic profiles for diagnostic purposes. Researchers will also be able to use the data to increase molecular insight into pathophysiologic mechanisms of headaches that will guide the design of novel treatment options;
   (f) neuroimaging data, which can also will play a role. Investigators hope to use neuroimaging data to compare brain structure and function among patients with different headache types and to correlate imaging findings with phenotypic and genetic data. They also plan to develop and evaluate mathematical models of imaging data that best differentiate between headache types.

Practical embodiments of the system can assist patients with headache disorders. The preferred apparatus in effect crowd-sources rich, dynamic data from patients with headaches, not just by manual input but also automatically using mobile device sensors, as well as their clinical team and electronic health records to generate headache diagnoses and personalised lifestyle advice, including individually-tailored treatment plans through machine-learning analysis of the populated data. The system can serve as self-monitoring and lifestyle guidance tool (patient), an aid to clinical management (doctors), and a data repository of invaluable rich data (healthcare organisations, clinical research, and industry).

It is to be understood that the examples which follow are indicative only of embodiments of the invention.

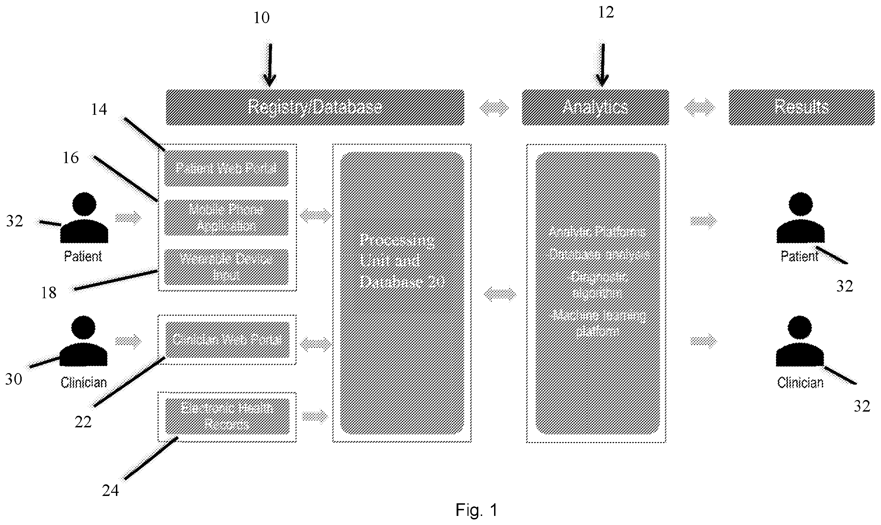

Referring first to Figure 1, this shows schematically the principal components of a preferred embodiment of system. The system comprises two primary sections, the first being a data logging and analysis section 10 which can be accessed both by the patient and by the clinician (for example a General Practitioner or Consultant). The second section is an analytics section 12 designed to collate together data from large numbers of patients and to correlate that data with common patient characteristics such as, for instance, ethnicity, genomics, lifestyle, other illnesses or disorders and so on. The analytics section 12 enables the system to modify diagnoses and treatments on the basis of historical learning in a manner that is not possible by a single doctor or group of doctors. The analytics section 12 will typically comprise the knowledge database and the diagnostic module mentioned above, although it is not excluded that either or both of these may reside in the analysis section 10 and in particular the processing unit 20.

In practice, the analytics section 12 will comprise at least one processing unit and at least one database, as well as inputs and outputs for receiving data from multiple patient and clinician sources and for transferring data back. The section 12 may be in the form of one or more data processors and servers and may be managed by a remote management unit or facility. A person with ordinary skill in the art will be knowledgeable of the components of hardware and set-up that are appropriate and suitable for the tasks performed by the analytics section 12, this being common practice in the art.

As described in further detail below, the analytics section 12 and/or the registry section 10, specifically the processing unit 20, will include the generic characterisation module, the differential diagnosis module and the type-specific log modules. The units 14 and 16 at the patient side comprise the output module described herein.

Considered important in the preferred embodiments is the facility that both the clinician 30 and the patient 32 are presented with the results of the analyses carried out by the system, which enables both the patient 32 and the clinician 30 to understand better the dynamics of the headache, in particular possible triggers, efficacy of treatments over fixed or variable time periods, thereby facilitating a faster solution or cure.

The data logging and analysis section 10 includes one or more of a personal computer 14, a mobile device 16 such as telephone or tablet, and optionally a sensing device such as a wearable bracelet 18. These devices 14 to 18 communicate preferably bi-directionally with a processing unit 20, which may be at the patient side, for instance in the computer 14 or mobile device 16, or may be located remotely at a remote server or at a clinical facility of the clinician 30.

The wearable device 18 may be any of a variety of data logging devices commonly known and typically used for monitoring a person's fitness activity. It may take any suitable form and have the associated sensor componentry, all of which are within the ordinary ability of the skilled person given devices of this type are commonplace. In some instances, instead of being a bracelet-type device, the sensing device 18 may be in the form of a necklace, an adhesive patch and so on.

In the preferred embodiments, the sensing device is configured and structured to sense one or more patient physiological parameters, such as: (i) number of steps walked, (ii) steps climbed, (iii) distance travelled, (iv) calories burned, (v) active time, (vi) heart rate, (vii) sleep time, (viii) sleep quality, (ix) levels of phone use, (x) sound levels, (xi) proportion of time spent indoors versus outdoors, (xii) blood pressure, (xiii) breath rate, (xiv) mood sensor, (xv) fatigue sensor, (xvi) oxygen levels, (xvii) blood sugar levels, (xvii) temperature and/or (xviii) alcohol levels in blood.

The system is preferably configured to predict when the patient is due to have the next attack on the basis of the inputs received from the sensing device during previous attacks, for instance by detecting a predictable pattern for the various inputs through the sensing device. When that pattern is being repeated, the system is advantageously configured to alert the patient that an attack is imminent. The patient can then take appropriate action to avert the attack.

The sensing device and type specific module(s) are preferably arranged to communicate with one another automatically. The sensing device includes an attachment element, for instance in the form of a wrist strap or bracelet.

The clinician 30 is provided with a portal device, which may be a computer 22 or other similar data processor, and also with a database 24 of patient health records for storing general patient data, at least some of which may be used by the system disclosed herein, and also for logging the data obtained from the patient 32 and the results of the data and treatment analyses carried out by the system.

The analytics section 12 is able to obtain data from the processing unit 20 and also to feed data back to the patient devices 14, 16 as well as to the clinician's devices 22, 24.

In practice, in the preferred embodiment, data can be entered into the registry, that is the processing unit 20, from various sources. Data can be collected from the patient via the patient website portal 14, the mobile device 16 and/or the wearable device 18. As described in detail below, this preferably involves a first stage in which a patient is asked to complete a comprehensive questionnaire when initially registering on the system. This may subsequently be followed up with further questionnaires completed every 3 to 6 months or as required by the clinician. Ongoing, a patient may keep a headache diary, thereby providing data on the frequency, severity and duration of headaches as well as the presence of any associated symptoms and triggers. Data can also be captured automatically from the wearable device 18 and this may include, for example: activity (number of steps walked, steps climbed, distance travelled, calories burned, active time), heart rate, sleep time and quality, levels of phone use, sound levels, fraction of time spent indoors versus outdoors (via GPS), and so on.

Moreover, the clinician 30 is preferably able to enter data into the processing unit 20 via the clinician website portal 22. Electronic health records 24 held at participating centres can also be accessed for pertinent information and populated into the registry at the processing unit 20, as well as into the analytics section 12.

Systematic and standardised collection of comprehensive data provides an unprecedented resource to advance knowledge for the improvement of diagnosis and treatment.

The table that follows shows the preferred data domains and data elements of the preferred system and database.

**Table 1**

| **DOMAIN** | **DATA ELEMENTS** | |
|---|---|---|
| Personal details and demographic data | Age and Date of birth | |
| | Gender | |
| | Handedness | |
| | Marital status | |
| | Sexual orientation | |
| | Ethnicity | |
| | Race category | |
| | Number of years of education | |
| | Occupation | |
| | Employment status | |
| | Primary language | |
| | Country of birth | |
| Headache and facial pain history | Onset and progression | |
| | Duration and frequency | |
| | Severity and characteristics of pain | |
| | Side and location of pain | |
| | Associated symptoms | |
| | Aura symptoms | |
| | Triggers and relieving factors | |
| | Timing of attacks | |
| | Diagnosis offered by health professionals | |
| Treatments | Medication history | |
| | Injectable treatments and infusions | |
| | Neurostimulators | |
| Medical history | Cancer, cardiovascular, cerebrovascular, gastrointestinal, endocrine, genitourinary, abuse, allergic/immunologic, musculoskeletal, neurological, psychiatric, substance abuse, sleep and other comorbid disorders | |
| Family history | Cancer, cardiovascular, cerebrovascular, gastrointestinal, endocrine, genitourinary, abuse, allergic/immunologic, musculoskeletal, neurological, psychiatric, substance abuse, sleep and other comorbid disorders | |
| Social and behavioural history | Smoking history | |
| | Alcohol history | |
| | Substance use history | |
| | Diet and exercise history | |
| Physical examination findings | | |
| Biobank specimen collection | Blood: specify type: platelet-rich plasma, platelet-free plasma, serum | |
| | Cerebrospinal fluid (CSF) | |
| | Urine | |
| | Saliva | |
| | Other | |
| Investigation results | Blood tests | |
| | Cerebrospinal fluid (CSF) analysis | |
| | Genetic testing | Gene-specific test |
| | | Genome-wide association study (GWAS) array |
| | | Whole Exome sequencing |
| | | Whole Genome sequencing |
| | Neuroimaging Study | Computed Tomography (CT) scan |
| | | Magnetic resonance imaging (MRI) scan |
| | | Functional MRI scan |
| | | Magnetic resonance angiography (MRA) |
| | | Magnetic resonance spectroscopy (MRS) |
| | | Positron emission tomography (PET) |
| | Electrophysiology studies | Electroencephalogram (EEG) |
| | | Trigeminal and facial nerve studies |
| | | Nociceptive blink reflex |
| | | Pain-related evoked potentials |
| | | Quantitative sensory testing |
| | | Transcranial magnetic stimulation |
| | | Magnetoencephalography |
| Headache diaries/calendars | | |
| Wearable device data | Activity levels: | Number of steps walked, steps climbed, distance travelled, calories burned, active time |
| | Heart rate | |
| | Sleep time and quality | |
| | Levels of phone use | |
| | Sound levels | |
| | Fraction of time spent indoors vs outdoors (via GPS) | |
| | Blood pressure | |
| | Breath rate | |
| | Mood sensor | |
| | Fatigue sensor | |
| | Oxygen levels | |
| | Blood sugar levels | |
| | Temperature | |
| | Alcohol levels in blood | |
| Diagnoses | Headache Pro Algorithm diagnosis | |
| | Clinician Diagnosis | |
| Outcome measures | Headache screening scores | ID Migraine |
| | Affective scores | Perceived Stress Scale (PSS) |
| | | Beck Anxiety Inventory (BAI) |
| | | Beck Depression Inventory (BDI-II) |
| | | Generalised Anxiety Disorder (GAD-7) |
| | | Hospital Anxiety and Depression Scale (HADS) |
| | | Patient Health Questionnaire-4 (PHQ-4) |
| | | Patient Health Questionnaire-9 (PHQ-9) |
| | | Depression Scale |
| | Assessing Comorbidities | Pittsburgh Sleep Quality Index (PSQI) |
| | | Mini International Neuropsychiatric |
| | | Interview (MINI) |
| | | Mood Disorder Questionnaire (MDQ) |
| | Patient-Reported Outcomes | Patient Global Impression of Change Scale |
| | Activities of Daily | Functional Impairment Scale |
| | Living/Performance | Health Needs Assessment Survey (HANA) |
| | | Migraine Disability Assessment Test (MIDAS) |
| | | Migraine Functional Impact |
| | | Questionnaire (MFIQ) |
| | | Migraine Interictal Burden Scale (MIBS) |
| | | Migraine Physical Function Impact |
| | | Diary (MPFID) |
| | Quality of Life | 24-hour Migraine Quality of Life |
| | | Questionnaire (24-Hr-MQoLQ) |
| | | Clinical Global Impression |
| | | EuroQoL |
| | | Headache Impact Questionnaire (HImQ) |
| | | Headache Impact Test-6 (HIT-6) |
| | | Health Related Quality of Life-14 (HRQOL-14) |
| | | Migraine specific Quality of Life |
| | | Questionnaire (MsQoL v2.1) |
| | | Quality of Life in Neurological Disorders (Neuro-QOL) |
| | | Short Form-12 Item Health Survey (SF-12) |
| | | Short Form-36 Item Health Survey (SF-36) |

The analytical platform 12 is designed to build high-dimensional models using machine-learning techniques from the data it receives and to send back predictions to the clinician 30. It can specifically isolate lifestyle components and therapies, allowing it to identify the subgroups in whom any particular therapy is most effective. The analytic platform 12 will begin with base assumptions that are set up by existing knowledge of the literature and changes them as more information is gathered from patients 32.

In accordance with Table 1 above, using the system preferably commences with what could be described as a generic characterisation module of which an embodiment is set out in Appendix 1 below. In this module, typically performed during an initial registration procedure and preferably updated at regular intervals such as six monthly or so, the patient is asked to provide a series of answers to questions ranging from personal details, generic headache factors including frequency and symptoms, possible triggers and relieving factors, previous treatments and medications, family history and social factors. The questions in this module are intended to be answered when the patient is free or substantially free of a headache and is more of a data gathering and analysis stage. The purpose is to collect data relating to the patient for future use, correlation with patients having similar characteristics such as ethnicity or environment, and also to generate at least one differential diagnosis of the patient's headaches (see detailed description below and the Summary Section in Appendix 1). The differential diagnosis is provided to the patient and advantageously includes a plurality of diagnoses, that is headache or potential headache type. This has the advantage of allowing the patient to consider the results of the differential diagnosis before visiting a doctor, which can assist in reaching a diagnostic conclusion faster and also early consideration of potential triggers and treatment efficacies.

The intention is that following the generic characterisation stage, the patient visits his/her doctor (clinician) to confirm the correct or most likely correct headache type. The doctor has the benefit of the differential diagnosis, which is based not only upon the patient's own results but also correlation with the database of records of other patients stored in the analytics section 12 and thus learning from other cases that would not be otherwise available to the doctor. On review by the doctor, one or more of the differential diagnoses may be confirmed or otherwise the doctor may decide the patient is suffering from a different headache type, in which case the doctor informs the system via the clinician's computer 22. That alternative diagnosis can then be assessed within the system 10/12.

The differential diagnosis also allows the patient to commence logging future headaches via one or more of the patient-side devices 14, 16, in the manner described in detail below.

The differential diagnosis is determined on the basis of an algorithm of which the current preferred embodiment is shown in Appendix 2 at the end of this description. As will be apparent from Appendix 2, the algorithm includes a plurality of processing modules in which data collected from the generic characterisation stage is analysed on the basis of a series of predetermined criteria, each set being associated with a particular headache type. In Annex 2 there are listed algorithms for identifying the following headache types and triggers:
migraine,
tension type headache,
probable migraine,
probable tension type headache,
new daily persistent headache,
Hemicrania Continua,
nummular headache,
cluster headache,
Paroxysmal Hemicrania,
short-lasting unilateral neuralgiform headache attacks,
Hypnic headache,
Valsalva-Manoeuvre headache,
exercise headache,
sex headache,
Trigeminal neuralgia,
primary stabbing headache,
high CSF pressure,
low CSF pressure,
Cervicogenic headache, and
medication overuse headache.

It is to be understood that these are examples only and that the algorithm collection of Appendix 2 could be altered to add further headache types and also to remove headache types that become relevant, for instance as a result of medical progress or re-characterisation.

As will be appreciated, each algorithm harvests from the generic characterisation module data deemed relevant to that headache type and applies a scoring to the results, generating a final value indicative of whether or not that headache type is exhibited by the patient's symptoms as set out in the general characterisation answers. Once the processing unit 20 works through the set of algorithms, there will typically be a plurality of positive outcomes, that is a plurality of possible headache types. This is intentional and built-in as a factor in the algorithms. The advantage of doing this is that the final diagnosis is left for the clinician to determine, and it also permits a patient to be diagnosed with a plurality of headache types, not currently done, which enables more effective monitoring and treatment while removing to a large extent the trial and error approach conventionally used. For instance, if on consultation with a doctor it is concluded that a patient is exhibiting symptoms of a plurality of headache types, the patient can continue to be monitored and treated on the basis of that plurality, which is possible by means of the system taught herein and explained in further detail below. As a result, it is not necessary for the clinician to select just one course of action for treatment that may ultimately be ineffective, a factor which may lie behind the low efficacy rates of medicaments as they are currently prescribed.

The system and in particular the analytics section 12 is designed to receive and analyse the results of patient diagnosis, both the differential diagnosis produced by the system and also the clinician's diagnosis, and to correlate these results with existing data from the patient group. This can be used in a plurality of ways. Of particular advantage is the ability of the analytics module 12 to reconfigure the algorithms of Appendix 2 on the basis of knowledge acquired over time.

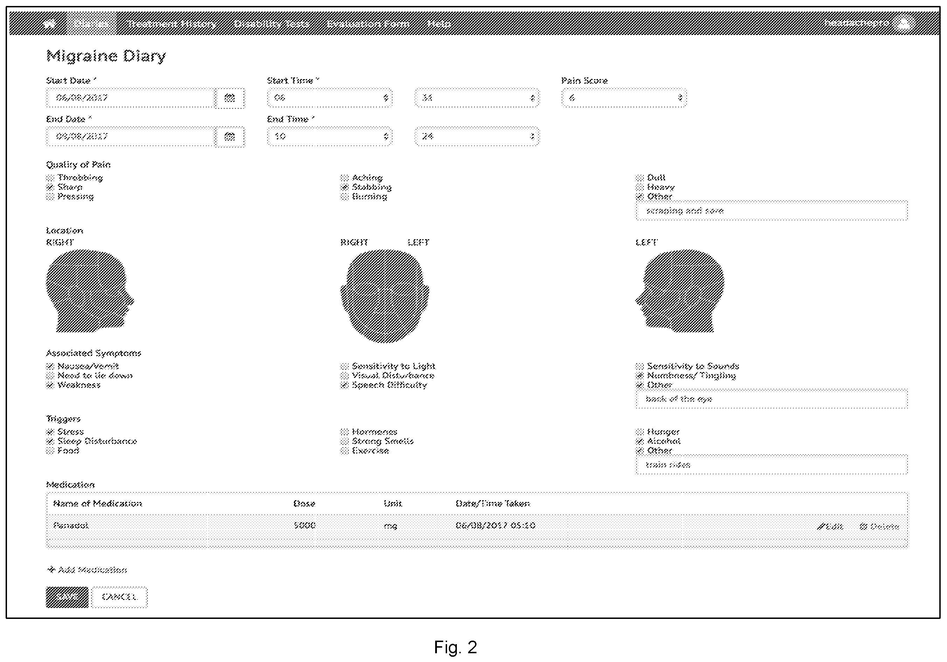

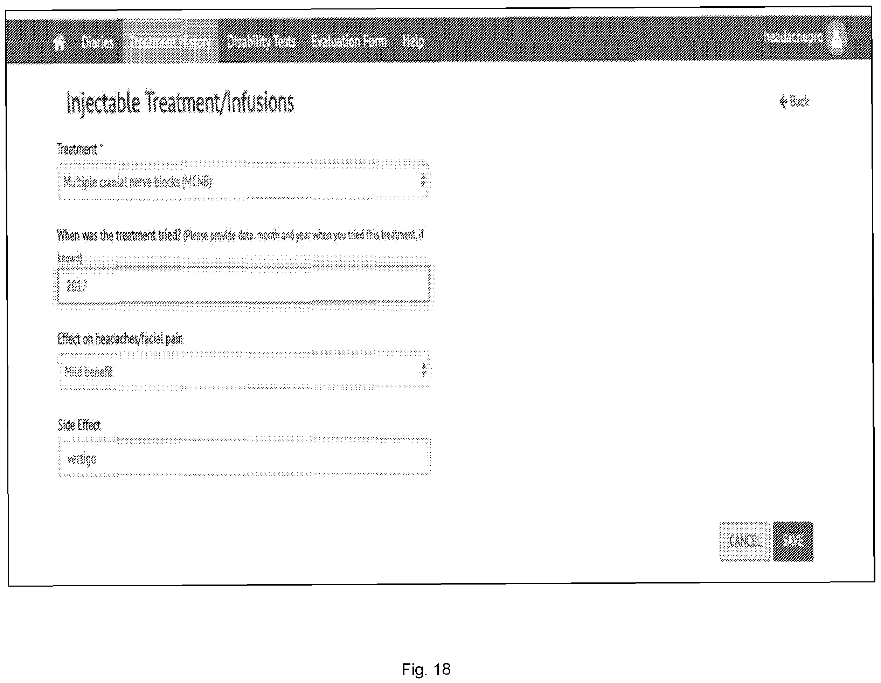

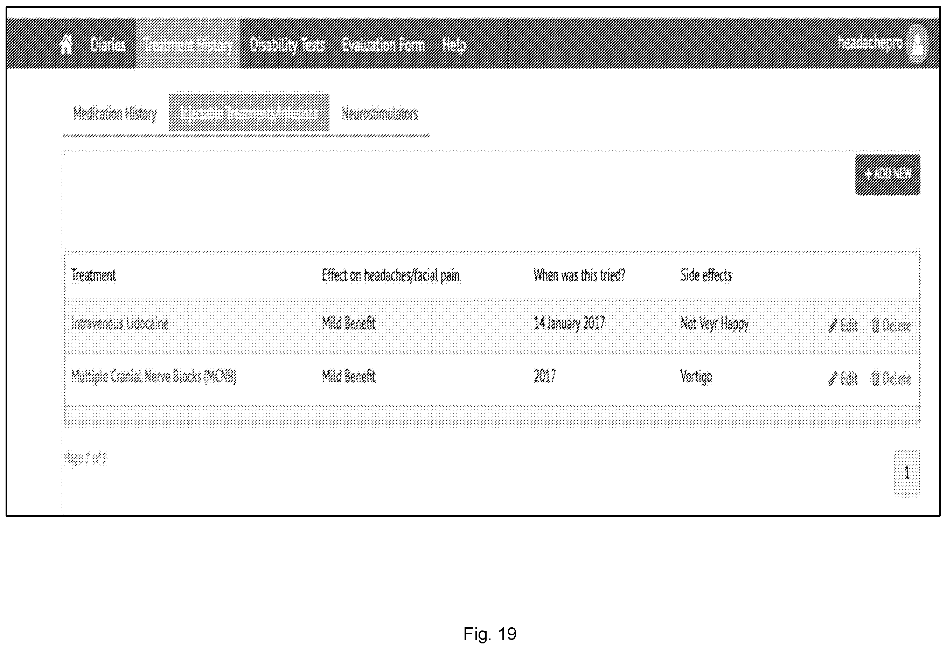

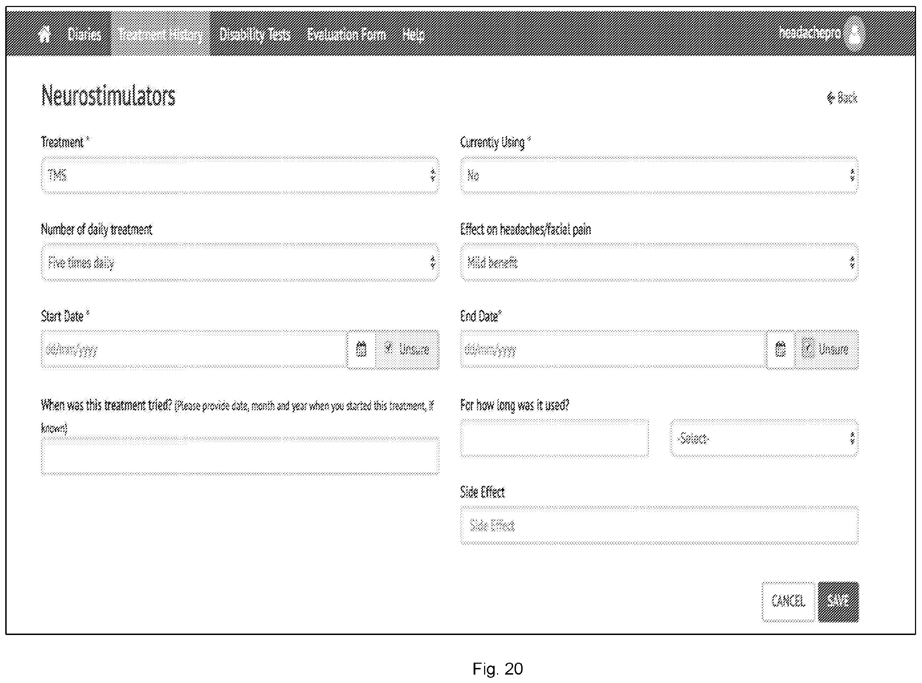

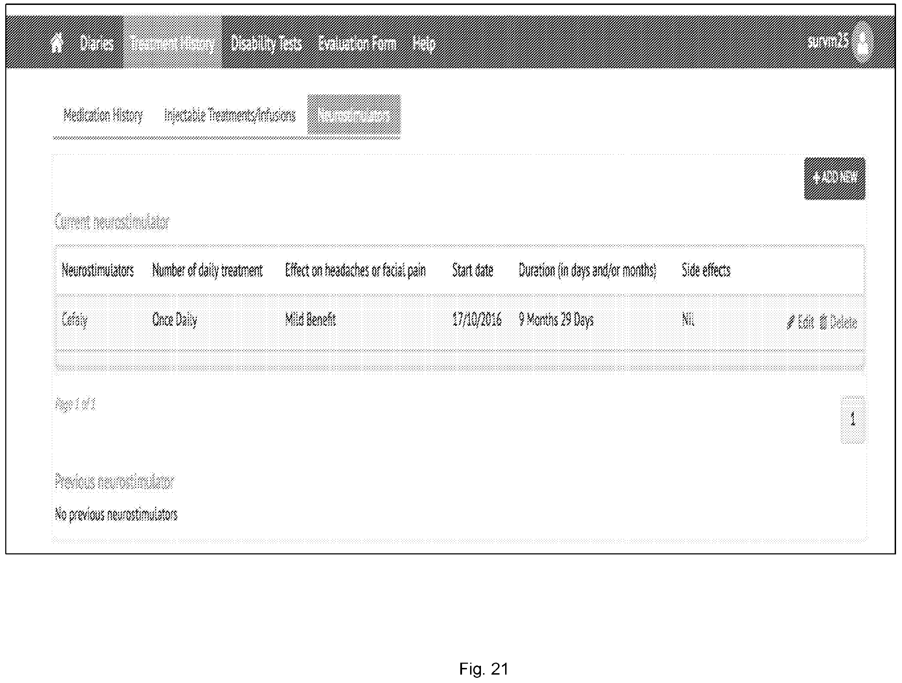

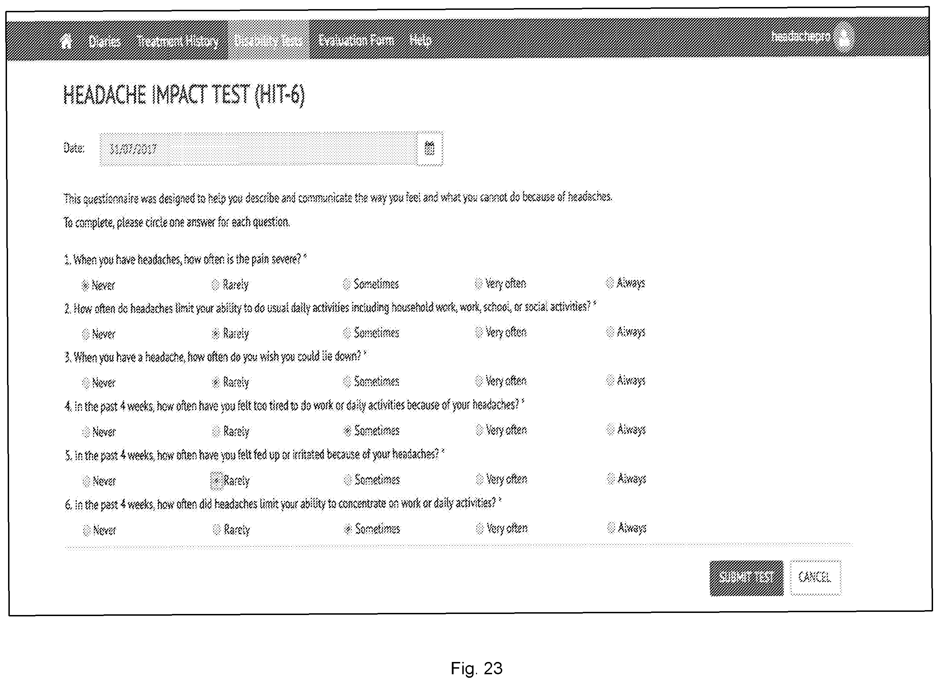

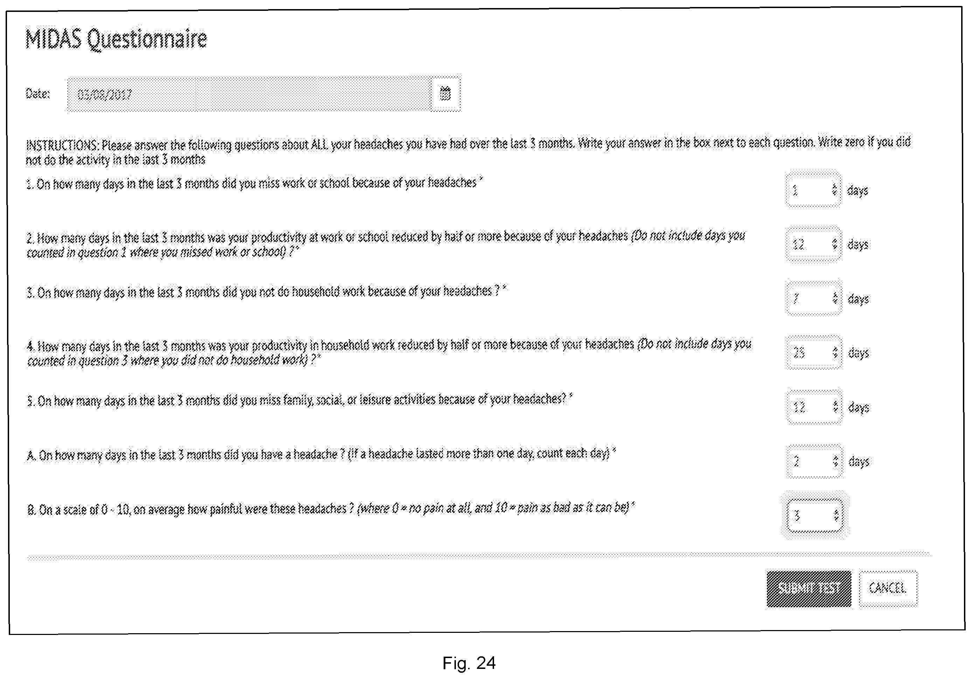

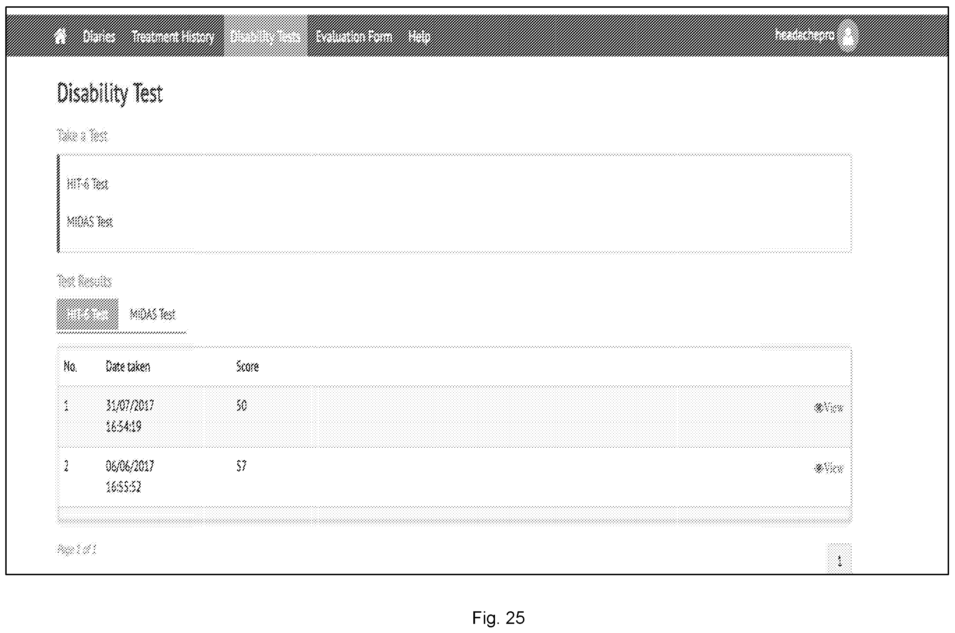

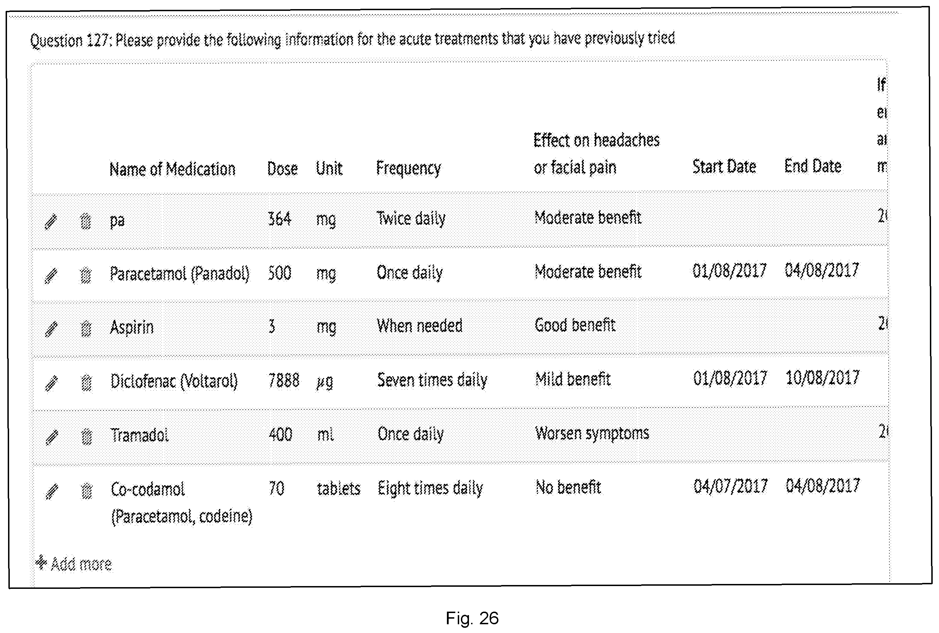

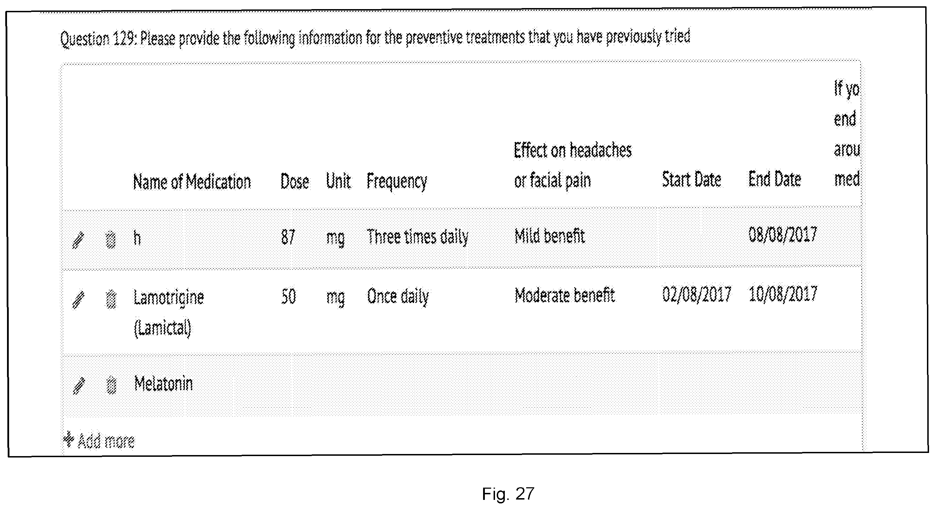

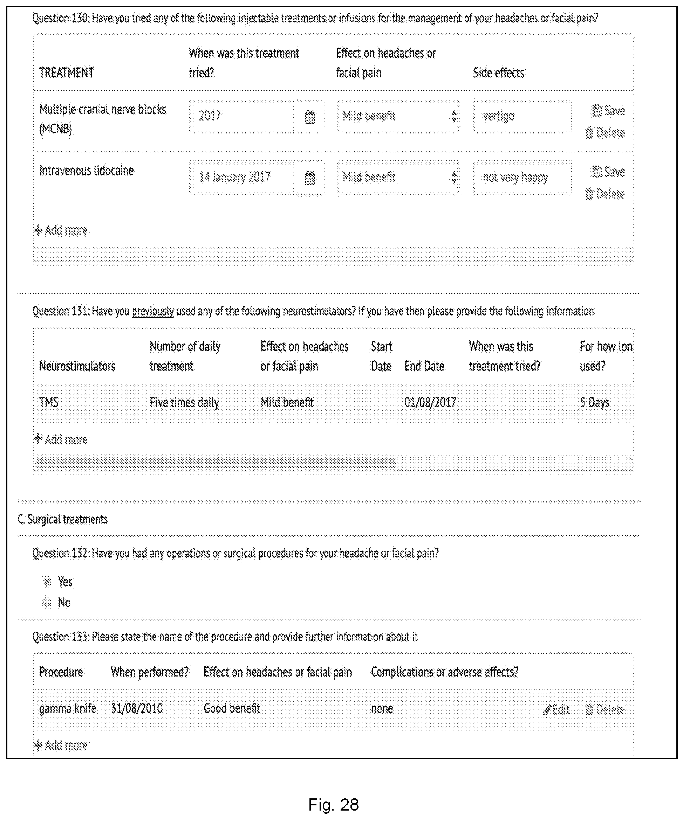

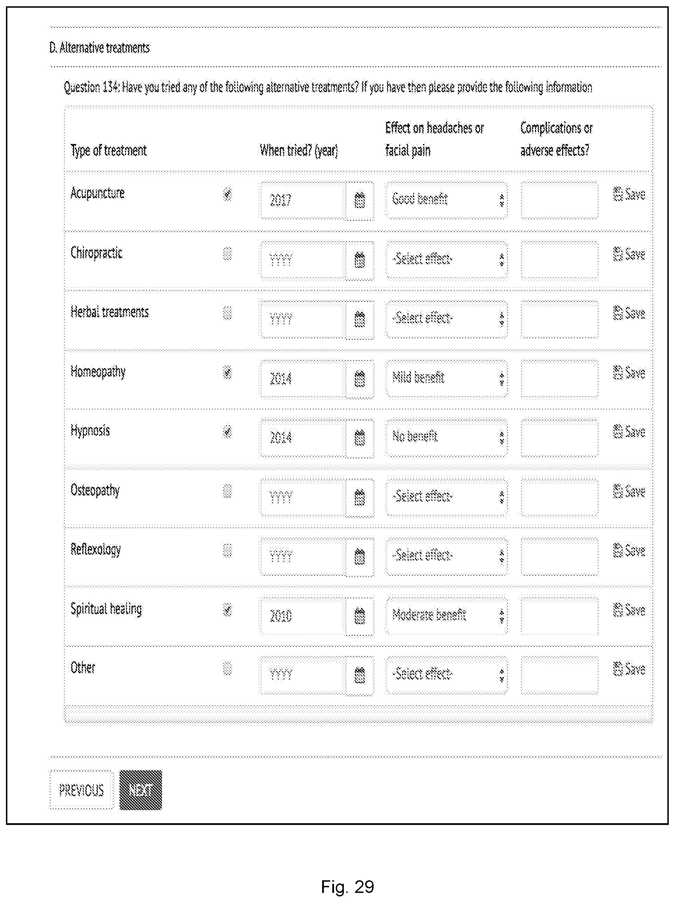

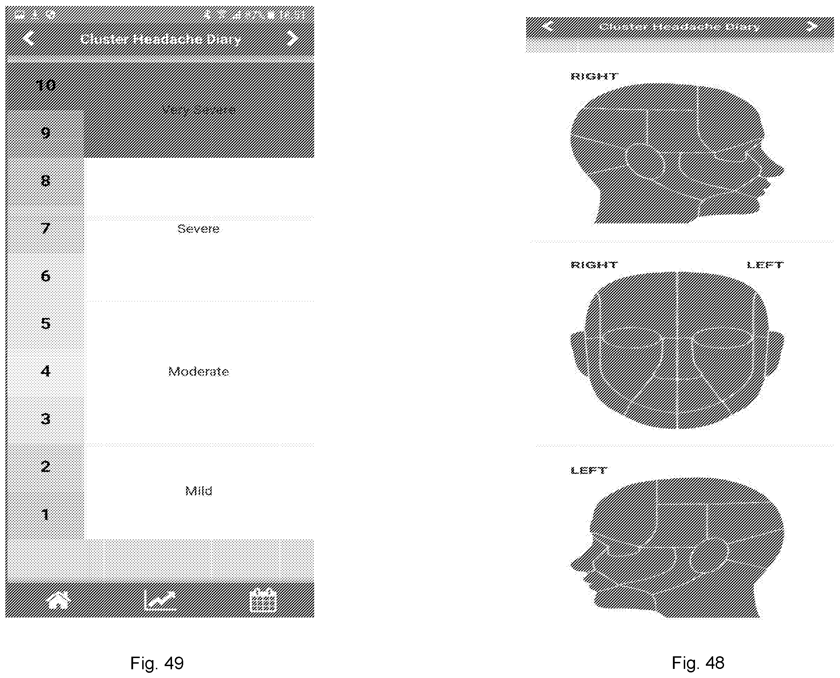

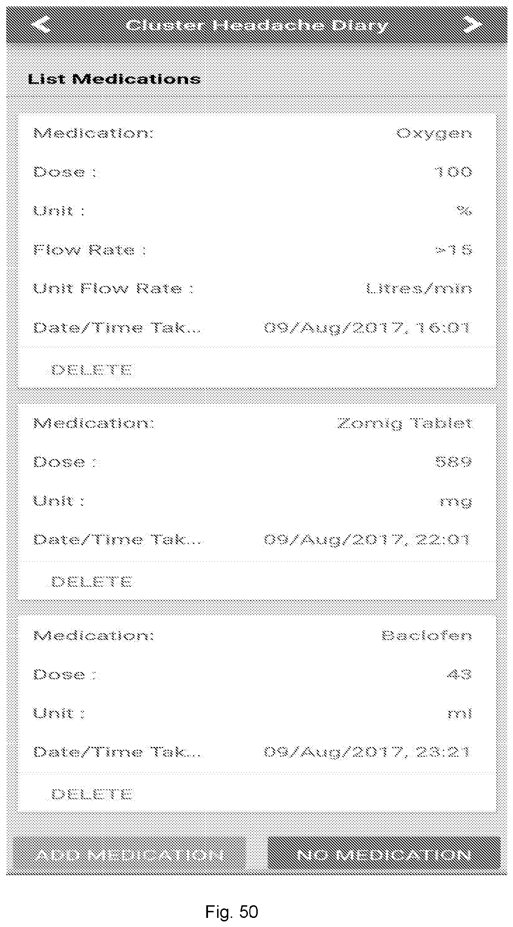

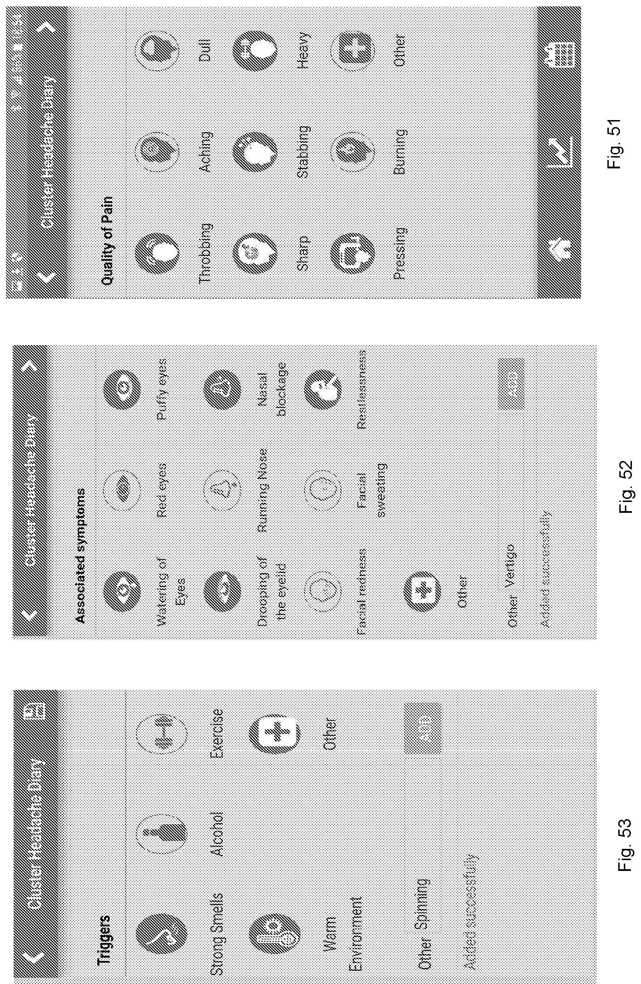

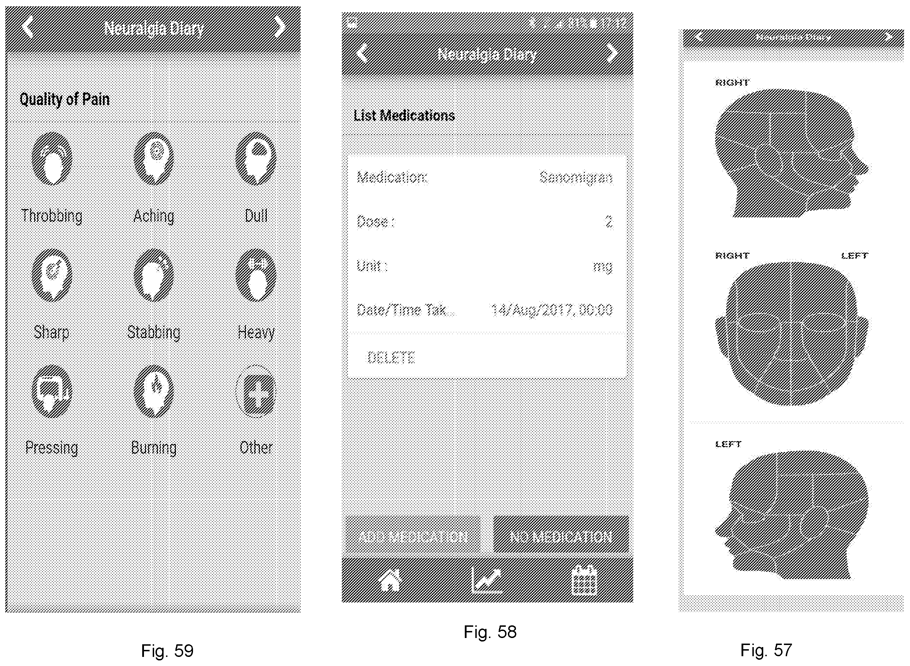

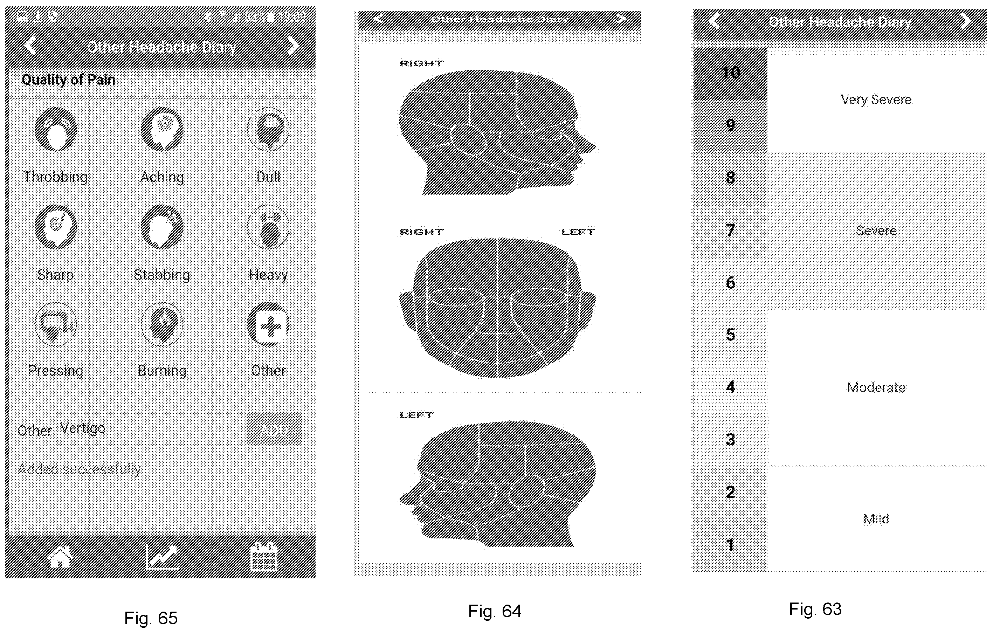

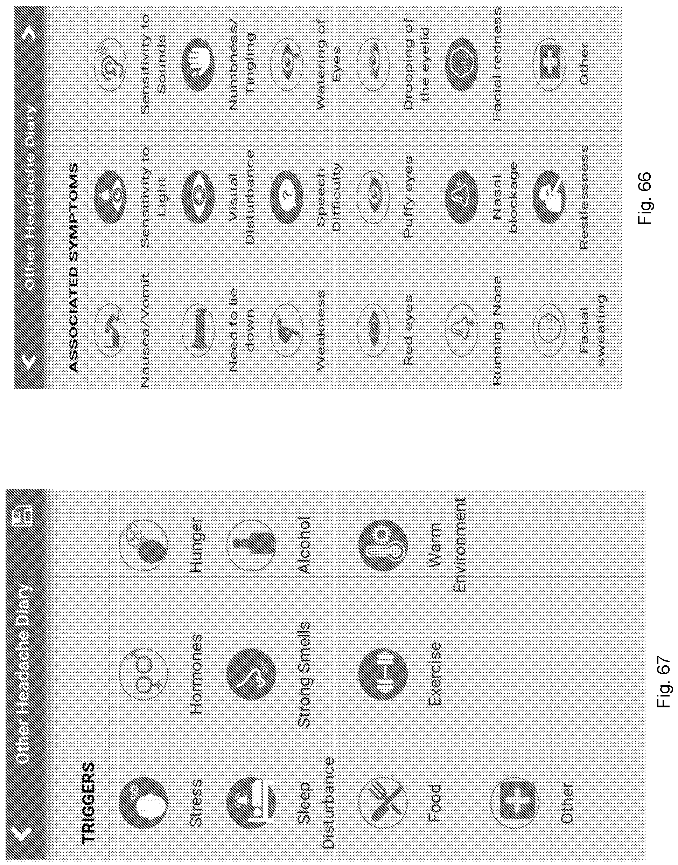

Embodiments of the headache specific log modules and the input/output modules are now described in connection with Figures 2 to 32 and Figures 33 to 73.

With reference first to Figure 2, this shows a view of a patient interface at the patient device. This interface is presented to the user subsequent to the generic data input, characterisation, differential diagnosis and preferably, but not necessarily, also confirmed diagnosis by a clinician. In other words, the patient 32 will have registered with the system and completed the initial assessment and diagnosis. At this stage, the patient is offered one or more headache diaries accessible from a drop-down menu, Figure 2 showing the selection of a Migraine Diary. The patient can by selection add the Migraine Diary to the patient system 20. This diary includes entry fields specific to migraines and in this embodiment comprises fields for entering timing of a migraine, severity (on a scale), quality of nature of the pain, location of the pain, associated symptoms, possible triggers and whether any medication has been taken.

The data in each of these fields is selected specifically for that headache type, in this example migraines, and is provided by easy tick boxes. For the location of the pain, this is provided by a series of head views that the patient can select and highlight with a cursor or finger. The page is used to record all the detail of the headache. Multiple medications can be added, with individual times specified for each medication. Once completed, the patient saves the inputted information and is returned to the main Diary page.

It will be apparent that the user interface is simple to use and, by selection of the appropriate fields based on headache type, comprehensive. It allows the user to log the headache in real time and simply, particularly relevant as the patient may be debilitated and/or distressed at the time.

Some of the triggers for this diary, and equally applicable to all the other diaries disclosed and contemplated herein, may be provided automatically from a sensor device 18. For instance, sleep disturbance and exercise can readily be logged by such devices, as can many other types of data including, for example, temperature, pulse rate, oxygen or carbon dioxide content in the blood, levels of hydration and so on. Ultimately, any data able to be collected by the sensor unit 18 and useful for headache monitoring or analysis may be used in the taught system.

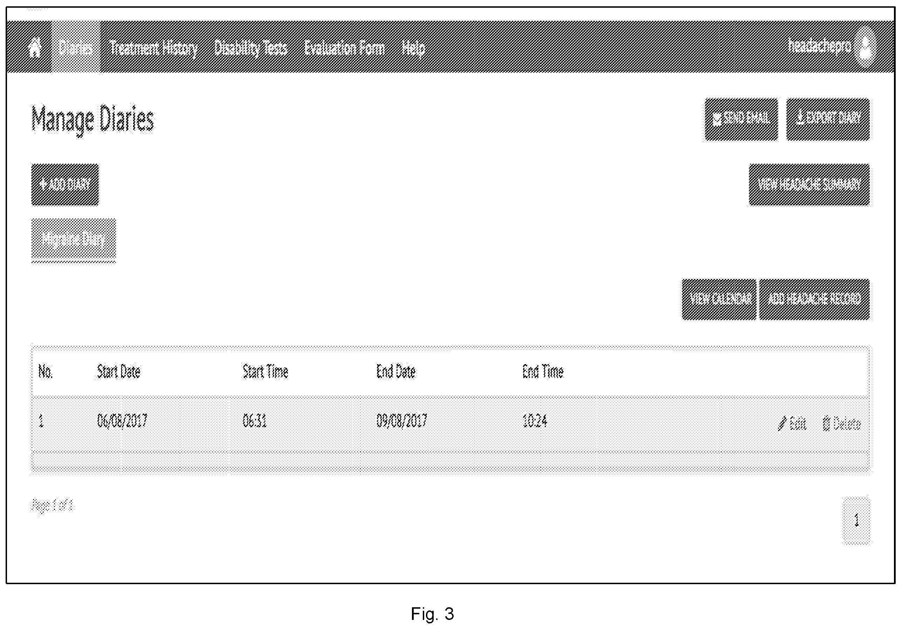

Referring to Figure 3, after completion of a diary entry and saving this to the processing unit 20, the diary record remains accessible together with any earlier completed and saved diaries. The user is provided with the ability to send the data of a headache event, for instance to a clinician 30 by email, for which the diary data may be converted to any suitable format including as a spread sheet. Not shown in the drawings is the option of a calendar view showing days of the week or of the month with the date(s) on which headache events have been recorded highlighted. The highlighting may be by colour and it is envisaged that different colours could be used for different headache types.

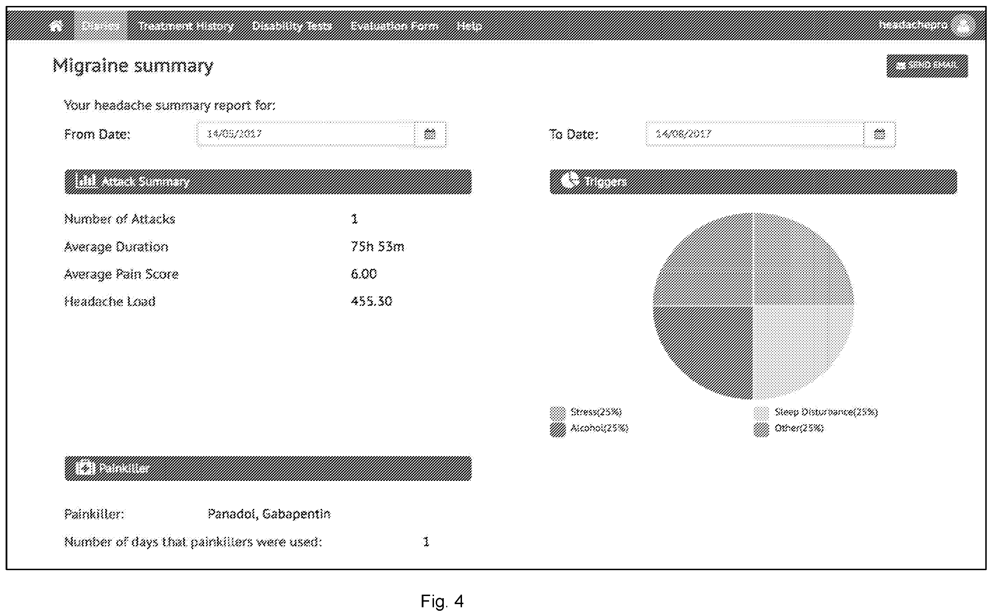

Referring now to Figure 4, the system 20 also allows the patient to view a summary of headaches by headache type. The example shown is a summary of migraines. As can be seen in Figure 4, the patient is presented with a summary over a given time period, which the patient may adjust in order to see progression of the nature, cause or treatment of the headache over a chosen period of time. The summary also gives an indication of the medicaments used and, importantly, of the potential triggers as recorded by the patient. The potential triggers are preferably given in graphical form, in the example shown as a pie chart, which is easy to understand and therefore can give a patient an effective measure of the possible causes of the headache. As the range of dates can be selected by the patient, the patient also has the ability to see how the frequency and severity of the headaches changes over time and over changes in triggers, as well as the efficacy of any medications used.

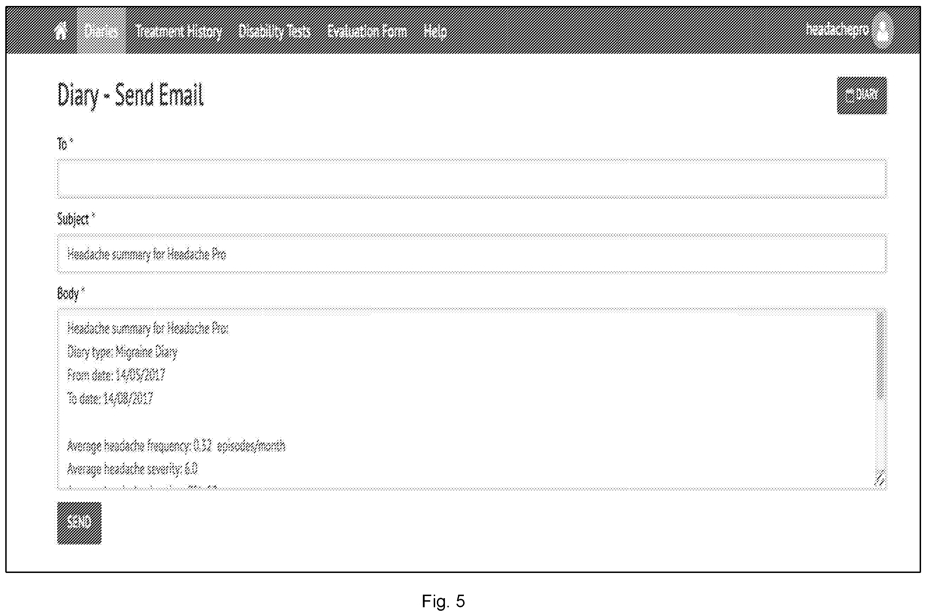

Figure 5 shows the functionality for sending the headache summary by email, for example to the clinician 30.

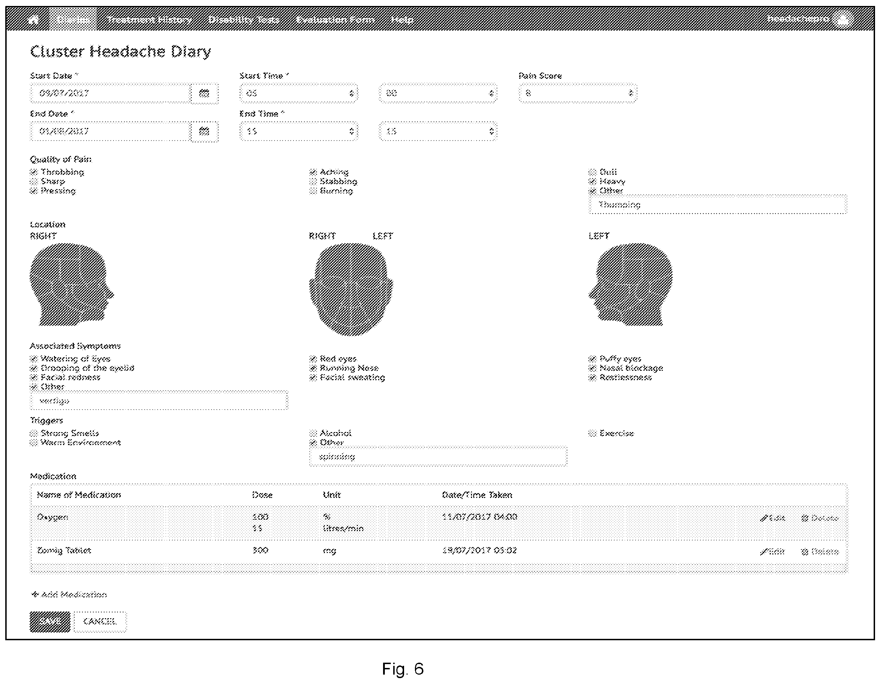

Figure 6 shows the data input page for recording cluster headaches, again accessible via the drop down menu. As with the other diaries, multiple medications can be added, with individual times specified for each medication. As will be apparent form Figure 6, the diary provided for this headache type has different fields specific to that type, in particular for the associated symptoms and potential triggers. As these are specific to the selected headache type, the fields can be kept to a manageable number so that the patient is not presented with a large amount of unnecessary selections as can occur with a generic diary, or otherwise with a much shorter list of selections in order to keep the logging procedure manageable. Again, the use of a graphical interface facilitates data entry, particularly important for real time event logging.

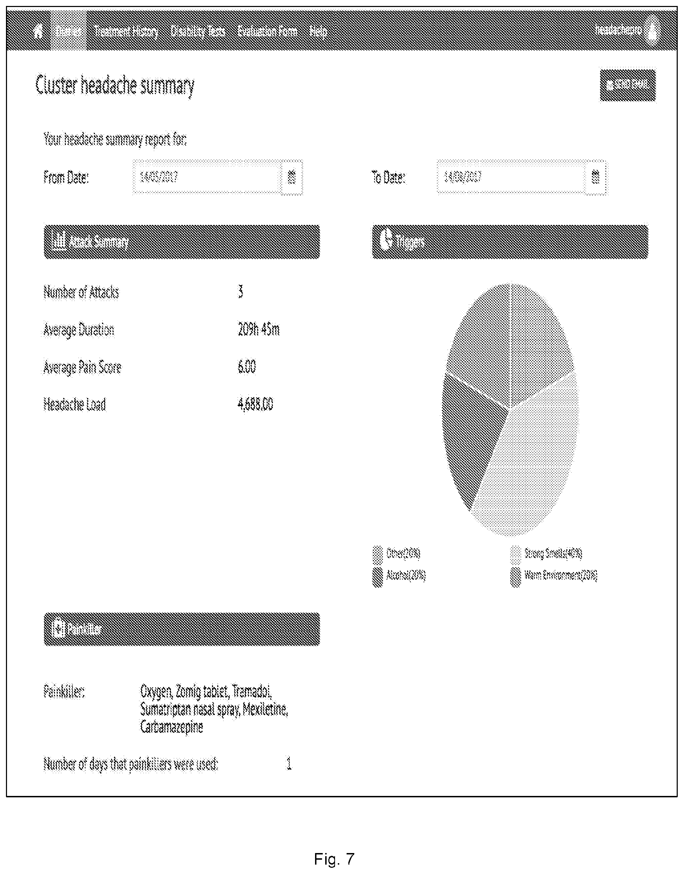

Figure 7 shows an example of a summary of cluster headaches, which is similar to that shown in Figure 4, save for being populated by data from the cluster headache diary. The features of this summary as the same as described above.

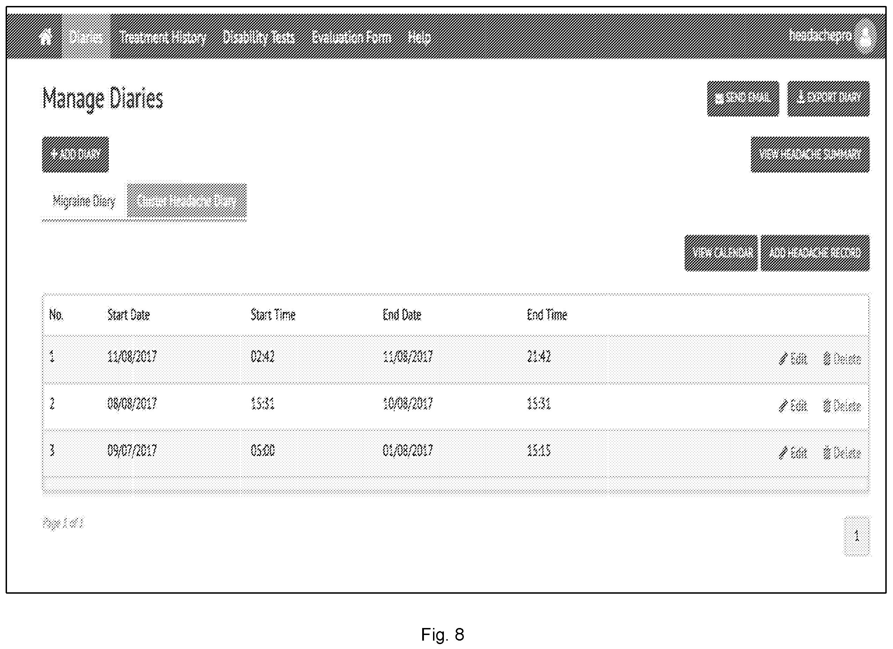

Figure 8 shows the list of events logged into the system and this may be separated into headache types, of which the patient may suffer a variety, as shown. In the alternative or additionally, the system may provide a single list of events with an indication alongside each entry of the headache type so that the patient can see in one screen the totality of headache events over time.

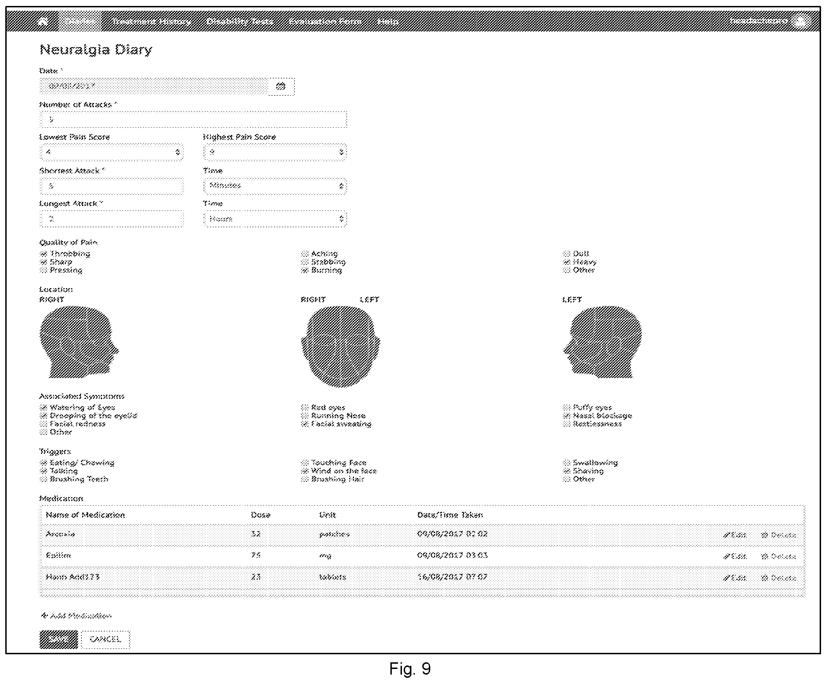

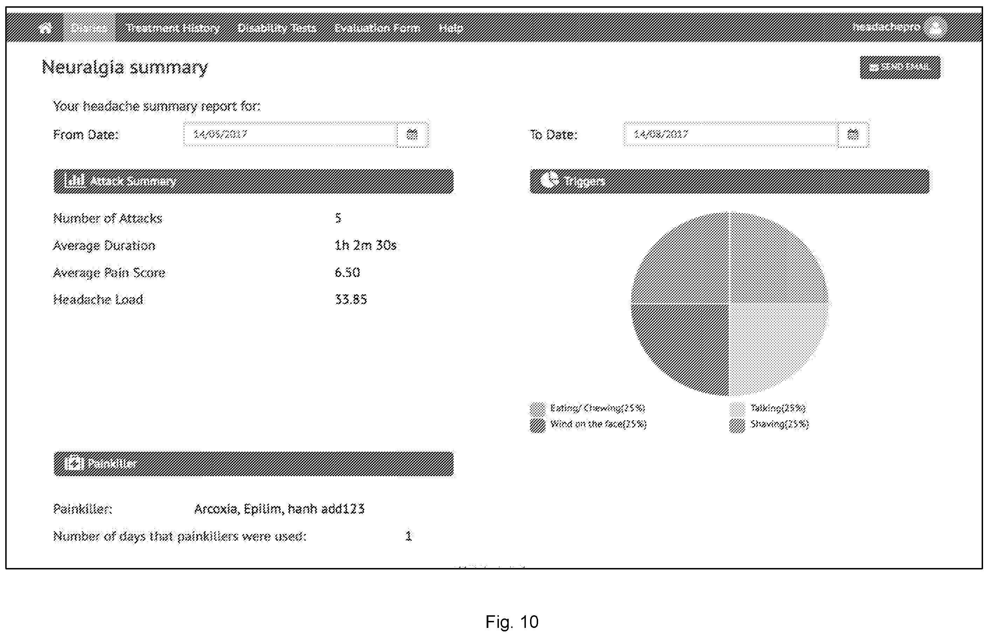

Referring to Figure 9, this shows a patient selection of a neuralgia diary, while Figure 10 shows an example of a neuralgia summary. These have the same characteristics as for the other headache type diaries described above, and is specific to neuralgia headaches particularly in the listed symptoms and triggers.

It will be appreciated that the above description and Figures 2 to 10 show just some examples and that for other headache types there will be associated specific diaries and summary sections. The fields for each such diary will be apparent particularly from Annex 2 below.

In practice, once the patient has had a differential diagnosis and/or a doctor's diagnosis, the patient may select the diaries appropriate to his or her identified headache types. This may be by a patient selection from a drop down list. In other embodiments, the processing unit 20 and/or analytics unit 12 may determine which type-specific diaries to present to the patient in the web or mobile device 14, 16.

It will be appreciated that for all diaries of the system each diary may be saved, emailed, exported, or otherwise sent, for example to the clinician 30.

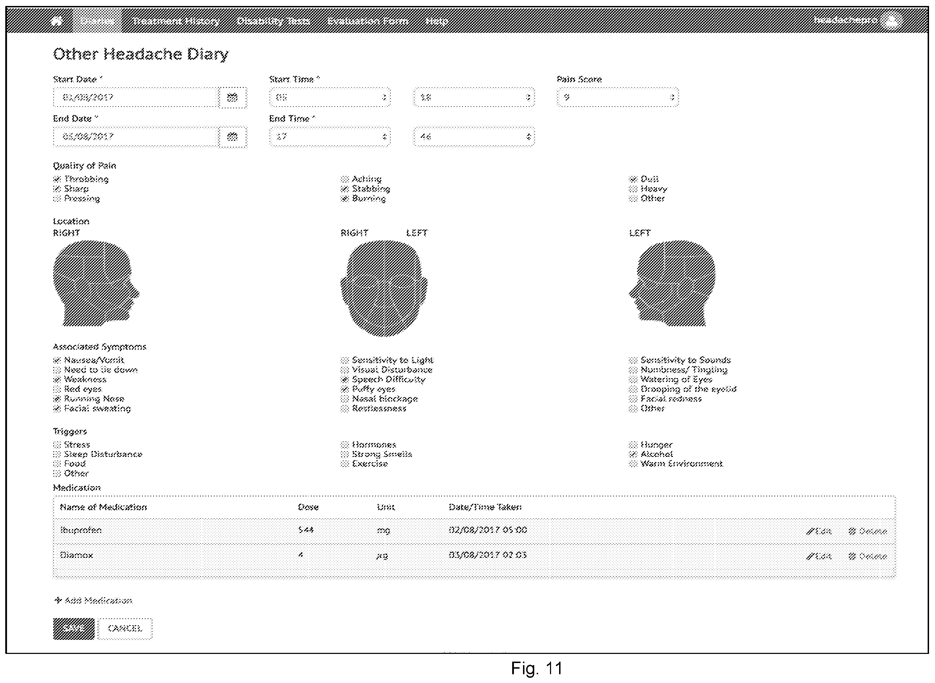

Referring now to Figure 11, the system also preferably provides an additional diary, named in this example as the Other Headache Diary, enabling a patient to log a headache event that the patient cannot associate with a particular headache type. This is useful in cases that, for instance, an initial diagnosis has been inconclusive or the patient believes the symptoms or potential causes are not typical.

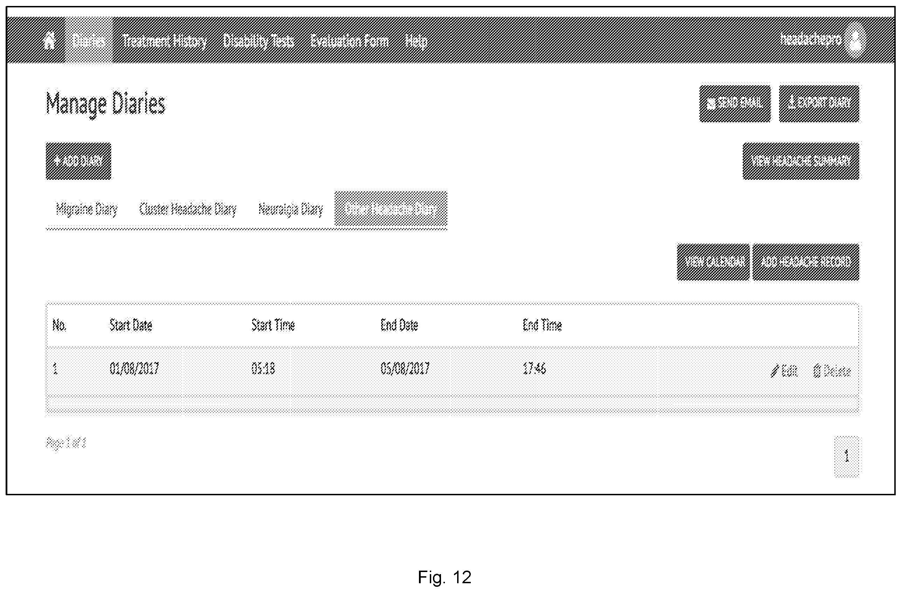

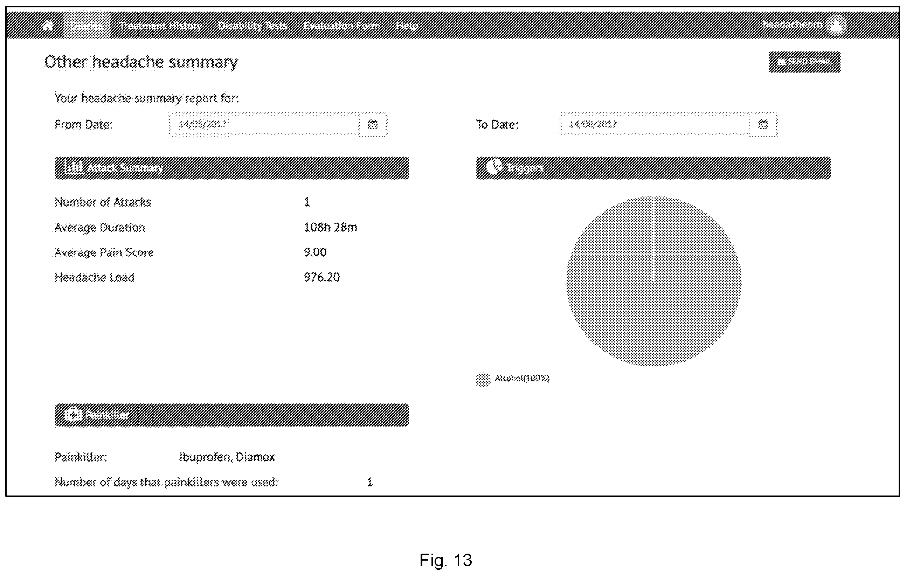

Figures 12 and 13 show examples of the diary management page and other headache diary summary page, which have the equivalent characteristics as explained above.

The data logged through the Other Headache Diary may be kept procedurally separate from the other diaries but in the preferred embodiment the processing unit 20 and/or the analytics section 12 may be configured to correlate that data with headache types for the generation of a differential diagnosis for patient/clinician consideration.

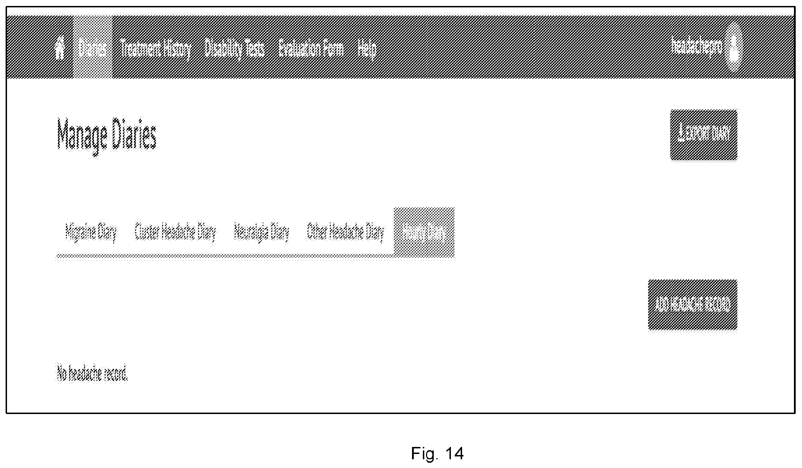

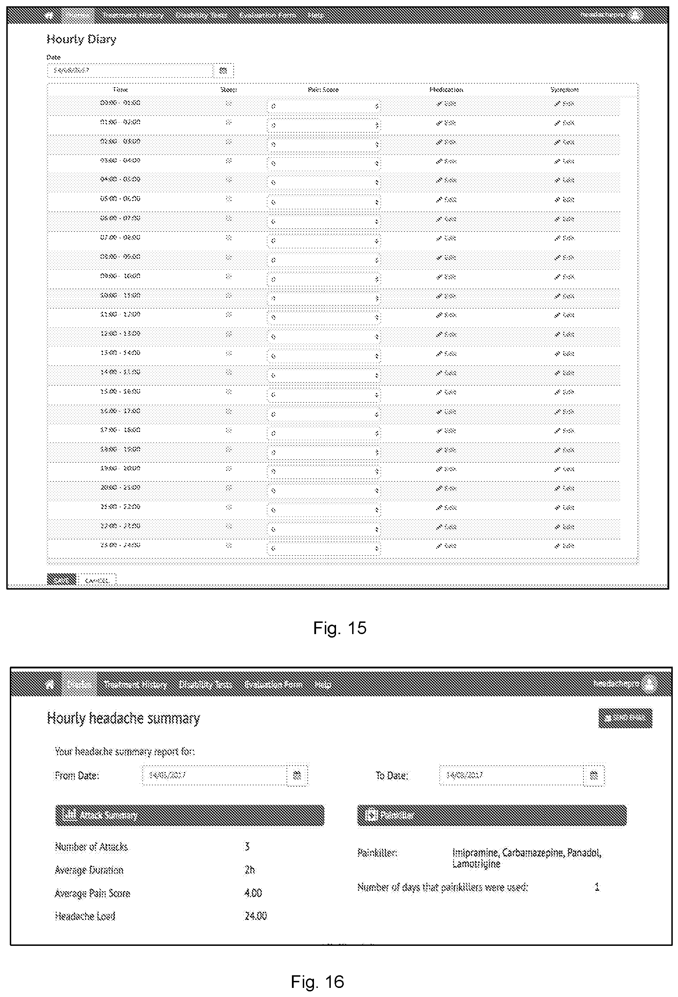

The preferred embodiment also provides a diary divided into time periods, for examples in hours of the day, as can be seen in Figure 15, selectable from the Diary option as shown in Figure 14. In this example, the diary entries are simple, comprising for each time slot an indication as to whether the patient was sleeping, the pain score, medication taken and symptoms suffered. The medication and symptom fields may be provided as drop-down windows allowing the patient to select from a list of medicaments and symptoms, as well as inputting custom data such as an unlisted medication. This simplicity facilitates the data entry for the patient.

This function shown in Figure 15 allows a patient to log and review with ease the timings of any headache, the efficacy of any medicaments and the effect of sleep. It can be used not only to monitor whether a particular treatment has functioned but also to record how the drug affects the progress of a headache over time. This can assist in better management of headaches by the patient personally, as well as by the clinician 30.

Each hourly diary, preferably logging one day, can be stored in similar manner to the other diaries for later review in a summary and comparison against other hourly diaries. Similarly, each hourly diary can be associated with a summary page as with the other diaries and as shown in Figure 16, as well as being able to be sent by email or other means or exported for instance into a spread sheet.

It will be appreciated that the system and in particular the processing unit 20 may be arranged so as to populate different diaries with entries into one of the diaries so as not to require the patient to duplicate entries. For instance, the type-specific diaries may also be logged into the Hourly Diary and vice versa, as can the Other Headache Diary. In other embodiments, the Hourly Diary is kept separate from the other diaries.

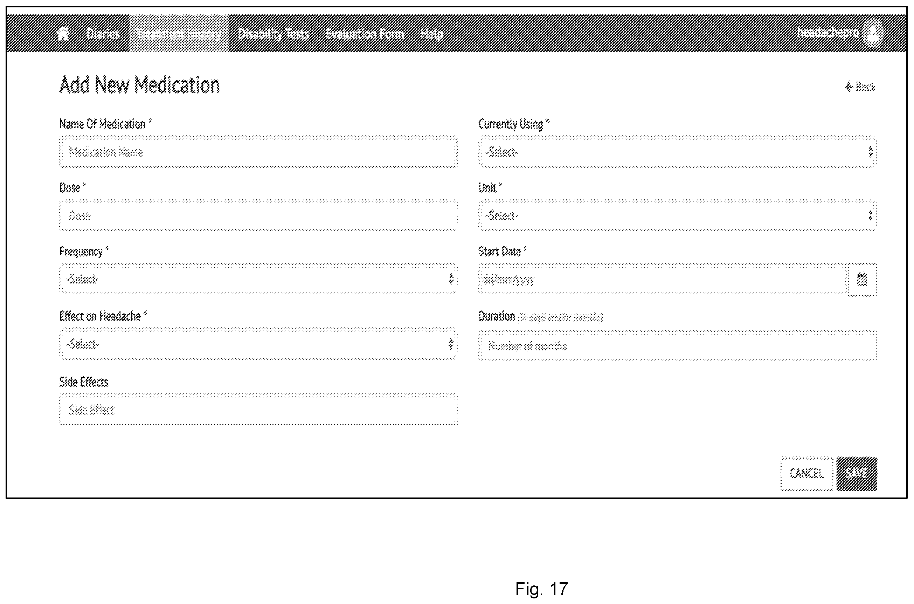

With reference to Figures 17 to 21, the system also provides for recording medicaments used by the patient as well as injectable treatments or infusions, and neurostimulators. These are stored in order to improve the ability of the patient and clinician to log the treatments used and verify their effectiveness, as well as for keeping this information recorded for extended periods of time.

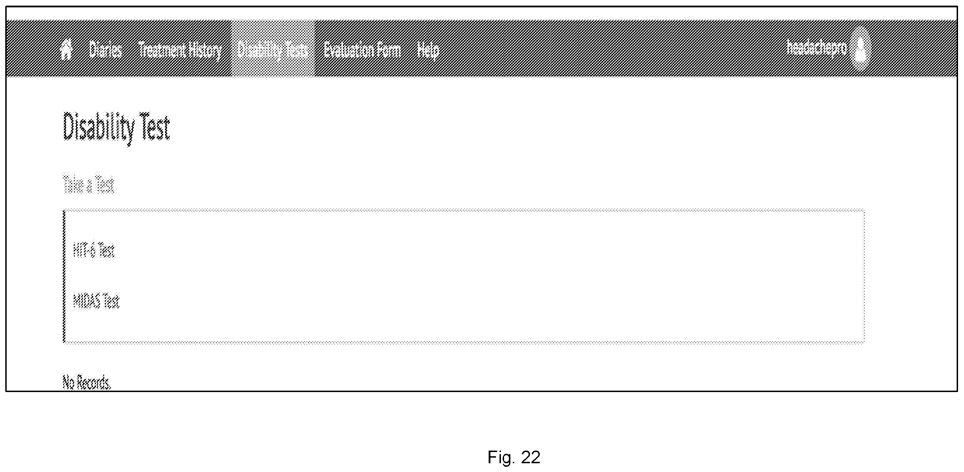

Figures 22 to 29 show the provision of disability tests for the patient to complete and store in the system 20. These may be standard tests such as the Headache Impact Test (HIT-6) and the MIDAS Test. With the system 10, it is possible for the user to take the test many times and the results will be recorded for each time.

In some cases the patient's doctor may request other types of test to be completed by the patient. Once the test has been completed, the result will be sent to the clinician 30 and the patient 32 may not see the results. In such cases there can be time stipulations for the patient to complete a test within a certain time period. The patient will be sent a notification, preferably by email, to complete the test.

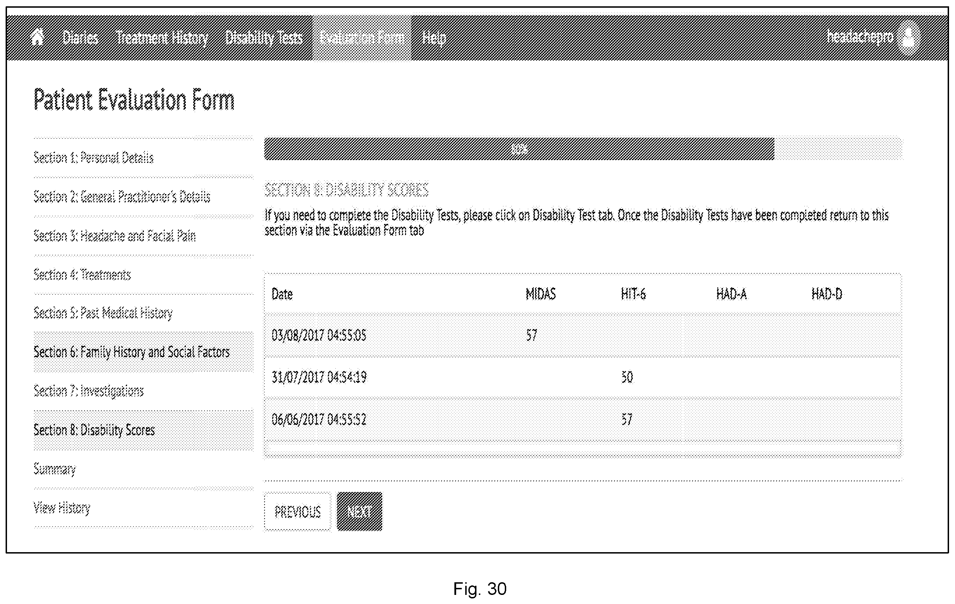

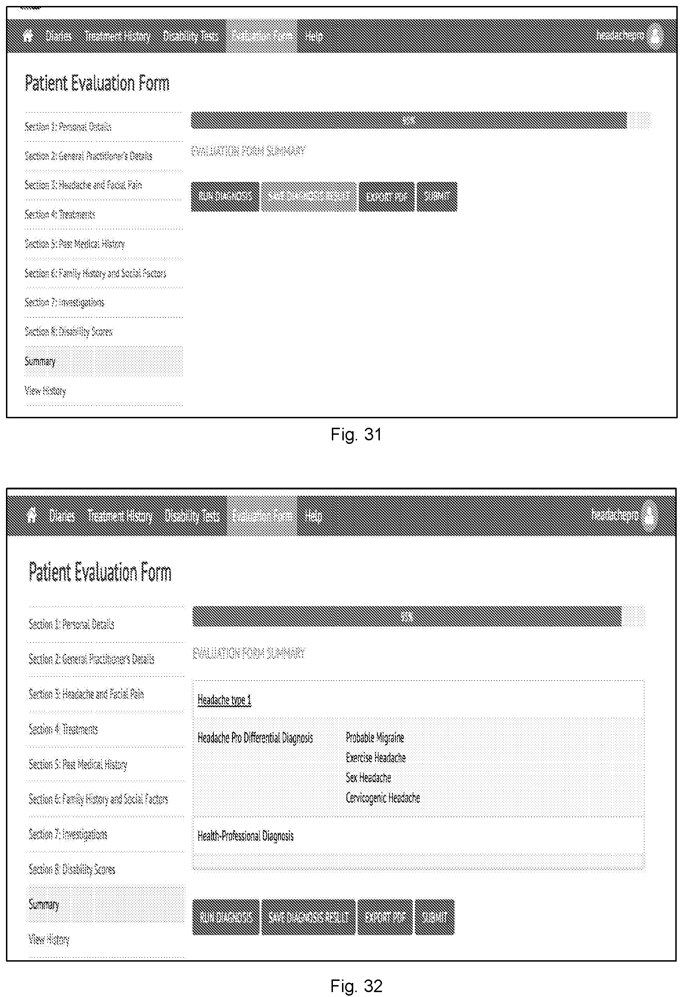

Figure 30 shows a screenshot of the preferred embodiment for the completion of a patient evaluation form, that is the population of the questions shown in Annex 1. The system 20 allows for the user to complete the generic questionnaire multiple times over chosen intervals and to store each set of data for future evaluation and comparison. The system provides for the patient to run a differential diagnosis (Figures 31 and 32) based on the answers provided during the evaluation. Each differential diagnosis result can be saved, which resets the evaluation form for completion afresh at a later point in time. The form data can also be exported or sent, for instance to the patient's clinician 30.

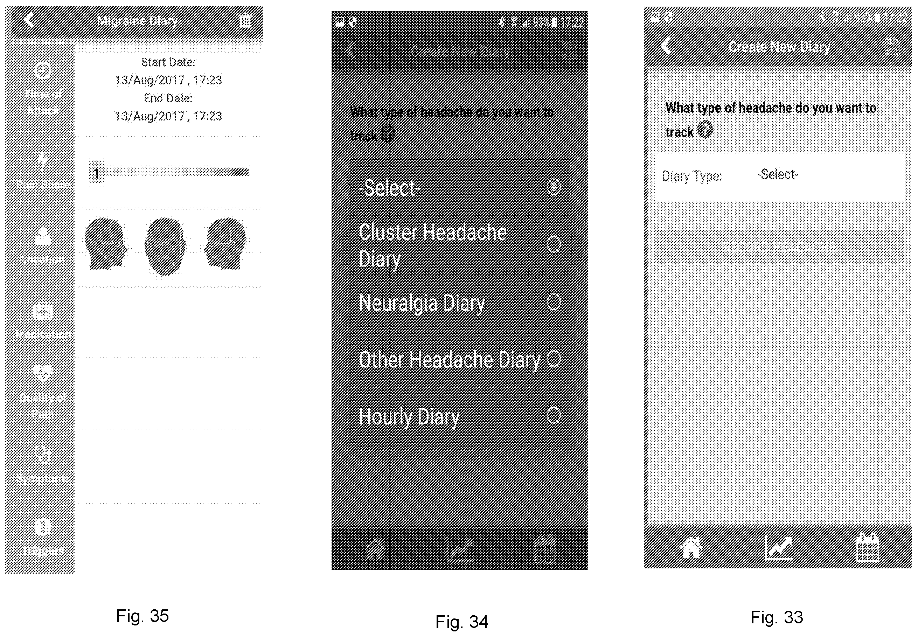

The above embodiment, the operating elements of which are shown in Figures 2 to 32, is typically implemented on a patient's computer 14. Figures 33 to 72 show another embodiment in which the input and output functionalities of the system and implemented on a portable device, for instance a mobile telephone or tablet computer device 16. This can be advantageous because there can be no guarantee that the patient will have ready access to a computer during the course of a headache, or the ability to manage a computer at that time. The interface for a telephone or similar device 16 is simple and easy to use particularly for a suffering of debilitated patient. The core elements of the implementation shown in Figures 33 to 72 is the same or similar to that of Figures 2 to 32 but with a simplified menu set and greater graphical assistance. As it is more likely that a patient will be near a mobile telephone or similar device, than by a computer, there is a greater chance for the patient to log headache events in real time. The processing unit 20 may be included within the functionality of the telephone or similar device 16 but in other embodiments the processing may be carried out remotely, such as in the patient's computer 14 or a remote server, which may be the analytics section 12. For this purpose, the portable device, such as telephone 16, may store the data until the next available opportunity to transfer that data for processing and eventual feedback of results.

With reference to Figures 33 and 34, this embodiment provides a simple selection by a drop down menu of the headache diaries available to the patient, following a differential diagnosis or clinician diagnosis.

Figures 35 to 44 show the graphical interfaces provided to the patient for entering migraine data into the system. The interface is heavily graphical for ease of entry particularly in difficult conditions and when the patient may be suffering the symptoms of a headache. Figure 35 shows a main page for the Migraine diary. It is a summary so the patient can get an overview of the diary. The patient can bypass certain areas that are not relevant on that occasion, by clicking one of the icons shown on the left hand side.

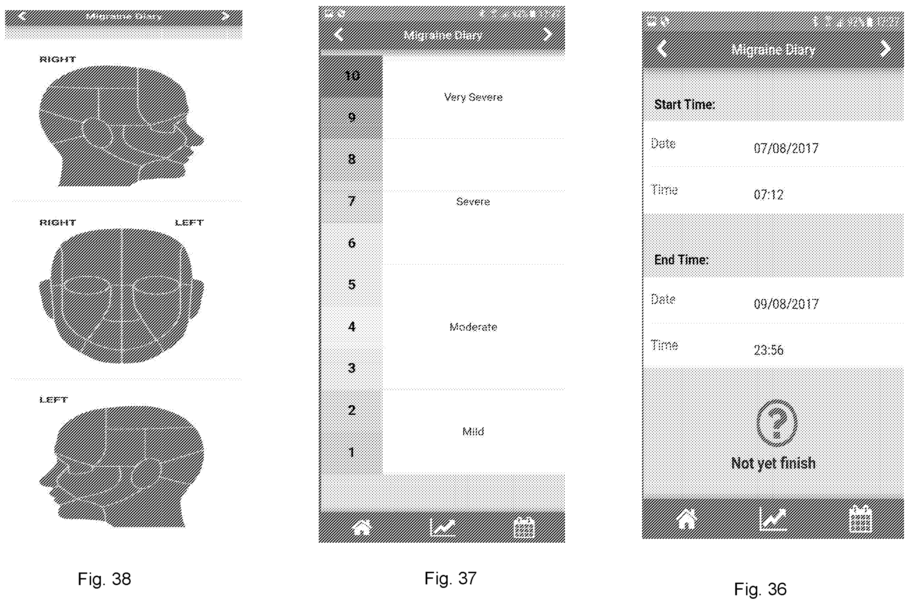

Figure 36 provides a simple entry system for logging start and end times of a headache, which may usefully be by selection from a drop down list. If the patient is completing the diary at the time of an attack, the end date/time can be left uncompleted. The patient can return to this page via the summary page of the diary (Figure 35).

Figure 37 provides a simple selectable bar chart for indicating overall pain level, that is the severity of the attack. The patient can press on number 1 to 10 to state the level of pain experienced during the headache. Once the number has been pressed, the system will highlight it and register it for that headache event. The patient can then move to the next data entry set by pressing the forward arrow at the top right-hand side.

Figure 38 allows for the user to press on one or more of the displayed head areas to indicate the head location or locations most affected by the headache. The patient is able by this functionality to input accurate locations of pain by clicking on the associated area. The head is preferably shown in side (right and left) and front views.

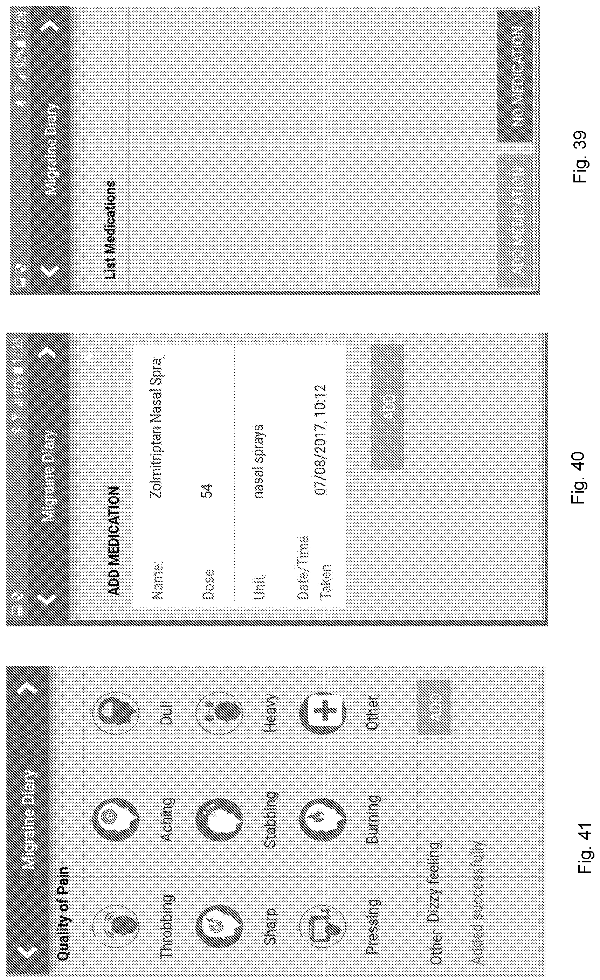

Figures 39 and 40 provide for entry of medications used and again this is preferably by selection from a drop-down list of medicaments (which may be populated only by medicines typically used for the identified headache type). Figures 41 to 43 provide simple graphical icons for identification of the quality of pain, associated symptoms and possible triggers. There may, of course, be different icons and selections from those shown in the Figures, which are indicative only, and there may be provided more than one graphical screen in cases where the total number of options is too large to fit comfortably on a single screen image. If there is no appropriate word/icon in the list, the "other" button allows the patient to input the detail and save by clicking the arrow in the top right-hand corner.

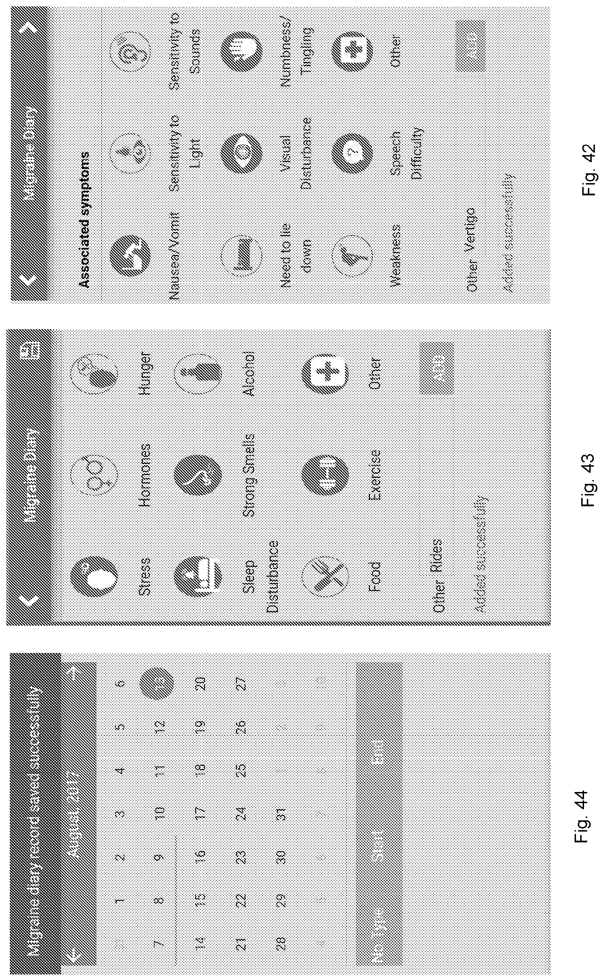

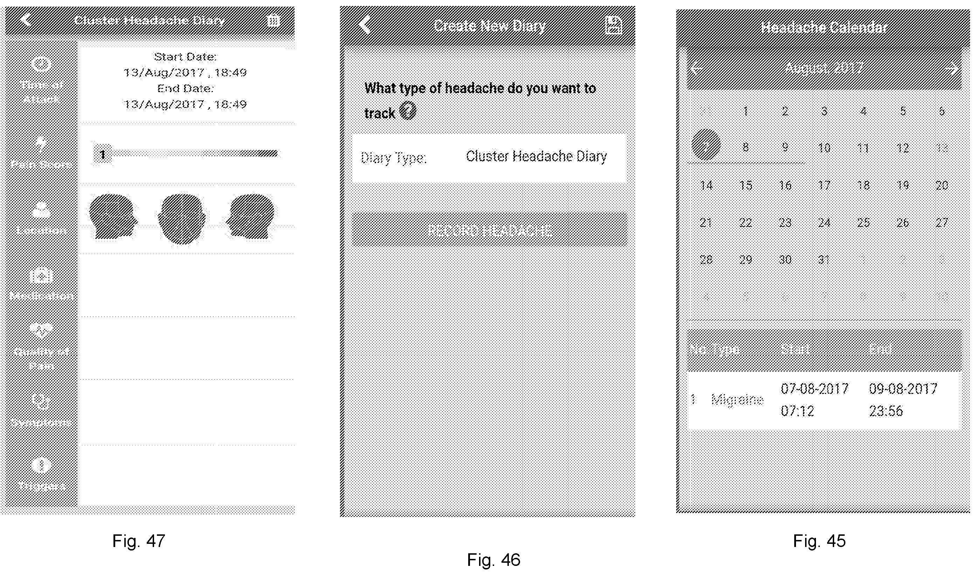

Figures 44 and 45 show how the logged headache data can be saved for later use.

Figures 47 to 53 show the screen shots for a cluster headache diary. Figure 47 is a summary page giving the patient an overview of the historical diary entries for cluster headaches. The patient can bypass certain options by clicking on one of the left hand icons rather than going through each page of the diary.

The other elements of this diary are as previously described, save for being specific to cluster headaches.

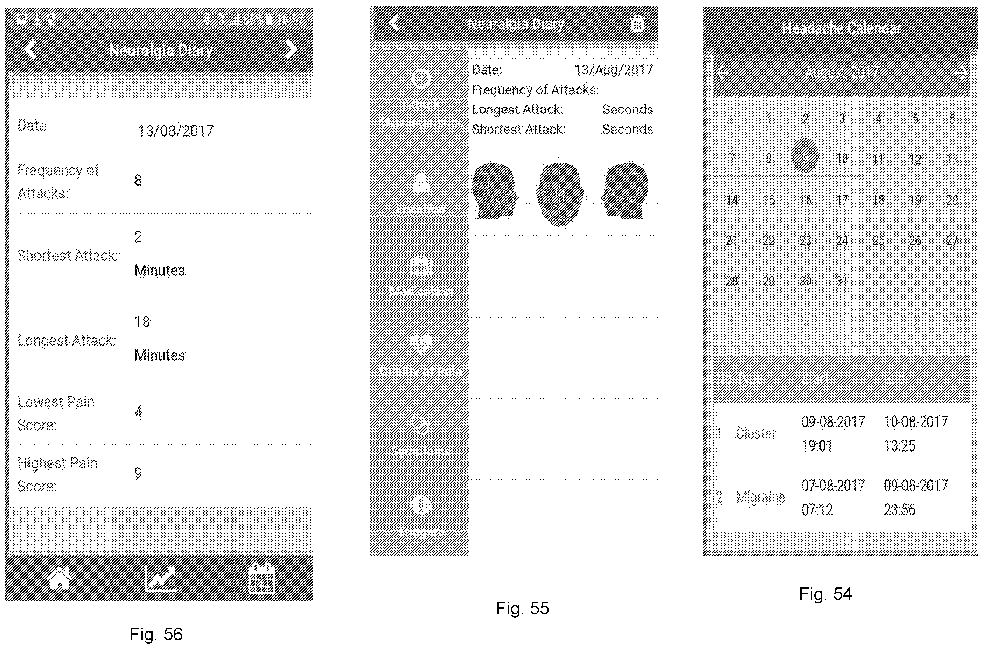

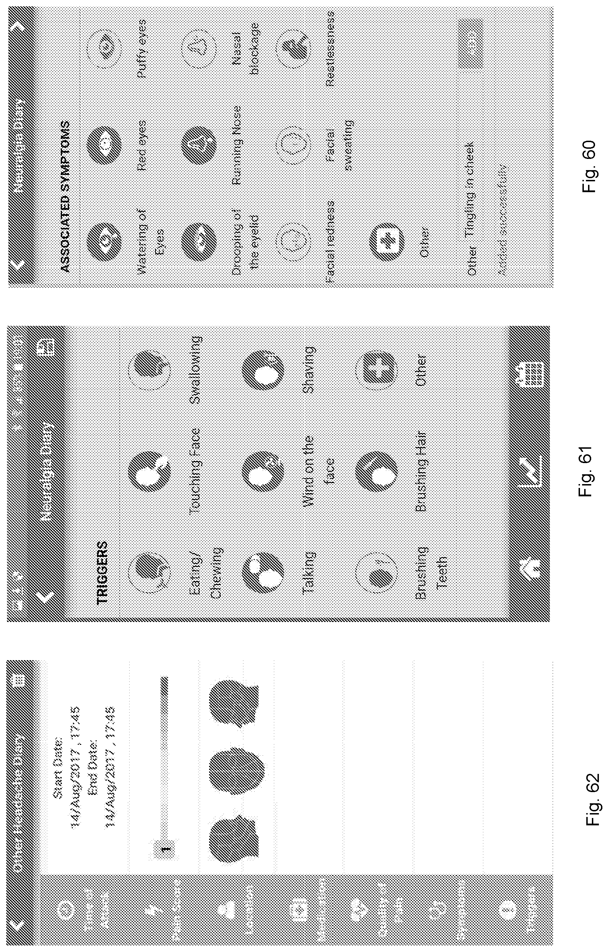

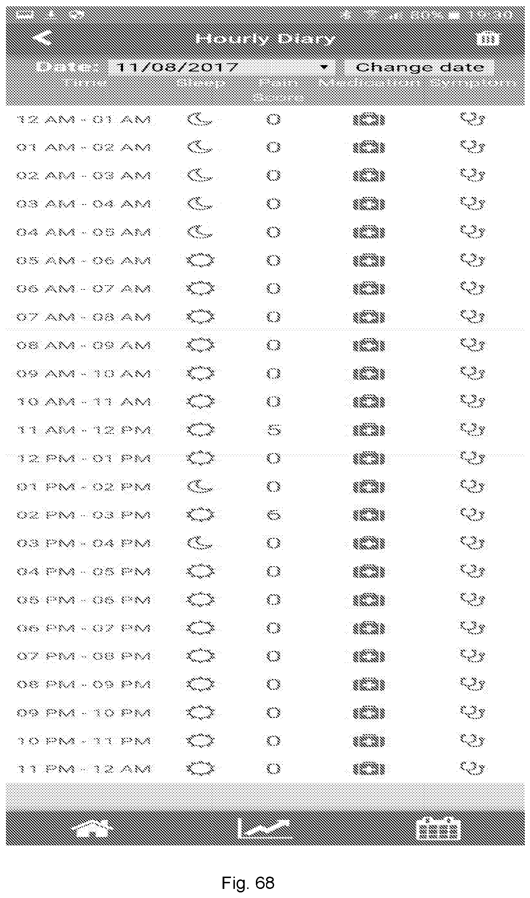

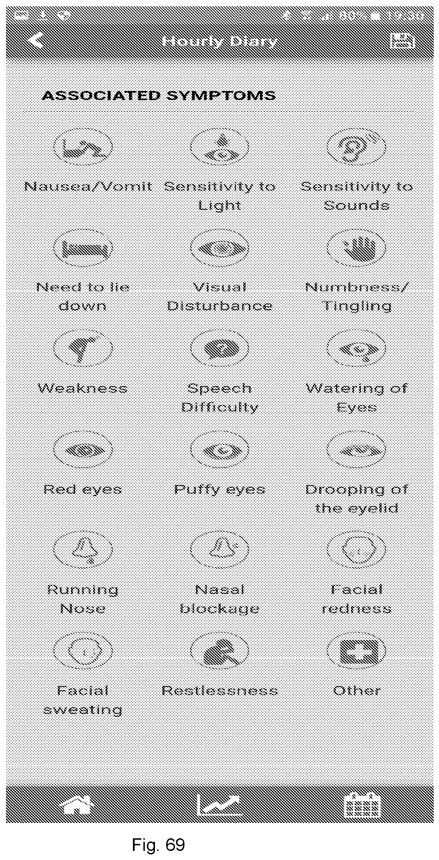

Figures 55 to 61 relate to a neuralgia diary, while Figures 62 to 67 relate to the "other headache diary". An hourly diary is shown in Figures 68 and 69. As the reader will appreciate, the associated symptoms and triggers differ in dependence upon the type of diary.

The hourly diary shown in Figure 68 has hours during which the patient can state when the headache occurred. It comprises also a number of selectable fields including a sleep field, at which the patient (or other user) can click on the sleep icons to state if the patient was sleeping (clicking on the sun sign toggles it into the half-moon crescent symbol). A pain score can be added for every hour.

As with all the other diaries, this diary allows the patient to input the medications used.

The associated symptoms icon will take the patient to a separate page for selecting the associated symptoms, shown in Figure 69.

Once the diary has been saved, its record will be accessible via a conventional calendar view.

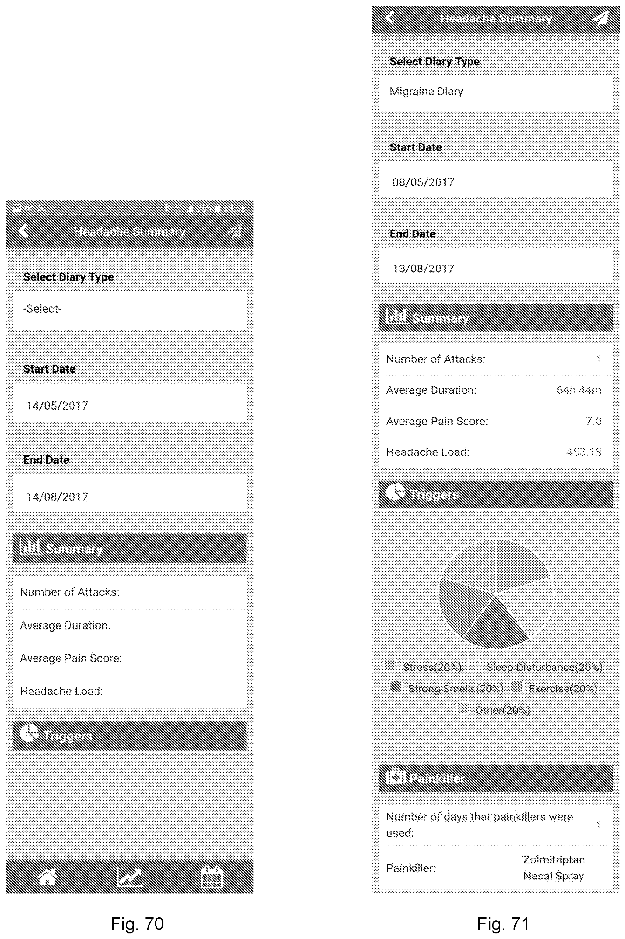

Figures 70 and 71 show the preferred headache summary views. Figure 71 provides the information, particularly relating to triggers is graphical form, to assist the patient in understanding possible causes of the headache.

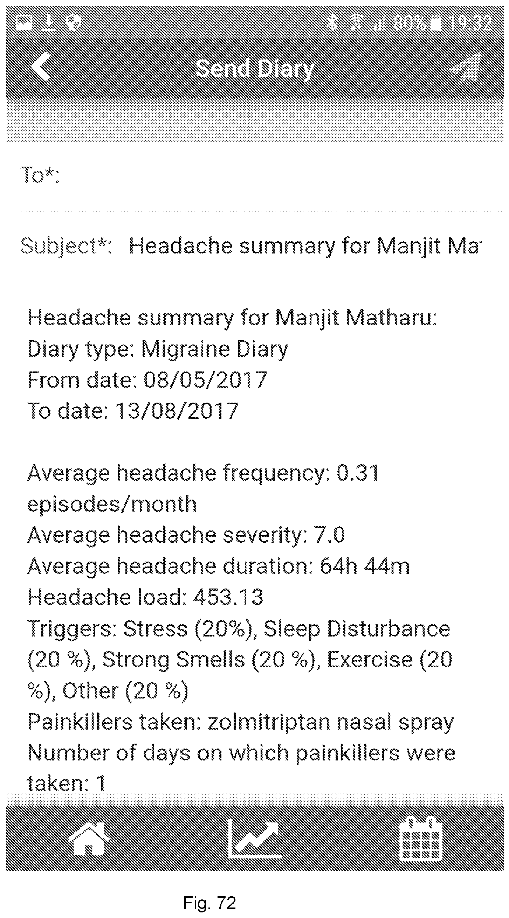

As with the desktop version, this embodiment provides functionality to send a copy of the diary, for example to the patient's clinician, as depicted in Figure 72.

In cases where the patient 32 is provided with and uses a sensor device 18, this will preferably automatically gather data and preferably also send it automatically to the registry/processing unit 20 for population of the relevant headache calendars. In some cases, data upload from the sensor device may require pairing by the user, in a manner known in the art.

It is to be appreciated that where references are made herein to a patient that these include also a person associated with a patient, that is a person who may assist a patient during the course of a headache event.

It will be appreciated from the above that the system disclosed herein provide a mechanism by which a patient has greater knowledge and potential control over the management of headaches, which can lead to more effective diagnosis and faster long term treatment, taking out a substantial proportion of the trial and error approach that is currently prevalent. The ability to log data effectively in real time, that is during the course of a headache, can provide much more precise gathering of data and identification of possible triggers, while the automatic harvesting of data from a sensing device 18 can collect data about the patient that the patient would otherwise not be able to collect personally.

The collection of data from potentially a large number of patients allows the symptoms and triggers to be categorised into patient type, which can lead to more effective diagnoses and treatments, as well as the ability to adapt over time the algorithms used for generating differential diagnoses. Furthermore, the system provides a platform by which the efficacy of medicaments and other treatments can be monitored.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

### APPENDIX 1: INITIAL GENERIC DATA COLLECTION

The headache diagnostic algorithm is based on the questions outlined below. User answer these questions and the algorithm (Appendix 2) is then applied to ascertain and generate potential diagnoses

### SECTION 1: PERSONAL DETAILS.

Please provide the following information

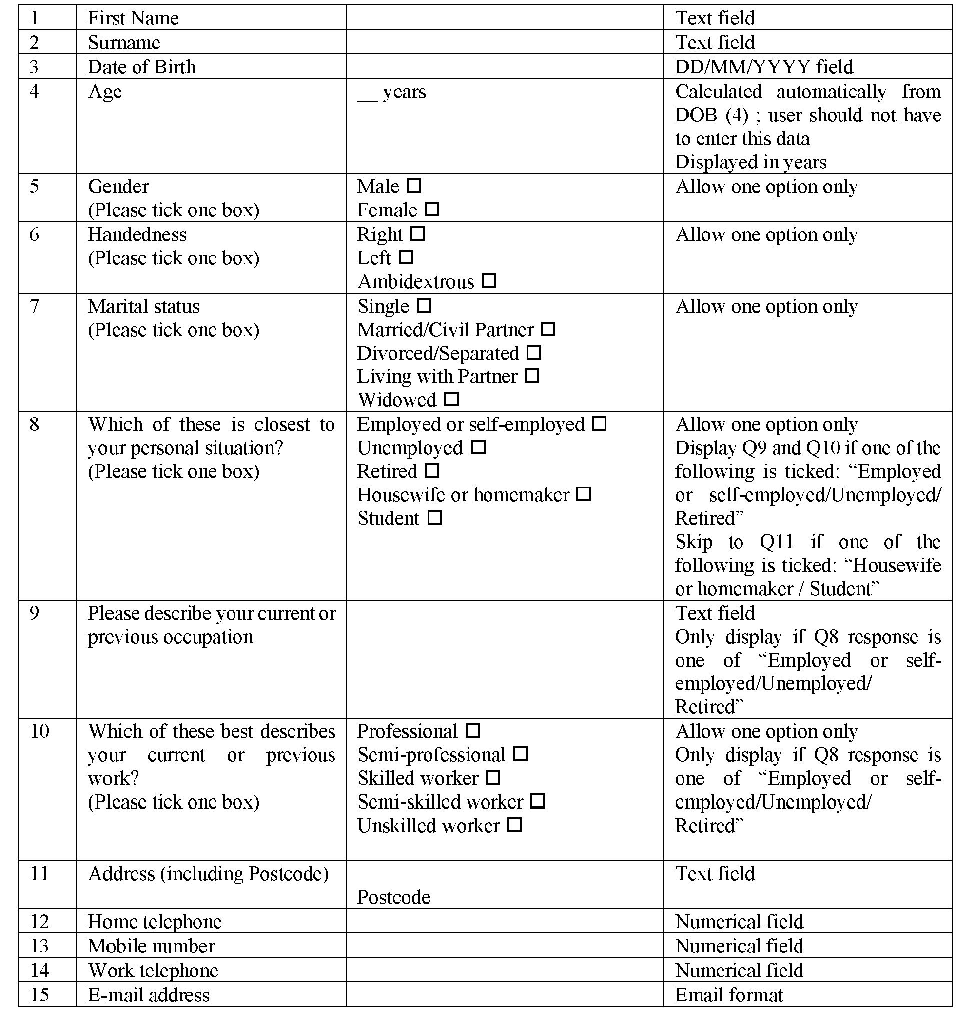

### SECTION 2: GENERAL PRACTITIONER'S DETAILS.

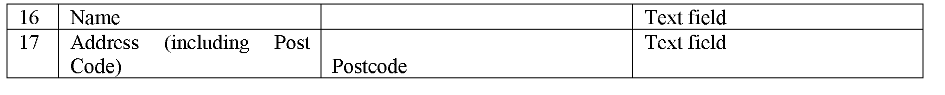

### SECTION 3: HEADACHE AND FACIAL PAIN

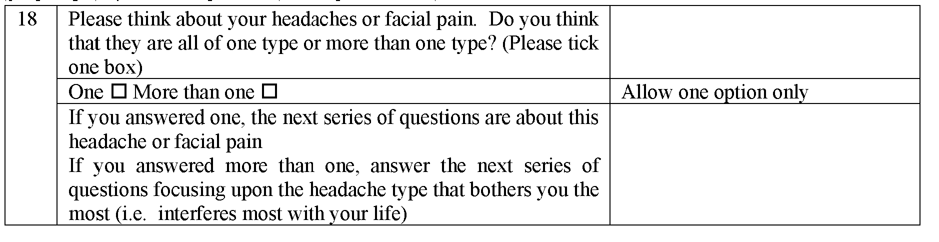

### SECTION 3.1: Headache or Facial Pain Type 1

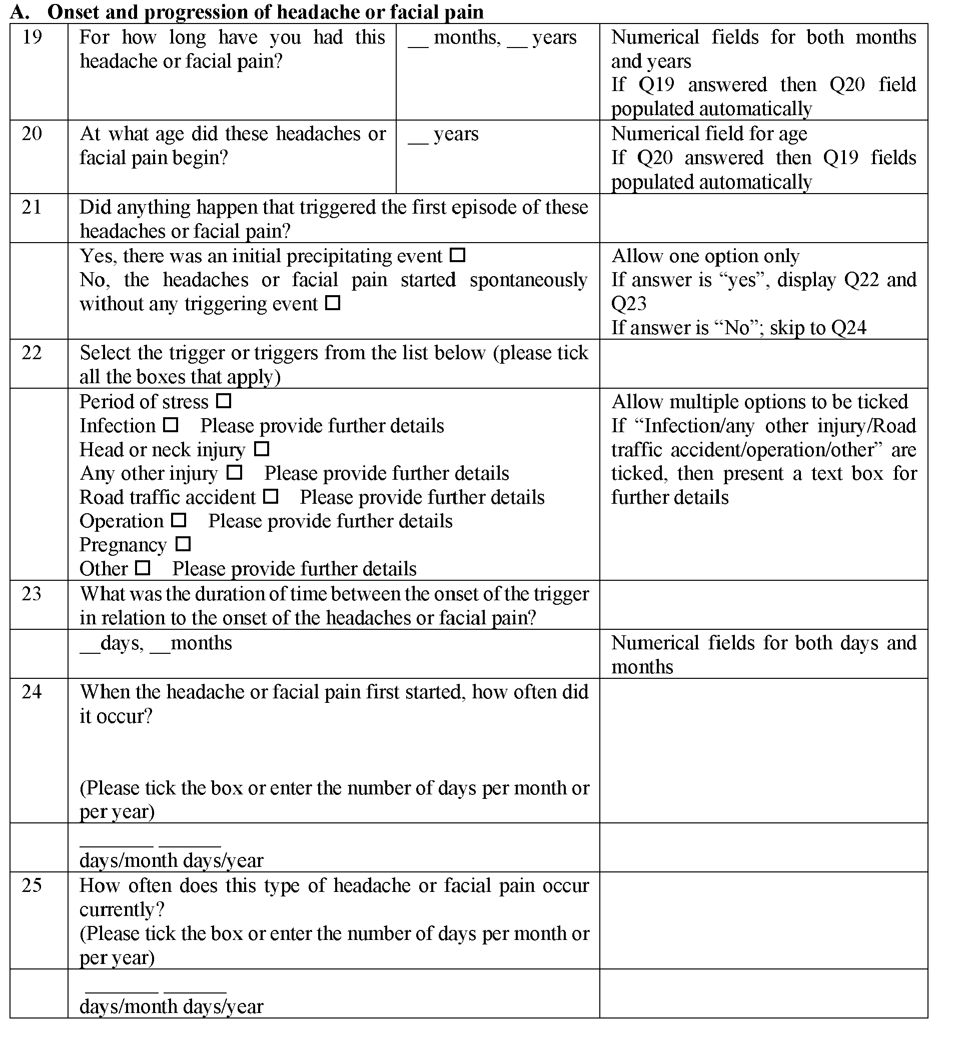

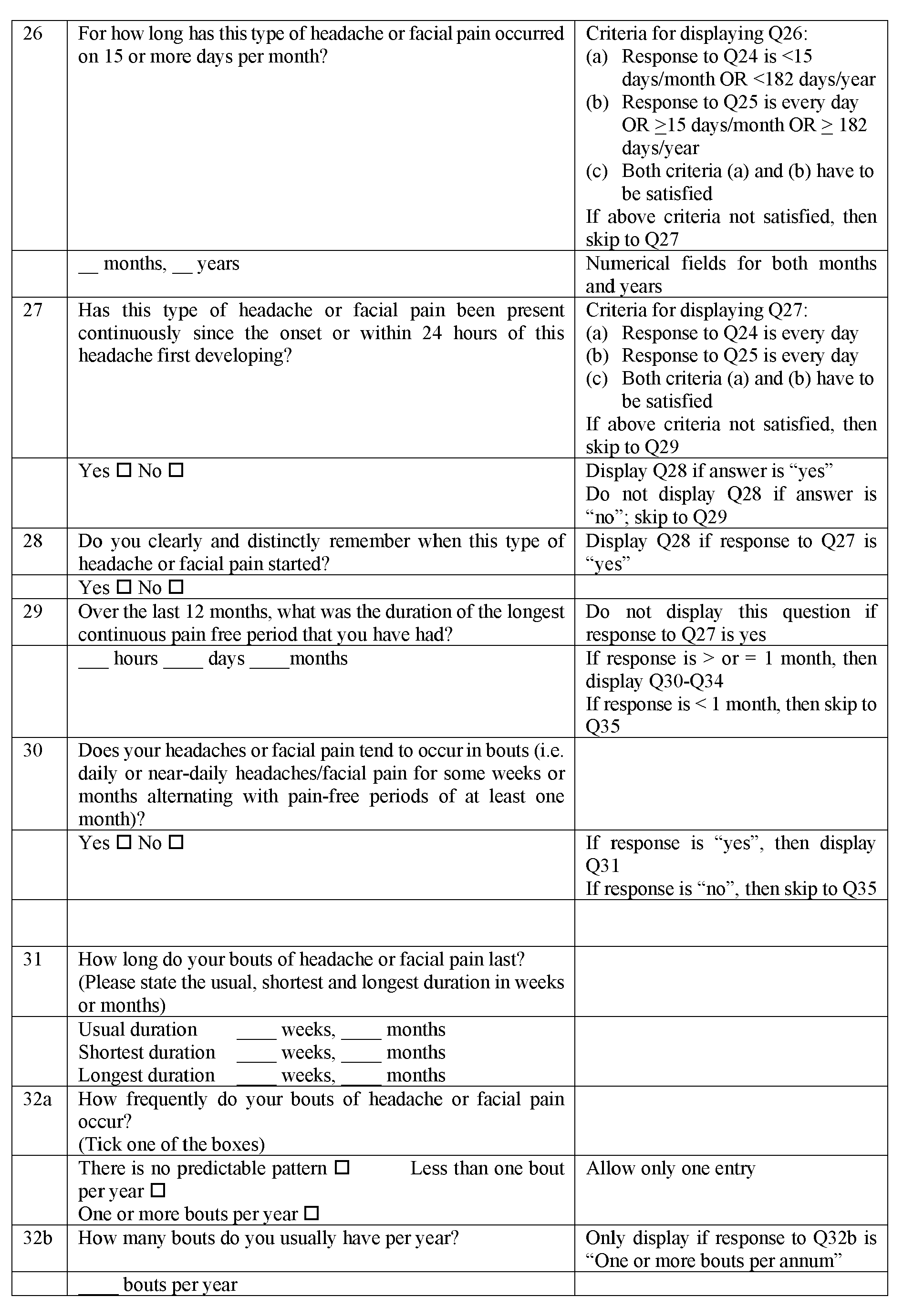

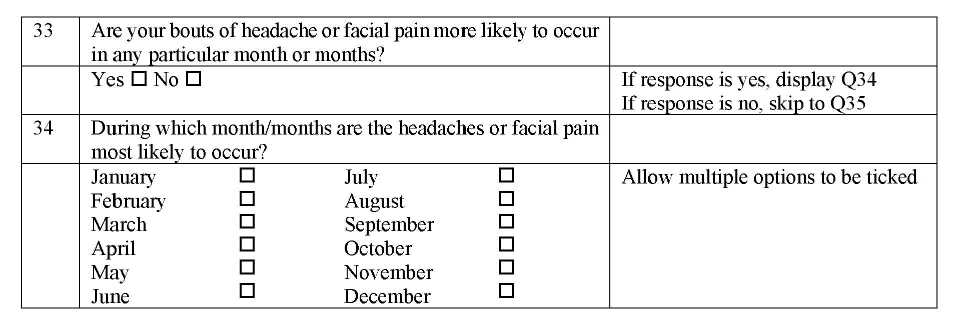

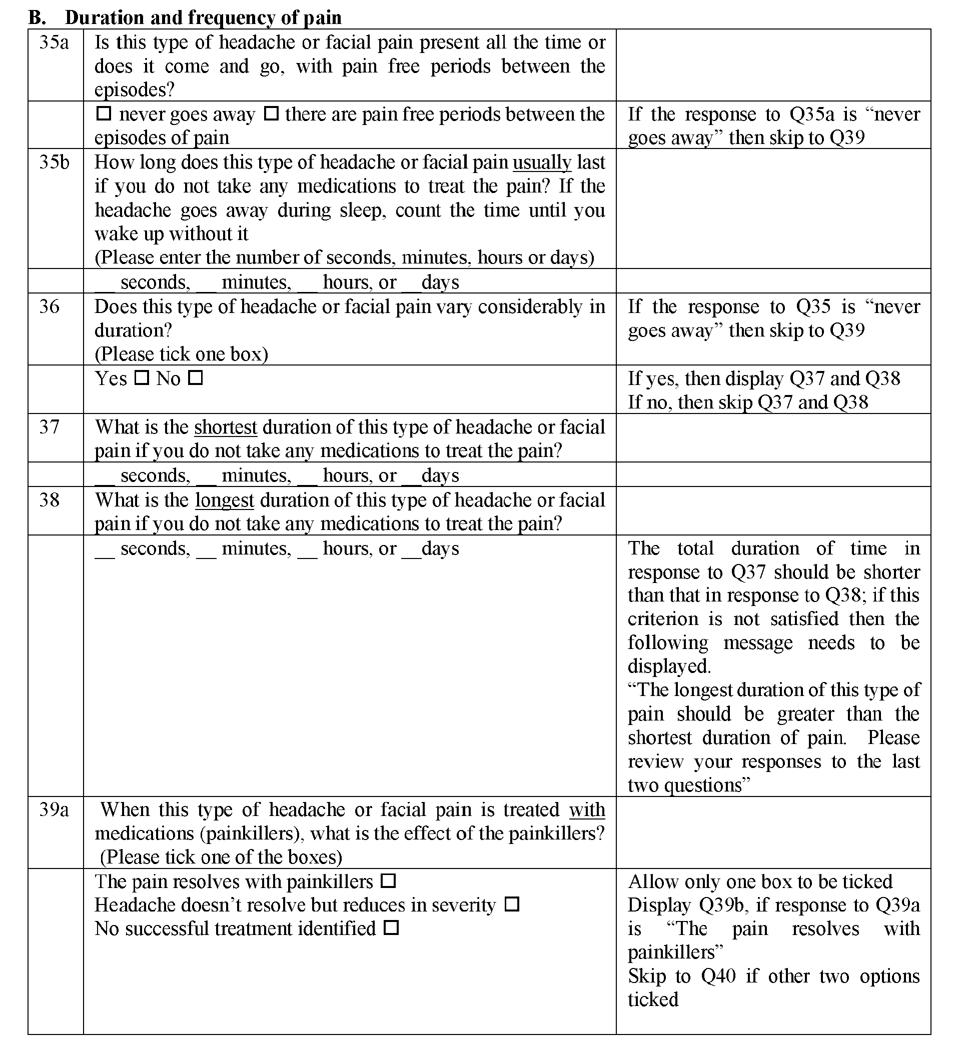

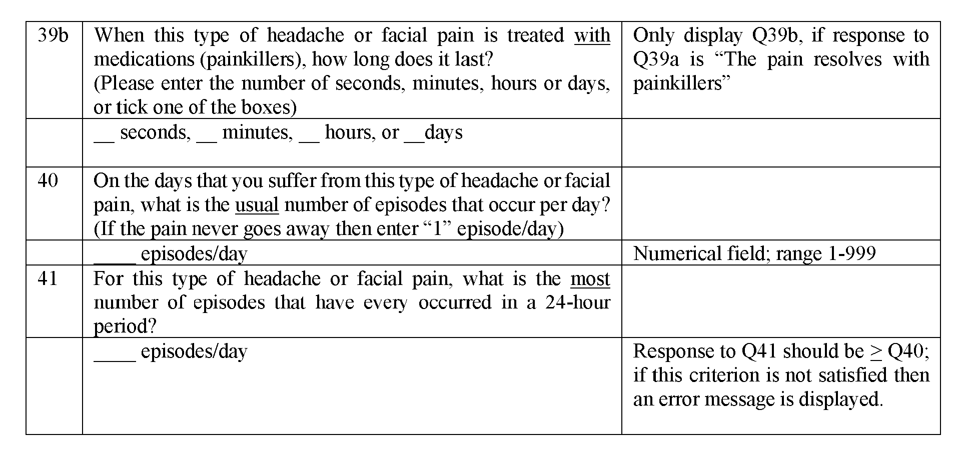

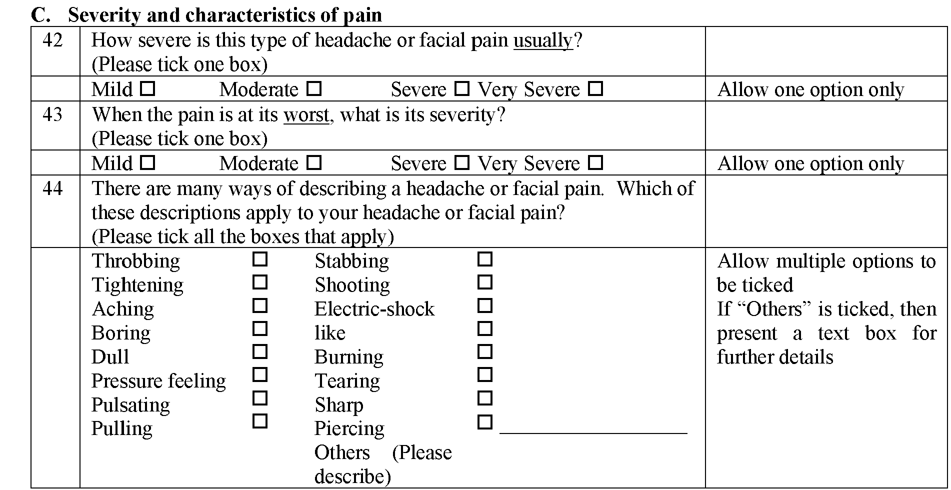

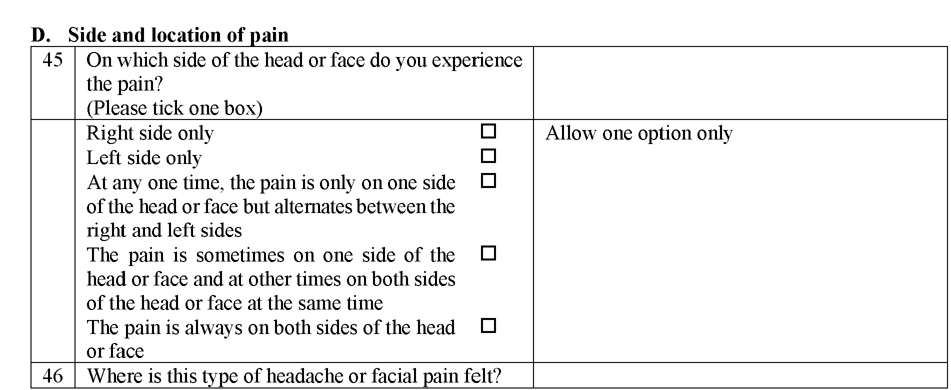

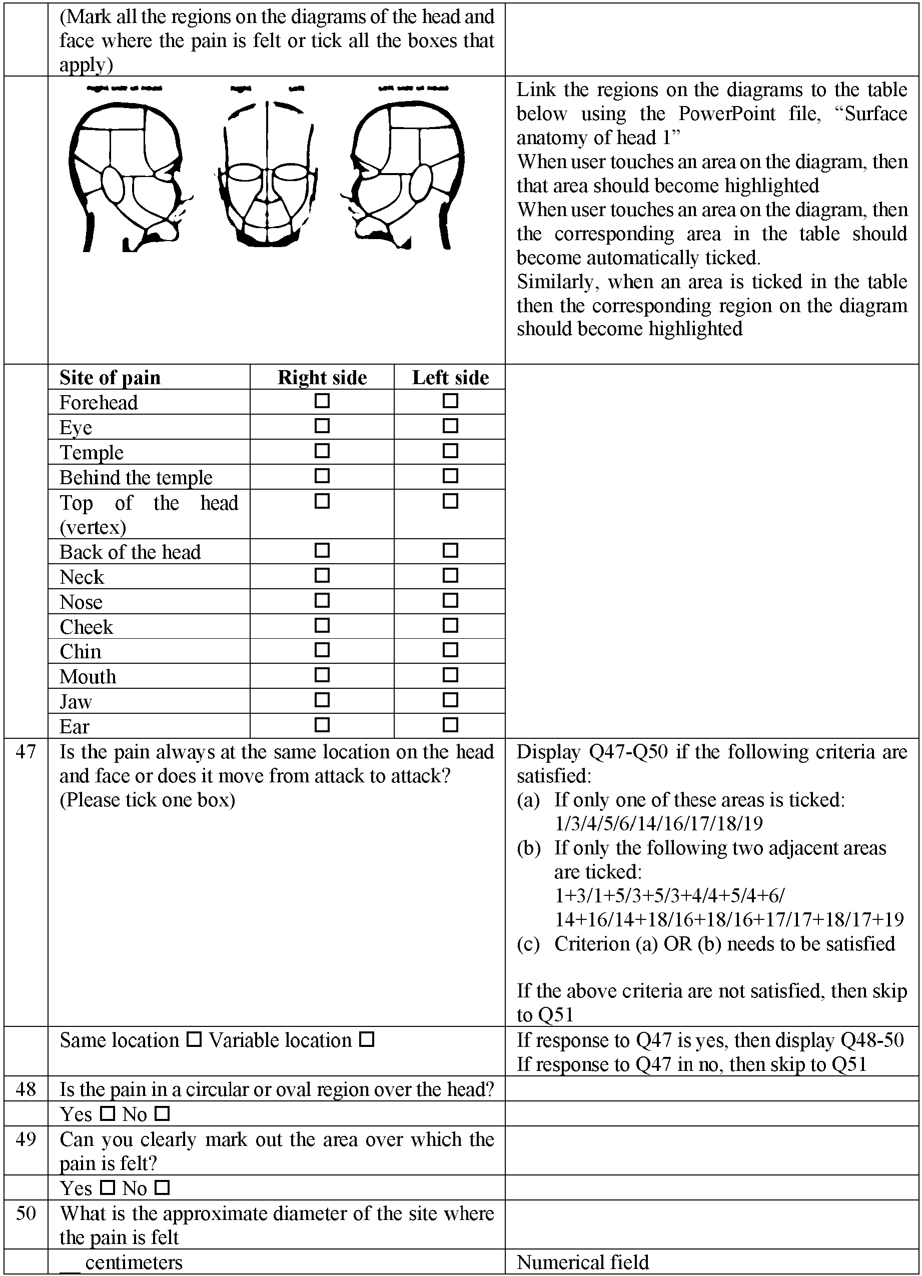

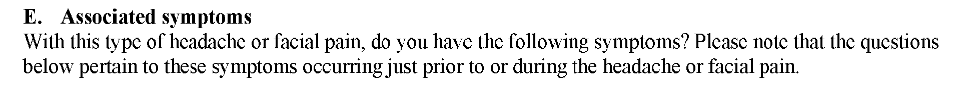

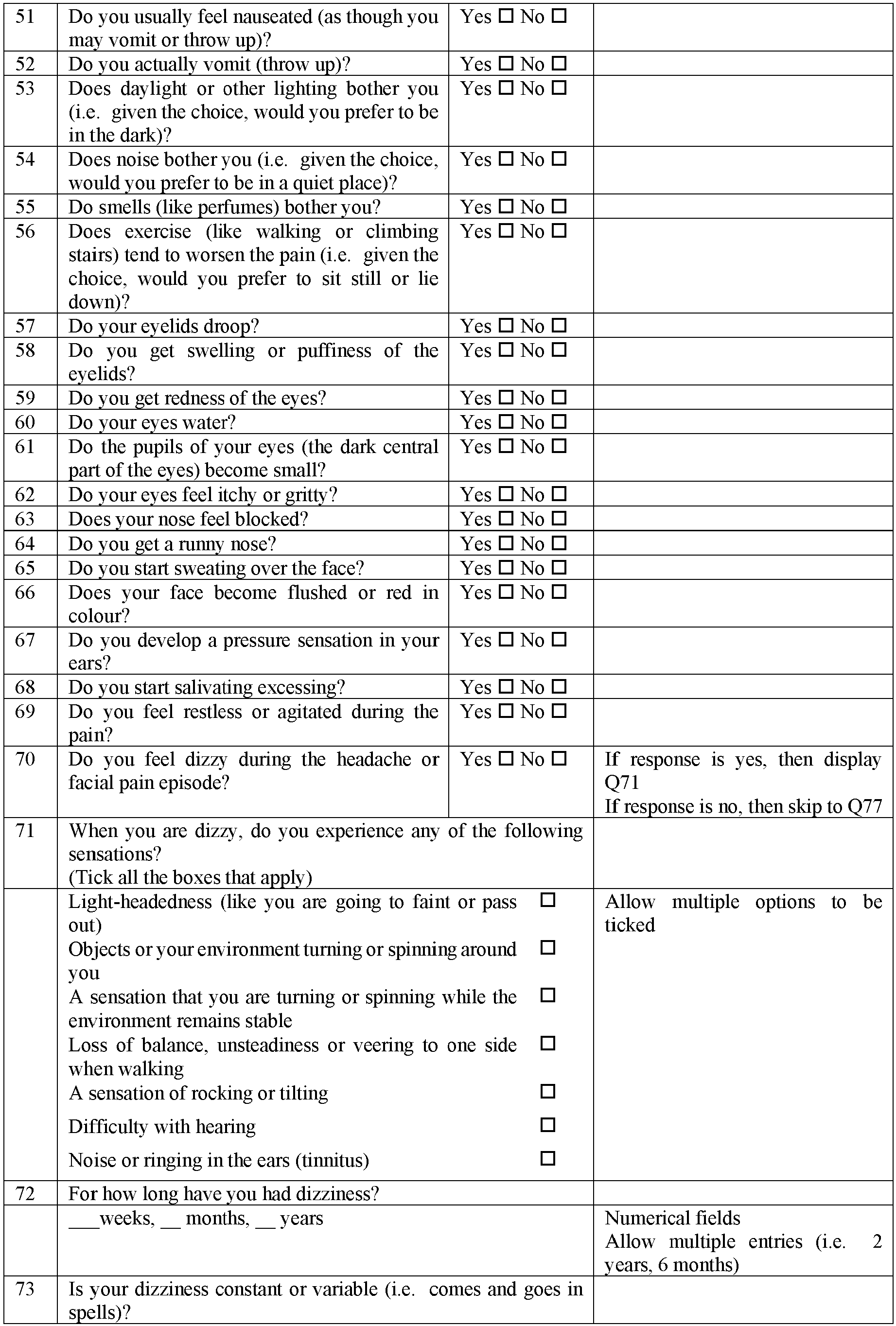

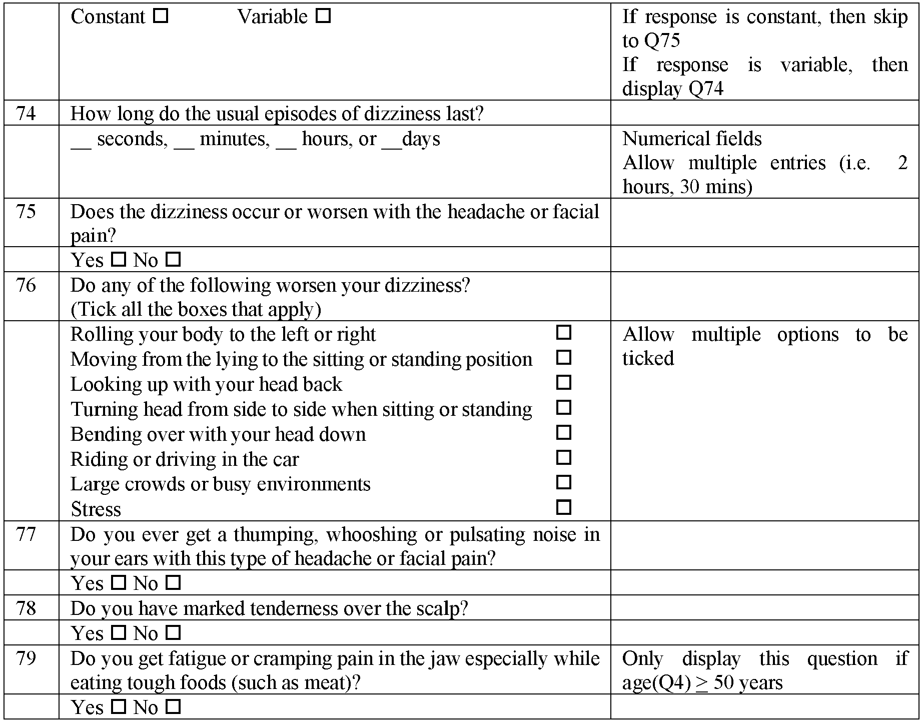

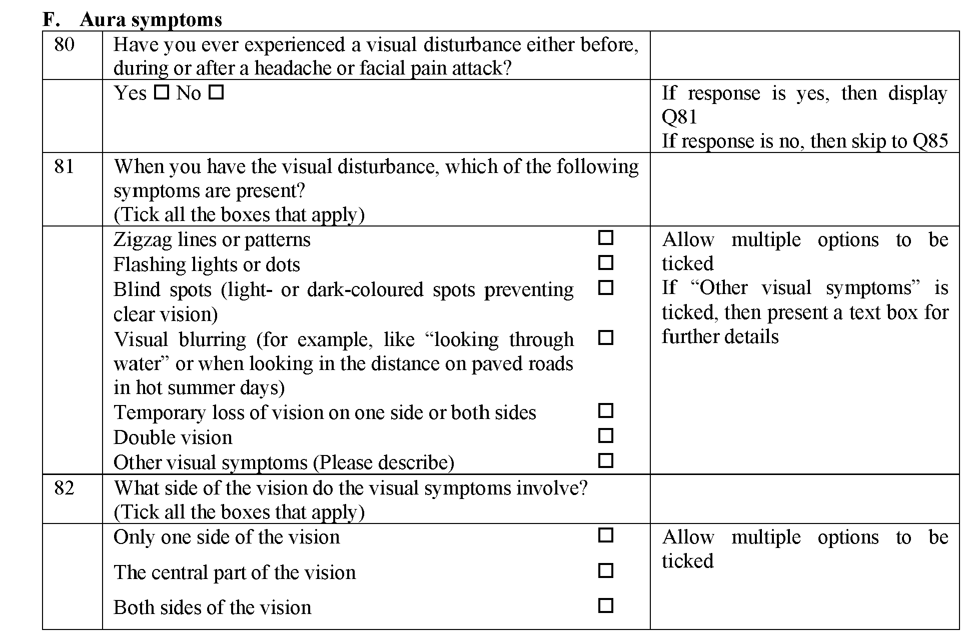

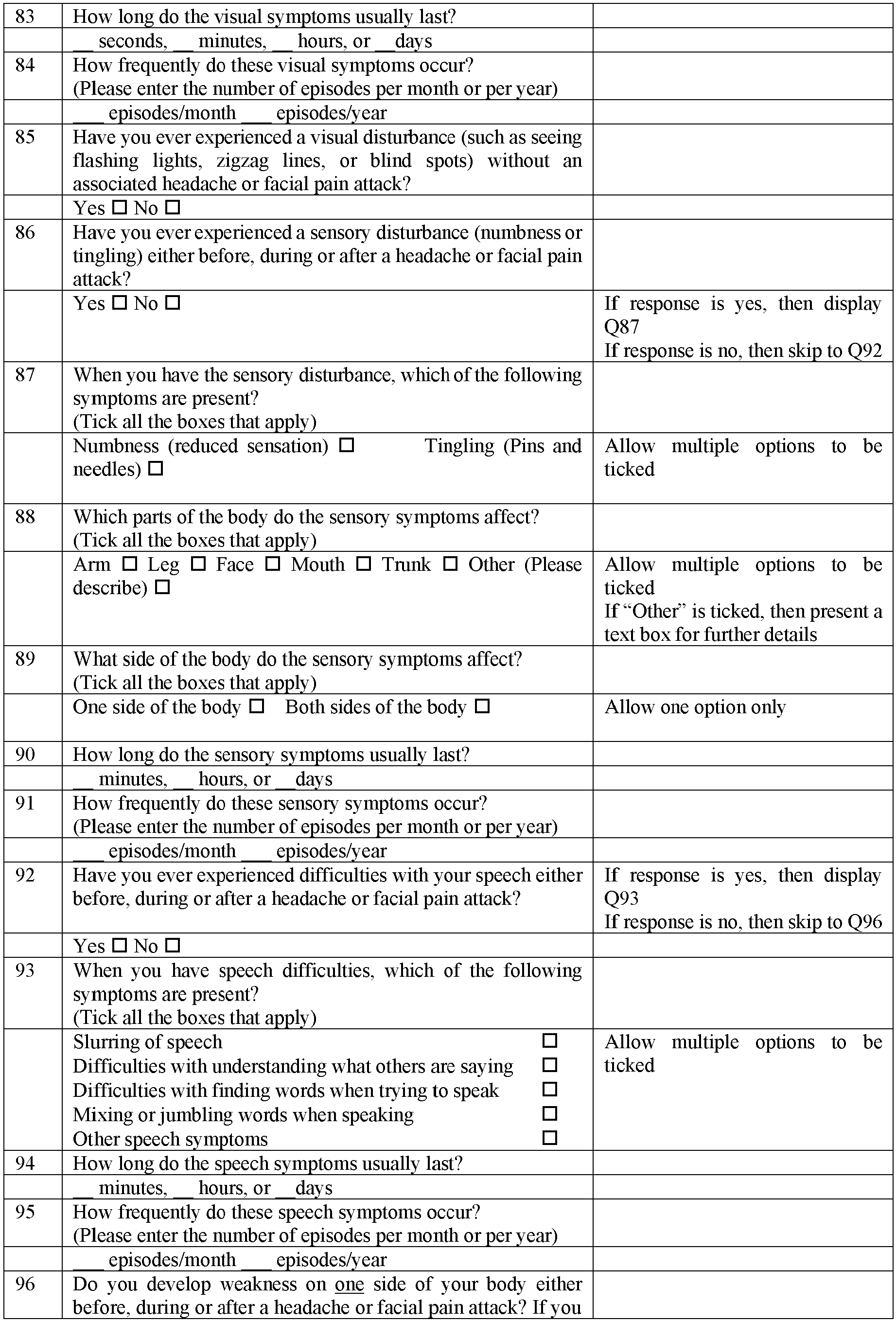

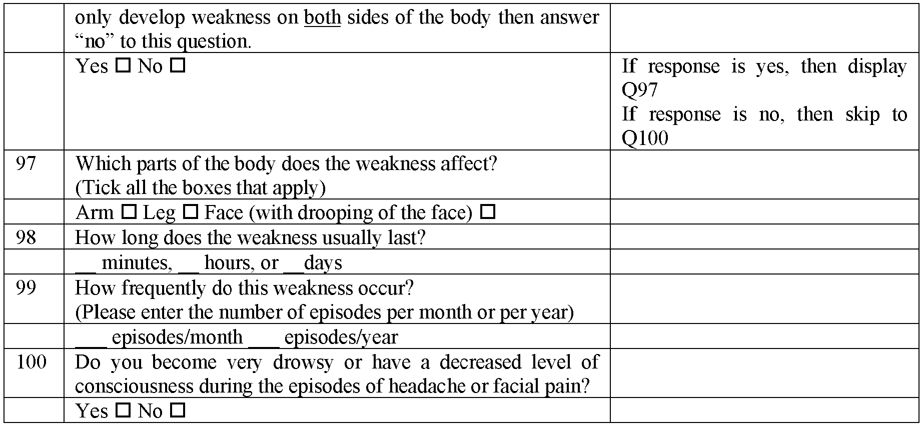

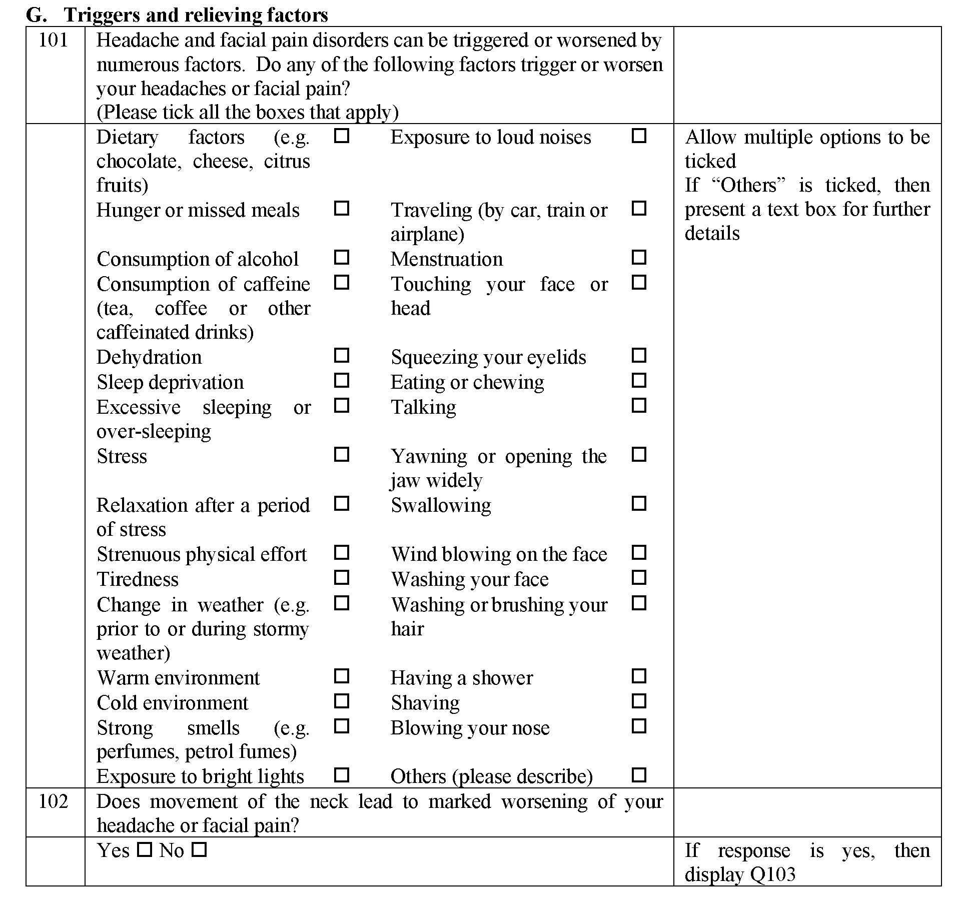

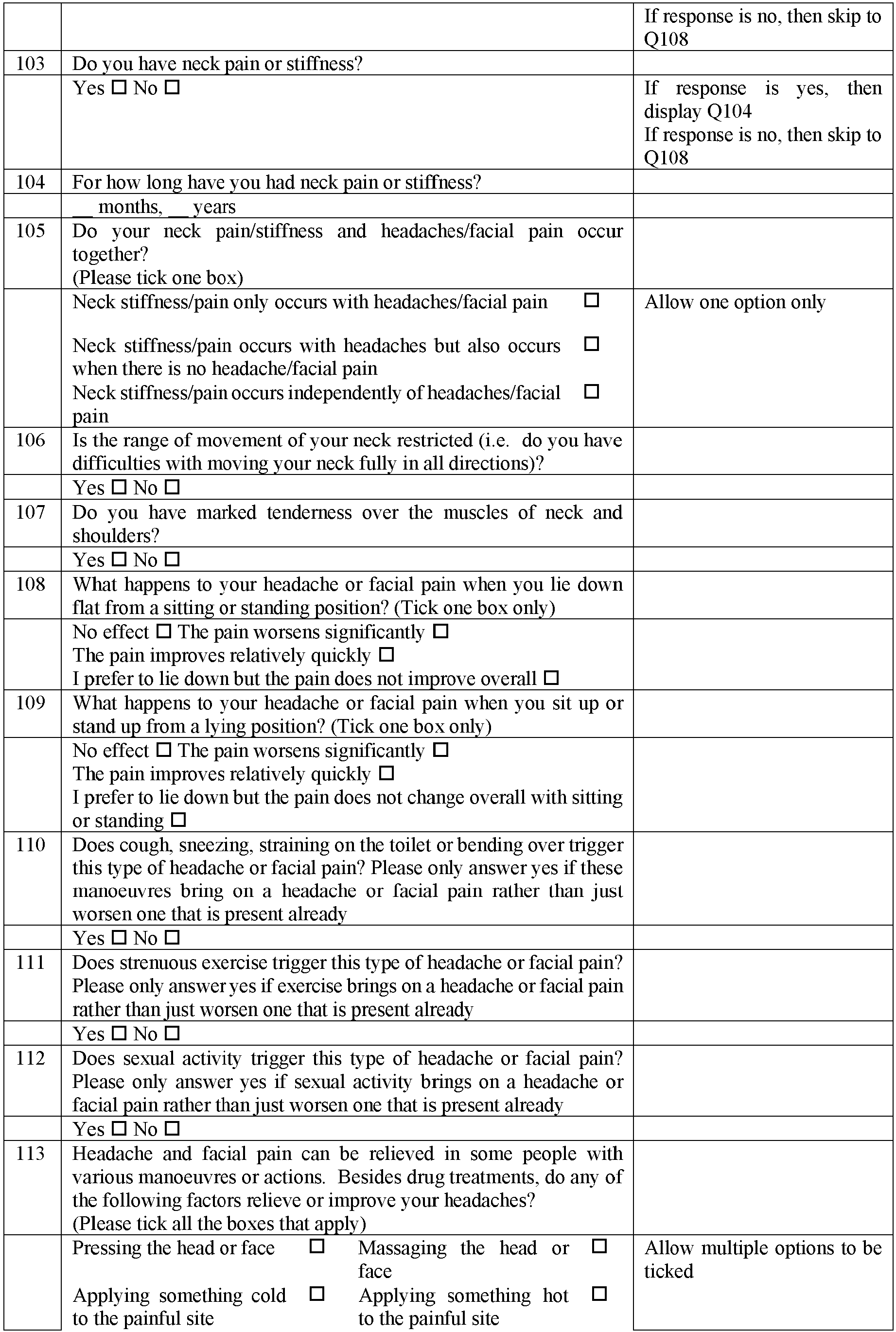

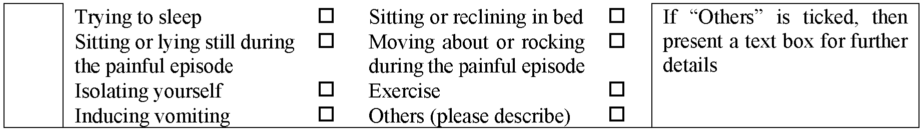

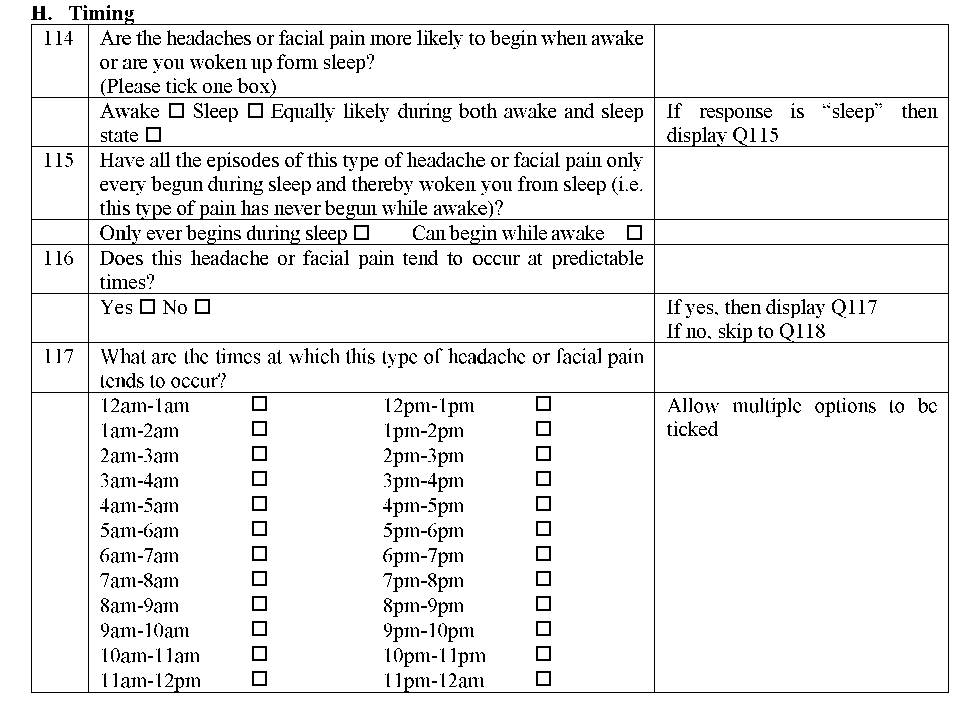

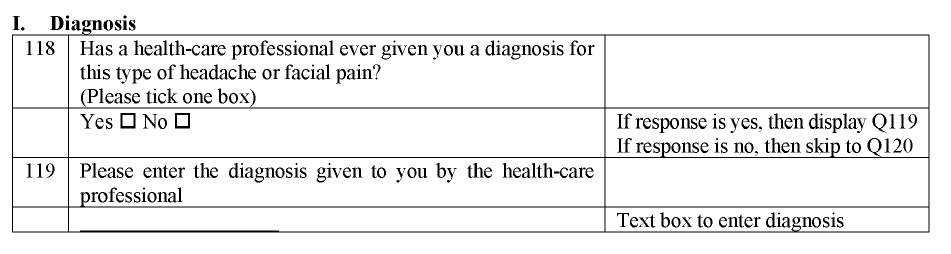

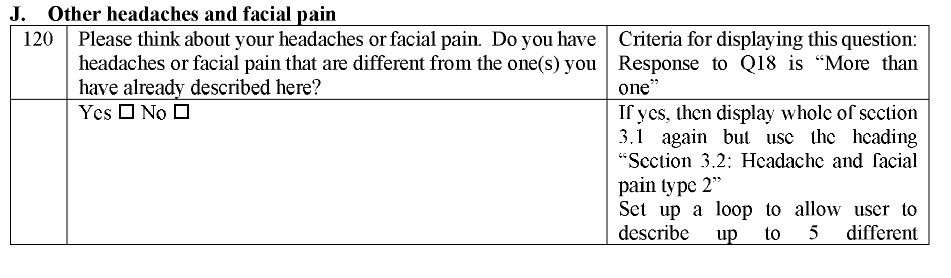

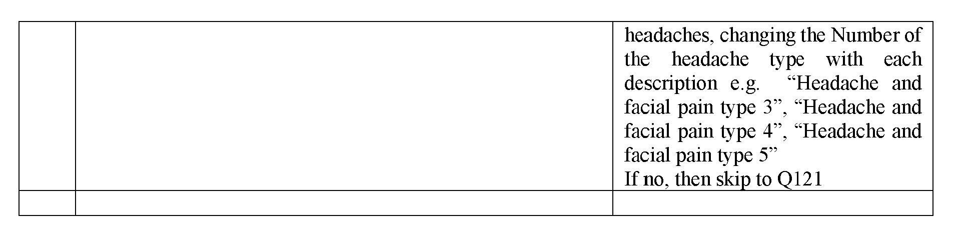

### SECTION 4. TREATMENTS

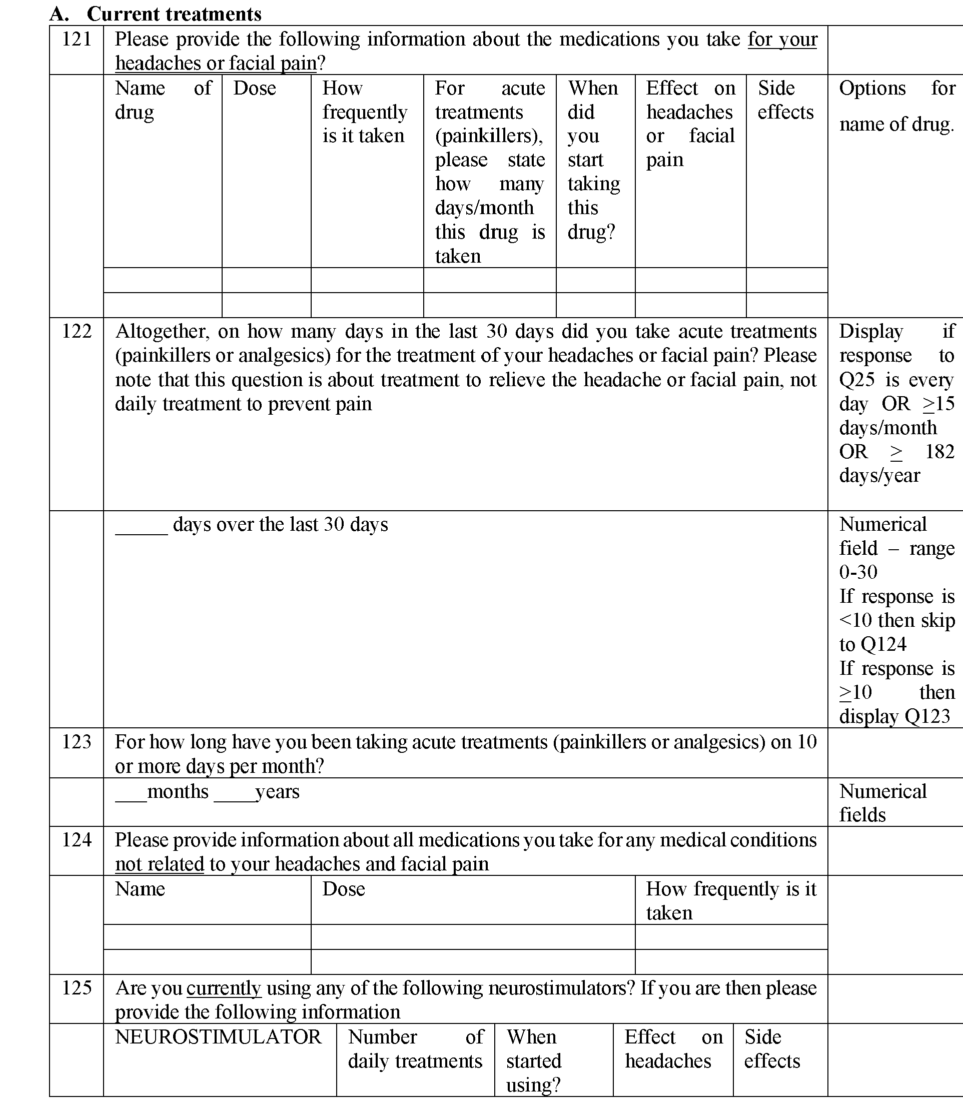

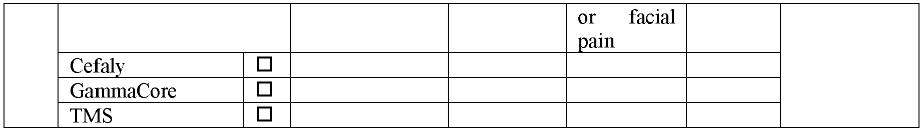

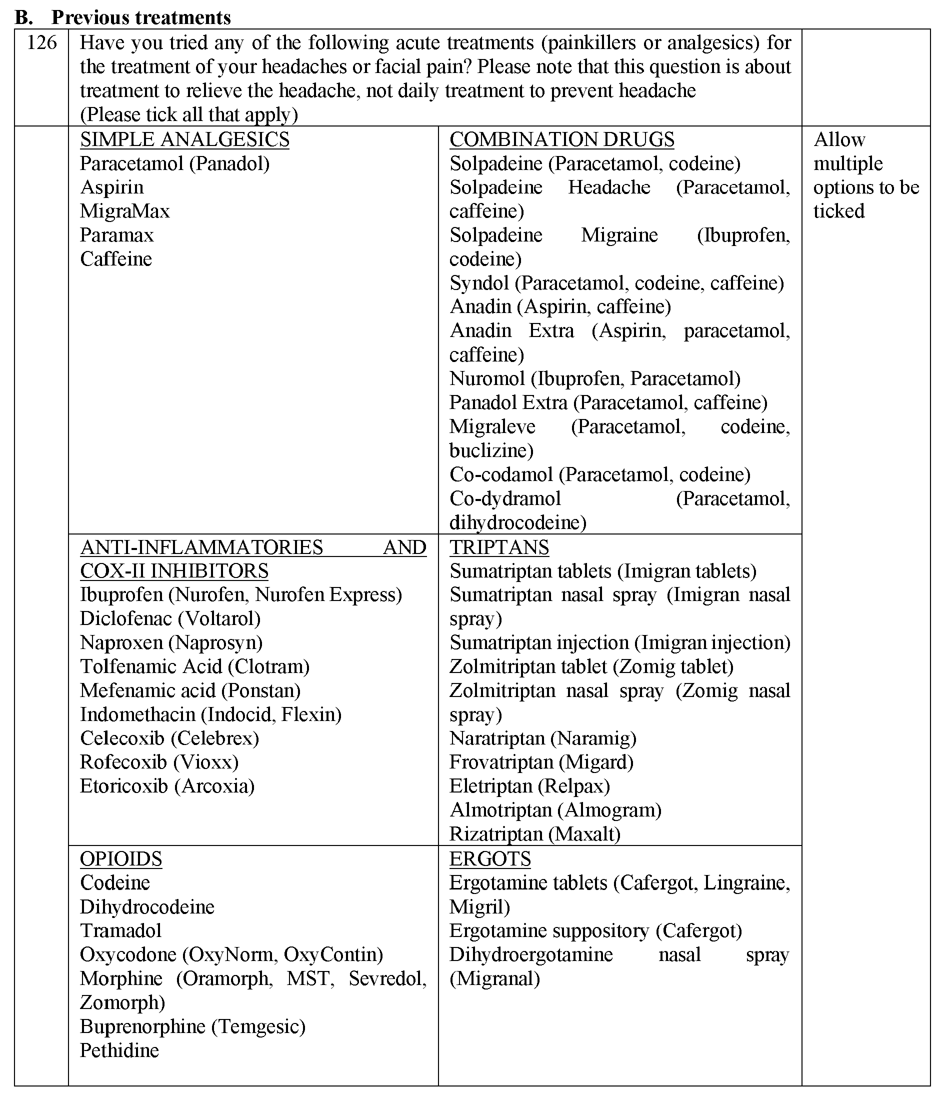

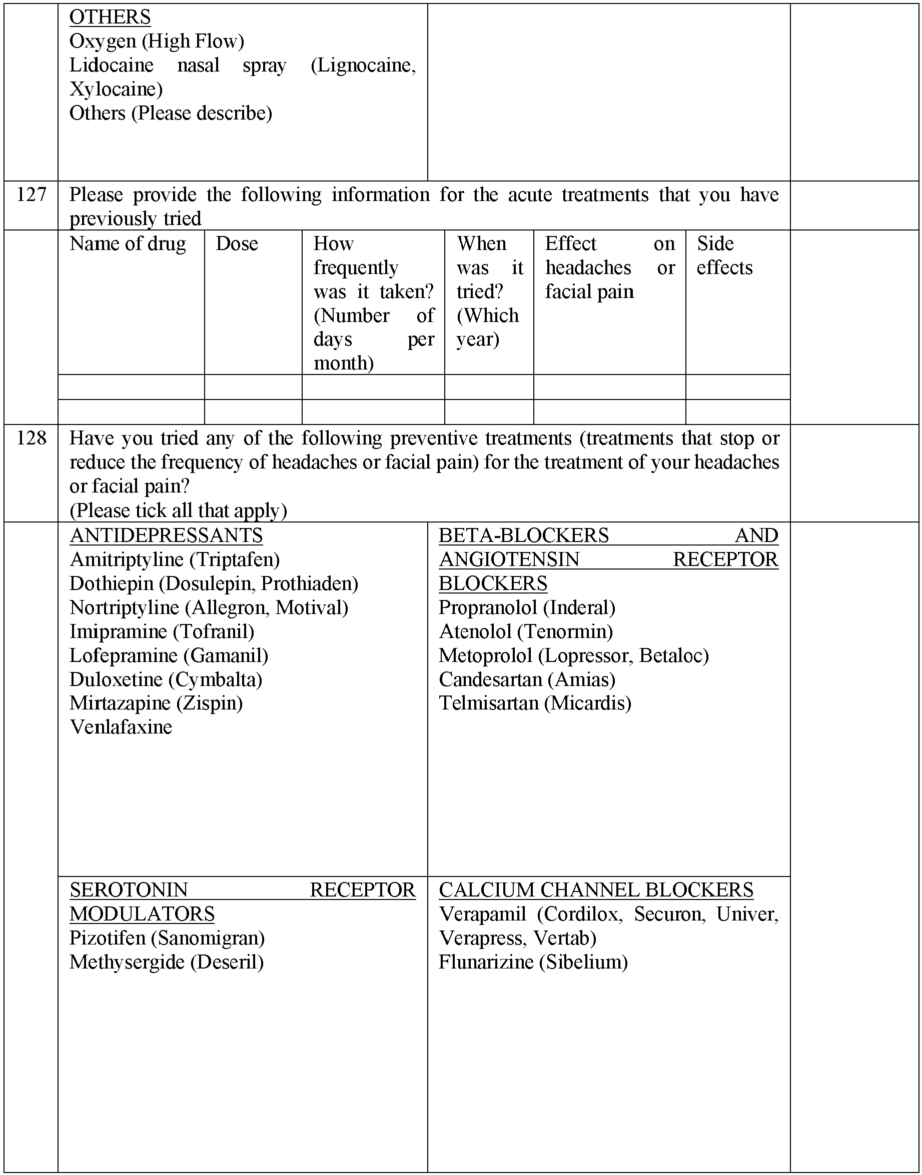

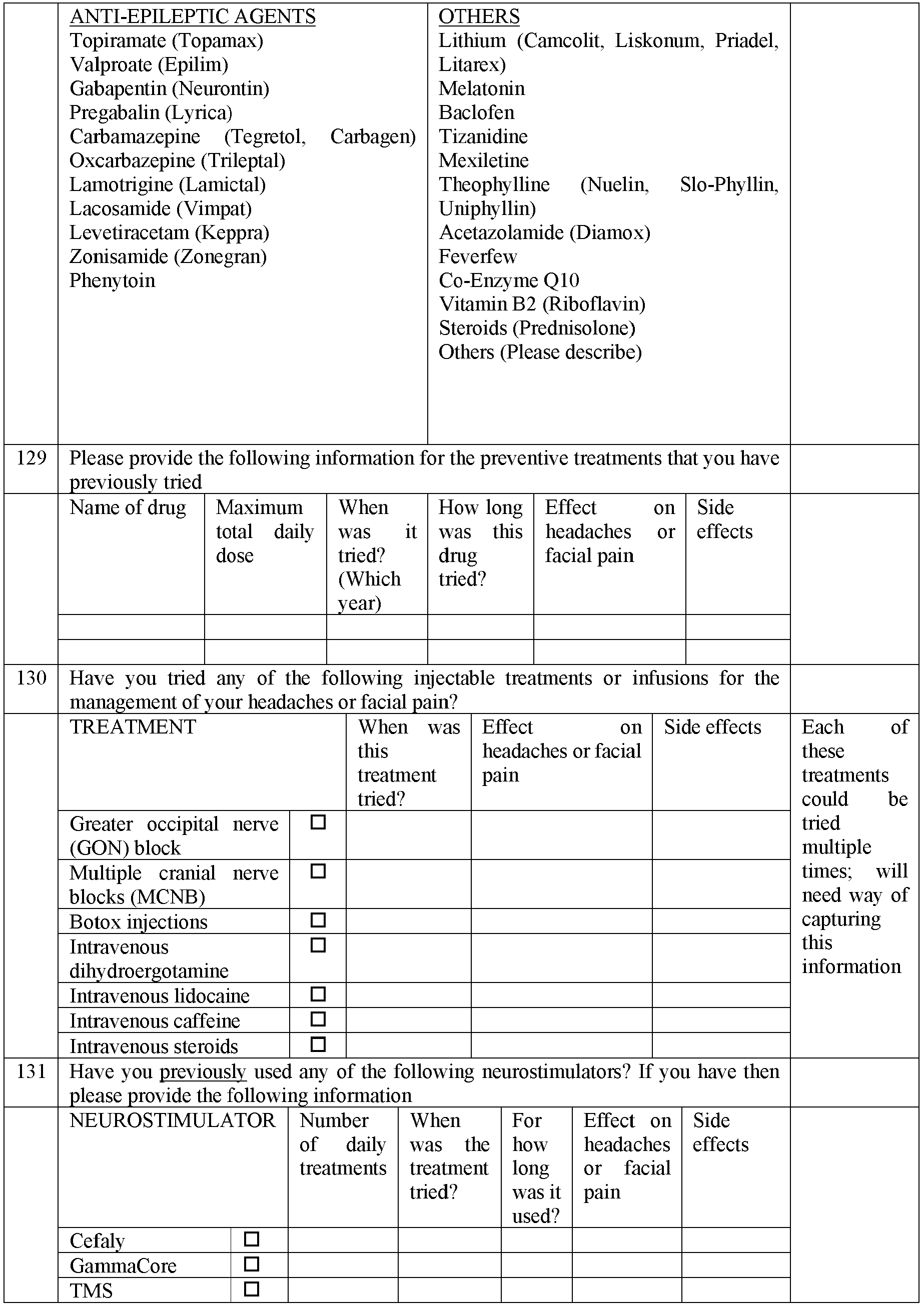

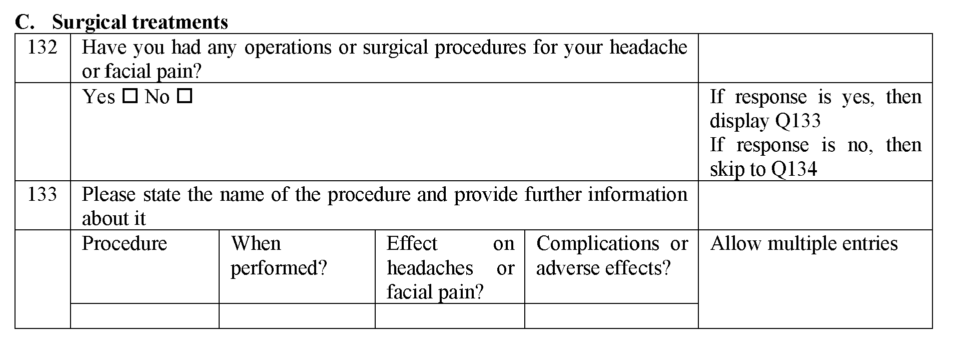

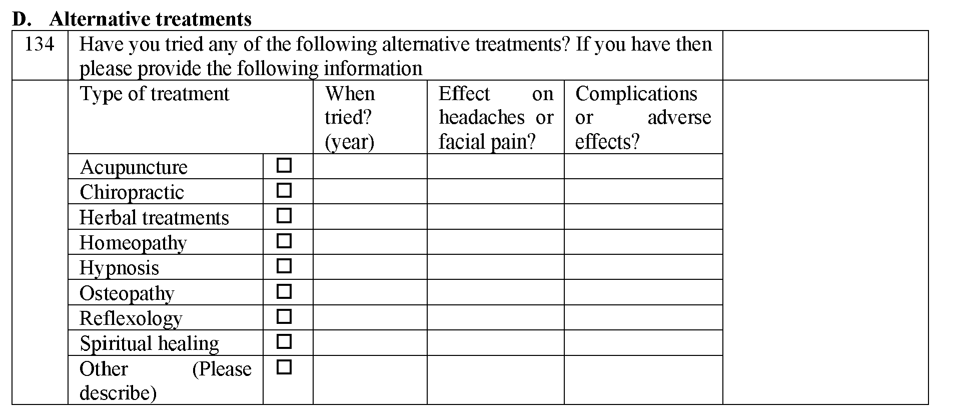

### SECTION 5. PAST MEDICAL HISTORY

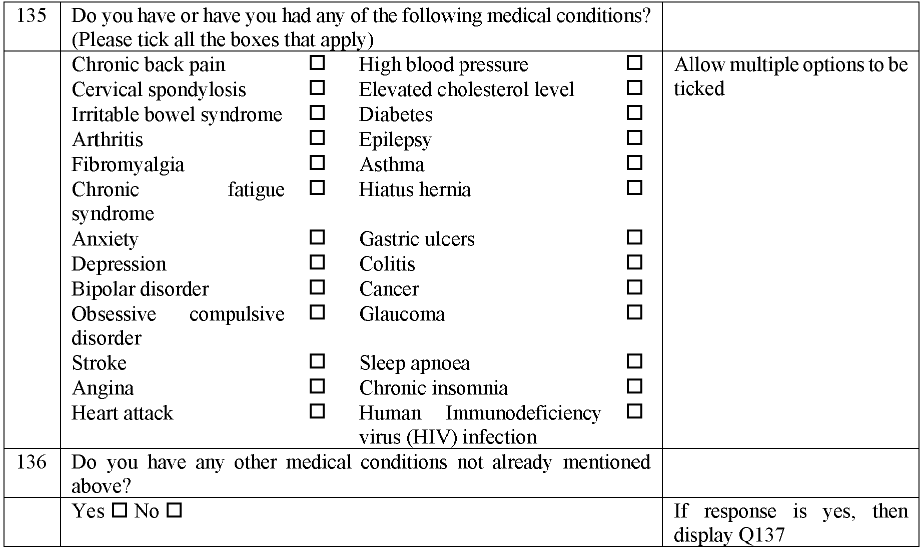

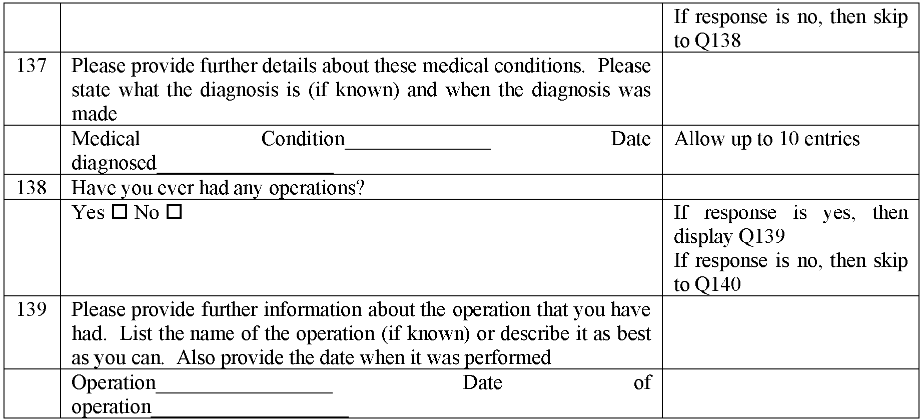

### SECTION 6. FAMILY HISTORY AND SOCIAL FACTORS

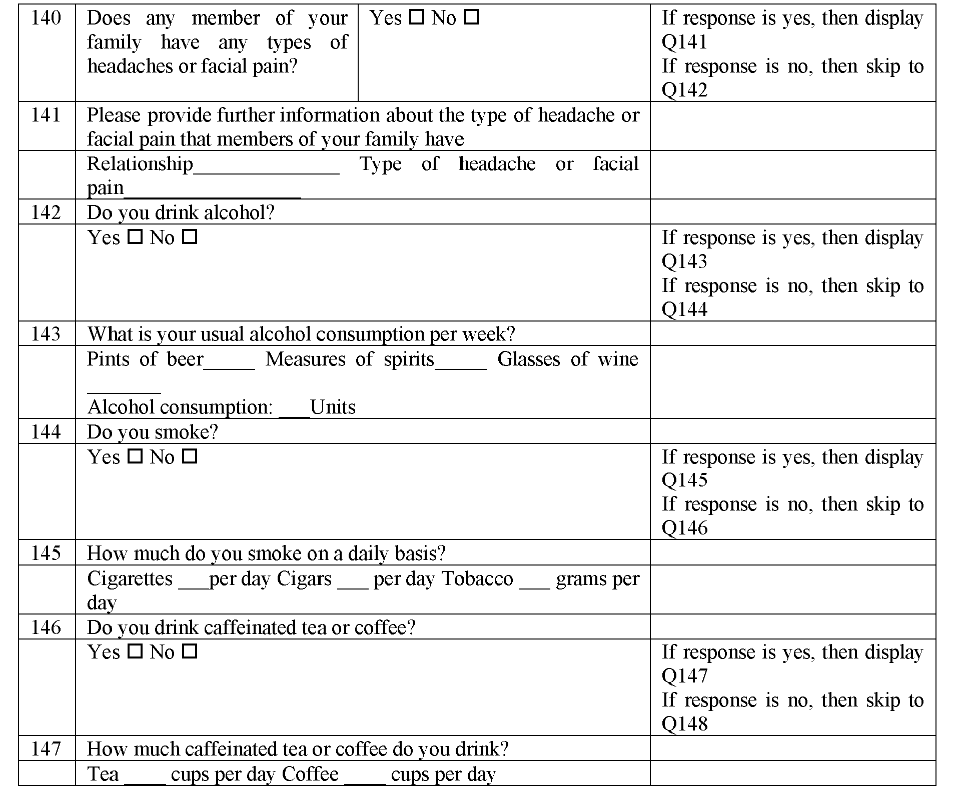

### SECTION 7. INVESTIGATIONS

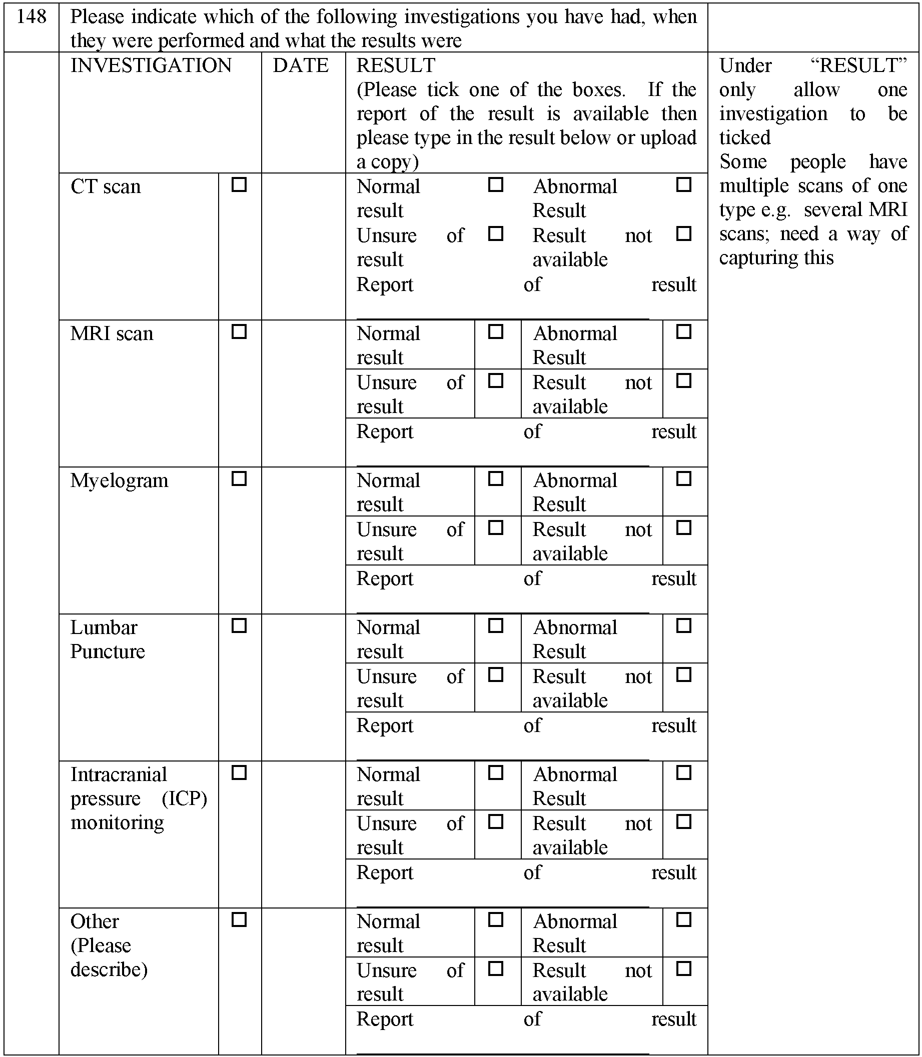

### SECTION 8. DISABILITY SCORES

The disability scores already provided need to be made available in this section.

Need a way for administrator to control which of the disability scores are displayed

### SECTION 9. EXAMINATION FINDINGS (ONLY DISPLAY TO ADMINISTRATOR)

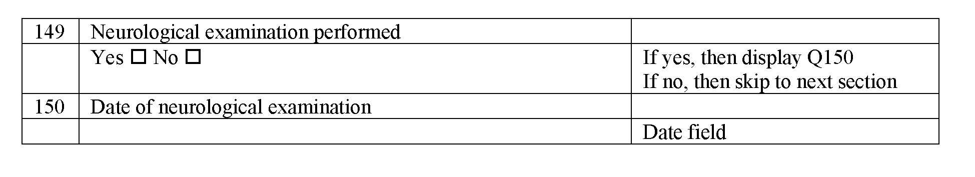

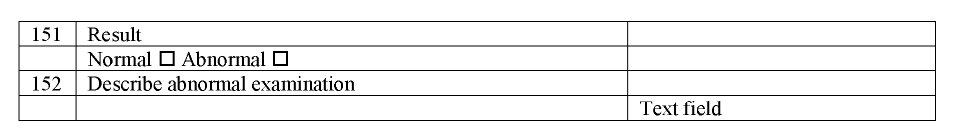

### SUMMARY

For each Headache/Facial Pain type the following data needs to be displayed. Display number of tables depending on how many types of headaches have been described by user

| **Headache type 1** | | |
|---|---|---|
| **Health-professional diagnosis** | | Populate data in this field from Q116 |
| **Headache Tracker differential diagnosis** | | Differential diagnosis to be populated from the algorithm |

| **Headache type 2** | | |
|---|---|---|
| **Health-professional diagnosis** | | |
| **Headache Tracker differential diagnosis** | | |

### Disability scores

| **Date** | **MIDAS** | **HIT-6** | **HAD-A** | **HAD-D** |
|---|---|---|---|---|
| | | | | |
| | | | | |
| | | | | |

Need to be able to display the results of all the disability scores in this table.

When disability scores repeated on the App or on website, then the scores need to be recorded on the different dates

### APPENDIX 2 - ALGORITHMS

### Algorithm 1: Migraine

| Algorith m | Questio n | Response | Value | Calculate 1^{st} intermediate score (FIS) | Calculate 2^{nd} intermediate score (SIS) | Final score | Diagnosis |
|---|---|---|---|---|---|---|---|
| A | 35b | <4 hours | 0 | | | 0 | |
| | | >3 days (72 hours) | 0 | | | 0 | |
| | | ≥4hours and ≤3 days | 1 | | | 1 | |
| B | 45 | Right side only | 1 | 45+44+42+56 ≥2 | | 1 | |
| | | Left side only | 1 | | | | |
| | | At any one time, the pain is only on one side of the head or face but alternates between the right and left sides | 1 | | | | |
| | | The pain is sometimes on one side of the head or face and at other times on both sides of the head or face at the same time | 1 | | | | |
| | | The pain is always on both sides of the head or face | 0 | 45+44+42+56 <2 | | 0 | |
| | 44 | Pulsating and/or throbbing ticked | 1 | | | | |
| | | Pulsating AND throbbing not ticked | 0 | | | | |
| | 42 | Mild | 0 | | | | |
| | | Moderate | 1 | | | | |
| | | Severe | 1 | | | | |
| | | Very severe | 1 | | | | |
| | 56 | Yes | 1 | | | | |
| | | No | 0 | | | | |
| C | 51 | Yes | 1 | 51+52≥1: Score 1 | | | |
| | | No | 0 | | | | |
| | 52 | Yes | 1 | 51+52=0: Score 0 | Sum FIS≥1 | 1 | |
| | | No | 0 | | | | |
| | 53 | Yes | 1 | 53+54=2: Score 1 | Sum FIS=0 | 0 | |
| | | No | 0 | | | | |
| | 54 | Yes | 1 | 53+54≤1: Score 0 | | | |
| | | No | 0 | | | | |
| D | | | | | | If A+B+C=3 | |
| | | | | | | Go to "E" | |
| | | | | | | If A+B+C<3 | |
| | | | | | | Go to Algorithm 2 | |
| E | 25 | ≥15 days/month or ≥182 days/ year | 1 | 25+26=2 | | 1 | Chronic Migraine |
| | | <15 days/month or < 182 days/ year | 0 | | | | |
| | 26 | ≥3 months | 1 | 25+26<2 | | 0 | |
| | | <3 months | 0 | | | | Episodic Migraine |
| | | No response | 0 | | | | |
| | | | | | | | After making one of these diagnoses, go to Algorithm 5 |

### Algorithm 2: Tension-Type Headache (TTH)

| Algorit hm | Questio n | Response | Value | Calculate 1^{st} intermediate score (FIS) | Calculate 2^{nd} intermediate score (SIS) | Final score | Diagnosis |
|---|---|---|---|---|---|---|---|
| A | 35b | <30 mins | 0 | | | 0 | |
| | | >7 days | 0 | | | 0 | |
| | | ≥30 mins and ≤7 days | 1 | | | 1 | |
| B | 45 | Right side only | 0 | 45+44+42+56≥2 | | 1 | |
| | | Left side only | 0 | | | | |
| | | At any one time, the pain is only on one side of the head or face but alternates between the right and left sides | 0 | | | | |
| | | The pain is sometimes on one side of the head or face and at other times on both sides of the head or face at the same time | 1 | | | | |
| | | The pain is always on both sides of the head or face | 1 | 45+44+42+56<2 | | 0 | |
| | 44 | (Tightening and/or pressure feeling ticked) and (Pulsating AND throbbing not ticked) | 1 | | | | |
| | | Tightening AND pressure feeling not ticked | 0 | | | | |
| | 42 | Mild | 1 | | | | |
| | | Moderate | 1 | | | | |
| | | Severe | 0 | | | | |
| | | Very severe | 0 | | | | |
| | 56 | Yes | 0 | | | | |
| | | No | 1 | | | | |
| C | 51 | Yes | 1 | 51+52≥1: Score 0 | Sum FIS=2 | 1 | |
| | | No | 0 | | | | |
| | 52 | Yes | 1 | 51+52=0: Score 1 | | | |
| | | No | 0 | | | | |
| | 53 | Yes | 1 | 53+54=2: Score 0 | Sum FIS<2 | 0 | |
| | | No | 0 | | | | |
| | 54 | Yes | 1 | 53+54≤1: Score 1 | | | |
| | | No | 0 | | | | |
| D | | | | | | If A+B+C=3 Go to "E" | |
| | | | | | | If A+B+C<3 go to Algorithm 3 | |
| E | 25 | ≥15 days/month or ≥182 days/ year | 1 | 25+26=2 | | 1 | Chronic Tension-Type Headache |
| | | <15 days/month or < 182 days/ year | 0 | | | | |
| | 26 | ≥3 months | 1 | 25+26<2 | | 0 | |
| | | <3 months | 0 | | | | Episodic Tension-Type Headache |
| | | No response | 0 | | | | |
| | | | | | | | After making one of these diagnoses, go to Algorithm 5 |

### Algorithm 3: Probable Migraine

| Algorith m | Quest ion | Response | Value | Calculate 1^{st} intermediate score (FIS) | Calculate 2^{nd} intermediate score (SIS) | Final score | Diagnosis |
|---|---|---|---|---|---|---|---|
| A | 35b | <4 hours | 0 | | | 0 | |
| | | >3 days (72 hours) | 0 | | | 0 | |
| | | ≥4hours and ≤3 days | 1 | | | 1 | |
| B | 45 | Right side only | 1 | 45+44+42+56≥2 | | 1 | |
| | | Left side only | 1 | | | | |
| | | At any one time, the pain is only on one side of the head or face but alternates between the right and left sides | 1 | | | | |
| | | The pain is sometimes on one side of the head or face and at other times on both sides of the head or face at the same time | 1 | | | | |
| | | The pain is always on both sides of the head or face | 0 | 45+44+42+56<2 | | 0 | |
| | 44 | Pulsating and/or throbbing ticked | 1 | | | | |
| | | Pulsating AND throbbing not ticked | 0 | | | | |
| | 42 | Mild | 0 | | | | |
| | | Moderate | 1 | | | | |
| | | Severe | 1 | | | | |
| | | Very severe | 1 | | | | |
| | 56 | Yes | 1 | | | | |
| | | No | 0 | | | | |
| C | 51 | Yes | 1 | 51+52≥1: Score 1 | | | |
| | | No | 0 | | | | |
| | 52 | Yes | 1 | 51+52=0: Score 0 | Sum FIS≥1 | 1 | |
| | | No | 0 | | | | |
| | 53 | Yes | 1 | 53+54=2: Score 1 | Sum FIS=0 | 0 | |
| | | No | 0 | | | | |
| | 54 | Yes | 1 | 53+54≤1: Score 0 | | | |
| | | No | 0 | | | | |
| D | | | | | | A+B+C=2 | Probable Migraine After making this diagnoses, go to Algorithm 5 |
| | | | | | | If A+B+C<2 Go to Algorithm 4 | |

### Algorithm 4: Probable Tension-Type Headache (TTH)

| Algorit hm | Quest ion | Response | Value | Calculate 1^{st} intermediate score (FIS) | Calculate 2^{nd} intermediate score (SIS) | Final score | Diagnosis |
|---|---|---|---|---|---|---|---|
| A | 35b | <30 mins | 0 | | | 0 | |
| | | >7 days | 0 | | | 0 | |
| | | ≥30 mins and ≤7 days | 1 | | | 1 | |
| B | 45 | Right side only | 0 | | | | |
| | | Left side only | 0 | | | | |
| | | At any one time, the pain is only on one side of the head or face but alternates between the right and left sides | 0 | | | | |
| | | The pain is sometimes on one side of the head or face and at other times on both sides of the head or face at the same time | 1 | 45+44+42+56≥2 | | 1 | |
| | | The pain is always on both sides of the head or face | 1 | 45+44+42+56<2 | | 0 | |
| | 44 | (Tightening and/or pressure feeling ticked) and (Pulsating AND throbbing not ticked) | 1 | | | | |
| | | Tightening AND pressure feeling not ticked | 0 | | | | |
| | 42 | Mild | 1 | | | | |
| | | Moderate | 1 | | | | |
| | | Severe | 0 | | | | |
| | | Very severe | 0 | | | | |
| | 56 | Yes | 0 | | | | |
| | | No | 1 | | | | |
| C | 51 | Yes | 1 | 51+52≥1: Score 0 | | | |
| | | No | 0 | | | | |
| | 52 | Yes | 1 | 51+52=0: Score 1 | Sum FIS=2 | 1 | |
| | | No | 0 | | | | |
| | 53 | Yes | 1 | 53+54=2: Score 0 | Sum FIS<2 | 0 | |
| | | No | 0 | | | | |
| | 54 | Yes | 1 | 53+54≤1: Score 1 | | | |
| | | No | 0 | | | | |
| D | | | | | | A+B+C=2 | Probable Tension-Type Headache |
| | | | | | | | After making this diagnosis, go to Algorithm 5 |
| | | | | | | If A+B+C<2 Go to Algorithm 5 | |

### Algorithm 5: New Daily Persistent Headache

| Algorith m | Quest ion | Response | Value | Calculate 1^{st} intermediate score (FIS) | Calculate 2^{nd} intermediate score (SIS) | Final score | Diagnosis |
|---|---|---|---|---|---|---|---|
| A | 24 | =30 day/month or ≥364 day/year | 1 | | | | |
| | | <30days/month or <364 days/year | 0 | | | | |
| | 25 | =30 day/month or ≥364 day/year | 1 | | | | |
| | | <30days/month or <364 days/year | 0 | 24+25+27+28=4 | | 1 | |
| | 27 | Yes | 1 | | | | |
| | | No | 0 | 24+25+27+28<4 | | 0 | |
| | | No response | 0 | | | | |
| | 28 | Yes | 1 | | | | |
| | | No | 0 | | | | |
| | | No response | 0 | | | | |
| B | 19 | ≥3months | 1 | | | 1 | |
| | | <3months | 0 | | | 0 | |
| C | | | | | | A+B=2 | New Daily Persistent Headache |
| | | | | | | | After making this diagnosis go to Algorithm 6 |
| | | | | | | A+B<2 Go to Algorithm 6 | |

### Algorithm 6: Hemicrania Continua

| Algorit hm | Questio n | Response | Value | Calculate 1^{st} intermediate score (FIS) | Calculate 2^{nd} intermediate score (SIS) | Final score | Diagnosis |
|---|---|---|---|---|---|---|---|
| A | 45 | Right side only | 1 | | | 1 | |
| | | Left side only | 1 | | | 1 | |
| | | At any one time, the pain is only on one side of the head or face but alternates between the right and left sides | 1 | | | 1 | |
| | | The pain is sometimes on one side of the head or face and at other times on both sides of the head or face at the same time | 0 | | | 0 | |
| | | The pain is always on both sides of the head or face | 0 | | | 0 | |
| B | 35a | Never goes away | 1 | 35a+35b=1 | | 1 | |
| | | There are pain free periods between the episodes of pain | 0 | | | | |
| | 35b | ≥8 hours | 1 | 35a+35b=0 | | 0 | |
| | | <8 hours | 0 | | | | |
| | | No response | 0 | | | | |
| C | 43 | Mild | 0 | | | 0 | |
| | | Moderate | 1 | | | 1 | |
| | | Severe | 1 | | | 1 | |
| | | Very severe | 1 | | | 1 | |
| D | 25 | ≥15 days/month or ≥182 days/ year | 1 | 25+26=2 | | 1 | |
| | | <15 days/month or <182 days/ year | 0 | 25+26<2 | | 0 | |
| | 26 | ≥3 months | 1 | | | | |
| | | <3 months | 0 | | | | |
| | | No response | 0 | | | | |
| E | 24 | ≥15 days/month or ≥182 days/ year | 1 | 24+25+19=3 | | 1 | |
| | | <15 days/month or <182 days/ year | 0 | | | | |
| | 25 | ≥15 days/month or ≥182 days/ year | 1 | 24+25+19<3 | | 0 | |
| | | <15 days/month or <182 days/ year | 0 | | | | |
| | 19 | ≥3 months | 1 | | | | |
| | | <3 months | 0 | | | | |
| F | 57 | Yes | 1 | 57+58+59+60+61+63+64+65+66+67+6 9≥1 | | 1 | |
| | | No | 0 | | | | |
| | 58 | Yes | 1 | | | | |
| | | No | 0 | | | | |
| | 59 | Yes | 1 | | | | |
| | | No | 0 | | | | |
| | 60 | Yes | 1 | | | | |
| | | No | 0 | | | | |
| | 61 | Yes | 1 | | | 0 | |
| | | No | 0 | 57+58+59+60+61+63+64+65+66+67+6 9=0 | | | |
| | 63 | Yes | 1 | | | | |
| | | No | 0 | | | | |
| | 64 | Yes | 1 | | | | |
| | | No | 0 | | | | |
| | 65 | Yes | 1 | | | | |
| | | No | 0 | | | | |
| | 66 | Yes | 1 | | | | |
| | | No | 0 | | | | |
| | 67 | Yes | 1 | | | | |
| | | No | 0 | | | | |
| | 69 | Yes | 1 | | | | |
| | | No | 0 | | | | |
| G | | | | | | A+B+C+D +E+F=5 | Hemicrania Continua |
| | | | | | | | After making this diagnosis go to Algorithm 7 |
| | | | | | | A+B+C+D +E+F<5 Go to Algorithm 7 | |

### Algorithm 7: Nummular Headache

| Algorithm | Question | Response | Value | Calculate 1^{st} intermediate score (FIS) | Calculate 2^{nd} intermediate score (SIS) | Final score | Diagnosis |
|---|---|---|---|---|---|---|---|
| A | 47 | Same location | 1 | 47+48+49+50=4 | | 1 | |
| | | Variable location | 0 | | | | |
| | | No response | 0 | | | | |
| | 48 | Yes | 1 | | | | |
| | | No | 0 | | | | |
| | 49 | Yes | 1 | 47+48+49+50<4 | | 0 | |
| | | No | 0 | | | | |
| | 50 | <1cm | 0 | | | | |
| | | >6cm | 0 | | | | |
| | | ≥1 and ≤6cm | 1 | | | | |
| B | | | | | | A=1 | Nummular Headache |
| | | | | | | | After making this diagnosis go to Algorithm 8 |
| | | | | | | A=0 Go to Algorithm 8 | |

### Algorithm 8: Cluster Headache

| Algorit hm | Questi on | Response | Value | Calculate 1^{st} intermediate score (FIS) | Calculate 2^{nd} intermediate score (SIS) | Final score | Diagnosis |
|---|---|---|---|---|---|---|---|
| A | 42 | Mild | 0 | | | | |
| | | Moderate | 0 | | | | |
| | | Severe | 1 | | | | |
| | | Very severe | 1 | | | | |
| | 45 | Right side only | 1 | 42+45+46+35b=4 | | 1 | |
| | | Left side only | 1 | | | | |
| | | At any one time, the pain is only on one side of the head or face but alternates between the right and left sides | 1 | | | | |
| | | The pain is sometimes on one side of the head or face and at other times on both sides of the head or face at the same time | 0 | 42+45+46+35b≤4 | | 0 | |
| | | The pain is always on both sides of the head or face | 0 | | | | |
| | 46 | Forehead and/or eye and/or temple ticked | 1 | | | | |
| | | Forehead AND eye AND temple not ticked | 0 | | | | |
| | 35b | <15 minutes | 0 | | | | |
| | | >3 hours | 0 | | | | |
| | | ≥15 minutes and ≤3 hours | 1 | | | | |
| | | | | | | | |
| B | 57 | Yes | 1 | | | | |
| | | No | 0 | | | | |
| | 58 | Yes | 1 | | | | |
| | | No | 0 | | | | |
| | 59 | Yes | 1 | | | | |
| | | No | 0 | | | | |
| | 60 | Yes | 1 | 57+58+59+60+61+63+64+ 65+66+67+69≥1 | | 1 | |
| | | No | 0 | | | | |
| | 61 | Yes | 1 | | | | |
| | | No | 0 | 57+58+59+60+61+63+64+ 65+66+67+69=0 | | 0 | |
| | 63 | Yes | 1 | | | | |
| | | No | 0 | | | | |
| | 64 | Yes | 1 | | | | |
| | | No | 0 | | | | |
| | 65 | Yes | 1 | | | | |
| | | No | 0 | | | | |
| | 66 | Yes | 1 | | | | |
| | | No | 0 | | | | |
| | 67 | Yes | 1 | | | | |
| | | No | 0 | | | | |
| | 69 | Yes | 1 | | | | |
| | | No | 0 | | | | |
| C | 40 | <0.5 | 0 | | | 0 | |
| | | >8 | 0 | | | 0 | |
| | | ≥0.5 AND ≤8 | 1 | | | 1 | |
| D | | | | | | If A+B+ C=3 Go to "E" | |
| | | | | | | If A+B+ C<3 Go to Algorit hm 9 | |
| E | 29 | <30 days or <1 month | 1 | | | 1 | Chronic cluster headache |
| | | >30 days or >1 month | 0 | | | 0 | Episodic cluster headache |
| | | | | | | | After making one of these two diagnoses, go to Algorithm 9 |

### Algorithm 9: Paroxysmal Hemicrania

| Algorith m | Questio n | Response | Valu e | Calculate 1^{st} intermediate score (FIS) | Calculate 2^{nd} intermediat e score (SIS) | Final score | Diagnosis |
|---|---|---|---|---|---|---|---|
| A | 42 | Mild | 0 | | | | |
| | | Moderate | 0 | | | | |
| | | Severe | 1 | | | | |
| | | Very severe | 1 | | | | |
| | 45 | Right side only | 1 | | | | |
| | | Left side only | 1 | | | | |
| | | At any one time, the pain is only on one side of the head or face but alternates between the right and left sides | 1 | 42+45+46+35b=4 | | 1 | |
| | | The pain is sometimes on one side of the head or face and at other times on both sides of the head or face at the same time | 0 | 42+45+46+35b≤4 | | 0 | |
| | | The pain is always on both sides of the head or face | 0 | | | | |
| | 46 | Forehead and/or eye and/or temple ticked | 1 | | | | |
| | | Forehead and/or eye and/or temple not ticked | 0 | | | | |
| | 35b | <2 minutes | 0 | | | | |
| | | >30 minutes | 0 | | | | |
| | | ≥2 minutes and ≤30 minutes | 1 | | | | |
| B | 57 | Yes | 1 | | | 1 | |
| | | No | 0 | | | | |
| | 58 | Yes | 1 | | | | |
| | | No | 0 | | | | |
| | 59 | Yes | 1 | | | | |
| | | No | 0 | | | | |
| | 60 | Yes | 1 | | | | |
| | | No | 0 | 57+58+59+60+61+63+64+65+ 66+67+69≥1 | | 0 | |
| | 61 | Yes | 1 | | | | |
| | | No | 0 | | | | |
| | 63 | Yes | 1 | | | | |
| | | No | 0 | 57+58+59+60+61+63+64+65+ 66+67+69=0 | | | |
| | 64 | Yes | 1 | | | | |
| | | No | 0 | | | | |
| | 65 | Yes | 1 | | | | |
| | | No | 0 | | | | |
| | 66 | Yes | 1 | | | | |
| | | No | 0 | | | | |
| | 67 | Yes | 1 | | | | |
| | | No | 0 | | | | |
| | 69 | Yes | 1 | | | | |
| | | No | 0 | | | | |
| C | 40 | <5 | 0 | | | 0 | |
| | | ≥5 | 1 | | | 1 | |
| D | | | | | | If A+B+C =3 Go to "E" | |
| | | | | | | If A+B+C <3 Go to Algorith m 10 | |
| E | 29 | <30 days or <1 month | 1 | | | 1 | Chronic paroxysmal hemicrania |
| | | ≥30 days or ≥1 month | 0 | | | 0 | Episodic paroxysmal hemicrania |
| | | | | | | | After making one of these two diagnoses, go to Algorithm 10 |

### Algorithm 10: Short-lasting unilateral neuralgiform headache attacks

| Algorit hm | Questi on | Response | Valu e | Calculate 1^{st} intermediate score (FIS) | Calculate 2^{nd} intermedia te score (SIS) | Final score | Diagnosis |
|---|---|---|---|---|---|---|---|
| A | 42 | Mild | 0 | 42+45+46+35b=4 | | 1 | |
| | | Moderate | 1 | | | | |
| | | Severe | 1 | | | | |
| | | Very severe | 1 | | | | |
| | 45 | Right side only | 1 | | | | |
| | | Left side only | 1 | | | | |
| | | At any one time, the pain is only on one side of the head or face but alternates between the right and left sides | 1 | | | | |
| | | The pain is sometimes on one side of the head or face and at other times on both sides of the head or face at the same time | 0 | 42+45+46+35b≤4 | | 0 | |
| | | The pain is always on both sides of the head or face | 0 | | | | |
| | 46 | Forehead and/or eye and/or temple and/or behind the temple and/or vertex and/or nose and/or cheek and/or chin and/or mouth and/or jaw and/or ear ticked | 1 | | | | |
| | | Forehead AND eye AND temple AND behind the temple AND vertex AND nose AND cheek AND chin AND mouth AND jaw AND ear not ticked | 0 | | | | |
| | | | | | | | |
| | 35b | <1 second | 0 | | | | |
| | | >10 minutes | 0 | | | | |
| | | ≥1 second and ≤10 minutes | 1 | | | | |
| B | 57 | Yes | 1 | 57+58+59+60+61+63+64+65 +66+67+69≥1 | | 1 | |
| | | No | 0 | | | | |
| | 58 | Yes | 1 | | | | |
| | | No | 0 | | | | |
| | 59 | Yes | 1 | | | | |
| | | No | 0 | | | | |
| | 60 | Yes | 1 | | | | |
| | | No | 0 | | | | |
| | 61 | Yes | 1 | | | 0 | |
| | | No | 0 | 57+58+59+60+61+63+64+65 +66+67+69=0 | | | |
| | 63 | Yes | 1 | | | | |
| | | No | 0 | | | | |
| | 64 | Yes | 1 | | | | |
| | | No | 0 | | | | |
| | 65 | Yes | 1 | | | | |
| | | No | 0 | | | | |
| | 66 | Yes | 1 | | | | |
| | | No | 0 | | | | |
| | 67 | Yes | 1 | | | | |
| | | No | 0 | | | | |
| | 69 | Yes | 1 | | | | |
| | | No | 0 | | | | |
| C | | | | | | If A+B= 2 Go to "D" | |
| | | | | | | If A+B< 2 Go to algori thm 11 | |
| D | 59 | Yes | 1 | | | | |
| | | No | 0 | 59+60=2 | | 1 | |
| | 60 | Yes | 1 | 59+60<2 | | 0 | |
| | | No | 0 | | | | |
| E | | | | | | D=1 | Short-Lasting Unilateral Neuralgifor m Headache with Conjunctiva 1 Injection and Tearing (SUNCT) |
| | | | | | | D=0 | Short-Lasting Unilateral Neuralgifor m Headache with Cranial Autonomic Symptoms (SUNA) |
| | | | | | | | After making one of these two diagnoses, go to Algorithm 11 |

### Algorithm 11: Hypnic Headache Algorithm

| Algorit hm | Questi on | Response | Value | Calculate 1^{st} intermediate score (FIS) | Calculate 2^{nd} intermediat e score (SIS) | Final score | Diagnosis |
|---|---|---|---|---|---|---|---|
| A | 115 | Only ever begins during sleep | 1 | | | 1 | |
| | | Can begin while awake | 0 | | | 0 | |
| | | No response | 0 | | | 0 | |
| B | 25 | Every day or ≥10 days/month or ≥120 days/year | 1 | 25+19=2 | | 1 0 | |
| | | <10 days/month or <120 days/year | 0 | 25+19<2 | | | |
| | 19 | ≥3 months | 1 | | | | |
| | | <3months | 0 | | | | |
| C | 35b | <15 minutes | 0 | | | 0 | |
| | | >4 hours | 0 | | | 0 | |
| | | ≥15 minutes and ≤4 hours | 1 | | | 1 | |
| D | 57 | Yes | 1 | 57+58+59+60+61+63+64+65+66+67+ 69≥1 | | 0 | |
| | | No | 0 | | | | |
| | 58 | Yes | 1 | | | | |
| | | No | 0 | | | | |
| | 59 | Yes | 1 | | | | |
| | | No | 0 | | | | |
| | 60 | Yes | 1 | | | | |
| | | No | 0 | | | | |
| | 61 | Yes | 1 | | | | |
| | | No | 0 | 57+58+59+60+61+63+64+65+66+67+ 69=0 | | 1 | |
| | 63 | Yes | 1 | | | | |
| | | No | 0 | | | | |
| | 64 | Yes | 1 | | | | |
| | | No | 0 | | | | |
| | 65 | Yes | 1 | | | | |
| | | No | 0 | | | | |
| | 66 | Yes | 1 | | | | |
| | | No | 0 | | | | |
| | 67 | Yes | 1 | | | | |
| | | No | 0 | | | | |
| | 69 | Yes | 1 | | | | |
| | | No | 0 | | | | |
| E | | | | | | A+B+C+D =4 | Hypnic Headache |
| | | | | | | | After making this diagnosis, Go to algorithm 12 |
| | | | | | | A=1 AND B+C+D=2 | Probable Hypnic Headache |
| | | | | | | | After making this diagnosis, Go to algorithm 12 |
| | | | | | | If above not satisfied, go to algorithm 12 | |

### Algorithm 12: Valsalva-Manoeuvre Headache

| Algorithm | Question | Response | Value | Calculate 1^{st} intermediate score (FIS) | Calculate 2^{nd} intermediate score (SIS) | Final score | Diagnosis |
|---|---|---|---|---|---|---|---|
| A | 110 | Yes | 1 | | | | |
| | | No | 0 | 109+35b=2 | | 1 | |
| | 35b | <1 second | 0 | | | | |
| | | >2 hours | 0 | 109+35b<2 | | 0 | |
| | | ≥1 second and ≤2 hours | 1 | | | | |
| B | | | | | | A=1 | Valsalva-Manoeuvre Headache |
| | | | | | | | After making this diagnosis, Go to algorithm 13 |
| | | | | | | A=0 | |
| | | | | | | Go to algorithm 13 | |

### Algorithm 13: Exercise Headache

| Algorithm | Question | Response | Value | Calculate 1^{st} intermediate score (FIS) | Calculate 2^{nd} intermediate score (SIS) | Final score | Diagnosis |
|---|---|---|---|---|---|---|---|
| A | 111 | Yes | 1 | | | | |
| | | No | 0 | 111+35b=2 | | 1 0 | |
| | 35b | <48 hours | 1 | 111+35b<2 | | | |
| | | ≥48 hours | 0 | | | | |
| B | | | | | | A=1 | Exercise Headache |
| | | | | | | | After making this diagnosis, Go to algorithm 14 |
| | | | | | | A=0 Go to algorithm 14 | |

### Algorithm 14: Sex Headache

| Algorit hm | Questio n | Response | Value | Calculate 1^{st} intermediate score (FIS) | Calculate 2^{nd} intermediate score (SIS) | Final score | Diagnosis |
|---|---|---|---|---|---|---|---|
| A | 112 | Yes | 1 | | | | |
| | | No | 0 | 1 12+35b=2 | | 1 | |
| | 35b | <1 minute | 0 | | | | |
| | | >72 hours | 0 | 1 12+35b<2 | | 0 | |
| | | ≥1 minute and ≤72 hours | 1 | | | | |
| B | | | | | | A=1 | Sex Headache |
| | | | | | | | After making this diagnosis, Go to algorithm 15 |
| | | | | | | A=0 | |
| | | | | | | Go to algorithm 15 | |

### Algorithm 15: Trigeminal neuralgia

| Algorit hm | Quest ion | Response | Value | Calculate 1^{st} intermediate score (FIS) | Calculate 2^{nd} intermediate score (SIS) | Final score | Diagnosis |
|---|---|---|---|---|---|---|---|
| A | 45 | Right side only | 1 | | | 1 | |
| | | Left side only | 1 | | | 1 | |
| | | At any one time, the pain is only on one side of the head or face but alternates between the right and left sides | 1 | | | 1 | |
| | | The pain is sometimes on one side of the head or face and at other times on both sides of the head or face at the same time | 0 | | | 0 | |
| | | The pain is always on both sides of the head or face | 0 | | | 0 | |
| B | 46 | Behind the ear AND back of the head AND neck not ticked | 1 | | | 1 | |
| | | Behind the ear and/or back of the head and/or neck not ticked | 0 | | | 0 | |
| C | 35b | ≤2 minutes | 1 | | | | |
| | | >2 minutes | 0 | | | | |
| | 42 | Mild | 0 | | | | |
| | | Moderate | 0 | | | | |
| | | Severe | 1 | | | | |
| | | Very severe | 1 | | | | |
| | 44 | Electric-shock like and/or shooting and/or stabbing and/or sharp | 1 | | | | |
| | | Electric-shock like AND shooting AND stabbing AND sharp not ticked | 0 | 35b+42+44+101≥3 | | 1 | |
| | | | | 35b+42+44+101<3 | | 0 | |
| | 101 | Touching your face or head and/or Squeezing your eyelids and/or Eating or chewing and/or Talking and/or Yawning or opening the jaw widely and/or Swallowing and/or Wind blowing on the face and/or Washing your face and/or Washing or brushing your hair and/or Having a shower and/or Shaving ticked | 1 | | | | |
| | | Touching your face or head AND Squeezing your eyelids AND "Eating or chewing" AND Talking AND "Yawning or opening the jaw widely" AND Swallowing AND Wind blowing on the face AND Washing your face AND "Washing or brushing your hair" AND Having a shower AND Shaving not ticked | 0 | | | | |
| D | | | | | | A+B+C =3 | Trigeminal Neuralgia |
| | | | | | | | After making this diagnosis, Go to algorithm 16 |
| | | | | | | A+B+C <3 | |
| | | | | | | Go to algorith m 16 | |

### Algorithm 16: Primary Stabbing Headache

| Algor ithm | Questi on | Response | Value | Calculate 1^{st} intermediate score (FIS) | Calculate 2^{nd} intermediate score (SIS) | Final score | Diagnosis |
|---|---|---|---|---|---|---|---|
| A | 35b | ≤2 minutes | 1 | | | 1 | |
| | | >2 minutes | 0 | | | 0 | |
| B | 101 | Touching your face or head and/or Squeezing your eyelids and/or Eating or chewing and/or Talking and/or Yawning or opening the jaw widely and/or Swallowing and/or Wind blowing on the face and/or Washing your face and/or Washing or brushing your hair and/or Having a shower and/or Shaving ticked | 0 | | | 0 | |
| | | Touching your face or head AND Squeezing your eyelids AND "Eating or chewing" AND Talking AND "Yawning or opening the jaw widely" AND Swallowing AND Wind blowing on the face AND Washing your face AND "Washing or brushing your hair" AND Having a shower AND Shaving not ticked | 1 | | | 1 | |
| | | | | | | | |
| C | 46 | Forehead and/or eye and/or temple and/or behind the temple and/or Top of the head and/or back of the head ticked | 1 | | | 1 | |
| | | Forehead AND eye AND temple AND behind the temple AND Top of the head AND back of the head not ticked | 0 | | | 0 | |
| D | 57 | Yes | 1 | | | 0 | |
| | | No | 0 | | | | |
| | 58 | Yes | 1 | | | | |
| | | No | 0 | | | | |
| | 59 | Yes | 1 | | | | |
| | | No | 0 | | | | |
| | 60 | Yes | 1 | 57+58+59+60+61+63+6 4+65+66+67≥1 | | | |
| | | No | 0 | | | | |
| | 61 | Yes | 1 | 57+58+59+60+61+63+6 4+65+66+67=0 | | 1 | |
| | | No | 0 | | | | |
| | 63 | Yes | 1 | | | | |
| | | No | 0 | | | | |
| | 64 | Yes | 1 | | | | |
| | | No | 0 | | | | |
| | 65 | Yes | 1 | | | | |
| | | No | 0 | | | | |
| | 66 | Yes | 1 | | | | |
| | | No | 0 | | | | |
| | 67 | Yes | 1 | | | | |
| | | No | 0 | | | | |
| | | | | | | | |
| E | 44 | Electric-shock like and/or shooting and/or stabbing and/or sharp | 1 | | | 1 | |
| | | Electric-shock like AND shooting AND stabbing AND sharp not ticked | 0 | | | 0 | |
| F | | | | | | A+B+C+ D+E=5 | Primary Stabbing Headache |
| | | | | | | | After making this diagnosis, Go to algorithm 17 |
| | | | | | | A+B+C+ D+E<5 | |
| | | | | | | Go to algorith m 17 | |

### Algorithm 17: High CSF Pressure

| Algorit hm | Quest ion | Response | Value | Calculate 1^{st} intermediate score (FIS) | Calculate 2^{nd} intermediat e score (SIS) | Final score | Diagnosis |
|---|---|---|---|---|---|---|---|
| A | 114 | Sleep | 1 | | | 1 | |
| | | Awake | 0 | | | 0 | |
| | | Equally likely during both awake and sleep state | 0 | | | 0 | |
| B | 108 | No effect | 0 | | | 0 | |
| | | The pain worsens significantly | 1 | | | 1 | |
| | | The pain improves relatively quickly | 0 | | | 0 | |
| | | I prefer to lie down but the pain does not improve overall | 0 | | | 0 | |
| C | 109 | No effect | 0 | | | 0 | |
| | | The pain worsens significantly | 0 | | | 0 | |
| | | The pain improves relatively quickly | 1 | | | 1 | |
| | | I prefer to lie down but the pain does not improve overall | 0 | | | 0 | |
| D | 110 | Yes | 1 | | | 1 | |
| | | No | 0 | | | 0 | |
| E | 81 | Temporary loss of vision on one side or both sides ticked | 1 | | | 1 | |
| | | Temporary loss of vision on one side or both sides not ticked | 0 | | | 0 | |
| F | 77 | Yes | 1 | | | 1 | |
| | | No | 0 | | | 0 | |
| G | | | | | | A+B+C+D+E +F≥4 | Cerebrospinal |
| | | | | | | | Fluid (CSF) |
| | | | | | | | Pressure |
| | | | | | | | Dysregulation |
| | | | | | | | After making this diagnosis, Go to algorithm 18 |
| | | | | | | A+B+C+D+E +F<4 | |
| | | | | | | Go to algorithm 18 | |

### Algorithm 18: Low CSF Pressure

| Algorithm | Question | Response | Value | Calculate 1^{st} intermediate score (FIS) | Calculate 2^{nd} intermediate score (SIS) | Final score | Diagnosis |
|---|---|---|---|---|---|---|---|
| A | 108 | No effect | 0 | | | 0 | |
| | | The pain worsens significantly | 0 | | | 0 | |
| | | The pain improves relatively quickly | 1 | | | 1 | |
| | | I prefer to lie down but the pain does not improve overall | 0 | | | 0 | |
| B | 109 | No effect | 0 | | | 0 | |
| | | The pain worsens significantly | 1 | | | 1 | |
| | | The pain improves relatively quickly | 0 | | | 0 | |
| | | I prefer to lie down but the pain does not improve overall | 0 | | | 0 | |
| C | | | | | | A+B=2 | Low |
| | | | | | | | Cerebrospinal |
| | | | | | | | Fluid (CSF) |
| | | | | | | | Pressure |
| | | | | | | | Headache |
| | | | | | | | After making this diagnosis, Go to algorithm 19 |
| | | | | | | A+B<2 Go to algorithm 19 | |

### Algorithm 19: Cervicogenic Headache

| Algorithm | Question | Response | Value | Calculate 1^{st} intermediate score (FIS) | Calculate 2^{nd} intermediate score (SIS) | Final score | Diagnosis |
|---|---|---|---|---|---|---|---|
| A | 102 | Yes | 1 | | | 1 | |
| | | No | 0 | | | 0 | |
| B | 103 | Yes | 1 | | | 1 | |
| | | No | 0 | | | 0 | |
| | | No response | 0 | | | 0 | |
| C | 105 | Neck stiffness/pain only occurs with headaches/facial pain | 1 | | | 1 | |
| | | Neck stiffness/pain occurs with headaches but also occurs when there is no headache/facial pain | 1 | | | 1 | |
| | | Neck stiffness/pain occurs independently of headaches/facial pain | 0 | | | 0 | |
| | | No response | 0 | | | 0 | |
| D | 106 | Yes | 1 | | | 1 | |
| | | No | 0 | | | 0 | |
| | | No response | 0 | | | 0 | |
| E | 107 | Yes | 1 | | | 1 | |
| | | No | 0 | | | 0 | |
| | | No response | 0 | | | 0 | |
| | | | | | | | |
| F | | | | | | A+B+C +D+E≥ 3 | Cervicogenic Headache |
| | | | | | | | After making this diagnosis, Go to algorithm 20 |
| | | | | | | A+B+C +D+E< 3 | |
| | | | | | | Go to algorith m 20 | |

### Algorithm 20: Medication Overuse Headache

Criteria for Running Algorithm on Medication Overuse Headache
User already has one of the following diagnosis:
Episodic Migraine
Chronic Migraine
Episodic Tension-Type headache
Chronic Tension-Type headache
Probable Migraine
Probable Tension-Type Headache
New Daily Persistent Headache
Hemicrania Continua

| Algorithm | Question | Response | Value | Calculate 1^{st} intermediate score (FIS) | Calculate 2^{nd} intermediate score (SIS) | Final score | Diagnosis |
|---|---|---|---|---|---|---|---|
| A | 122 | ≥10 days/month | 1 | | | 1 | |
| | | <10 days/month | 0 | | | 0 | |
| B | 123 | ≥3 months | 1 | | | 1 | |
| | | <3 months | 0 | | | 0 | |
| | | No response | 0 | | | 0 | |
| C | | | | | | A+B=2 | Medication |
| | | | | | | | Overuse |
| | | | | | | | Headache |
| | | | | | | A+B<2 | No Medication |
| | | | | | | | Overuse |
| | | | | | | | Headache |

| | | | | | | | |
|---|---|---|---|---|---|---|---|
| Notes The final diagnoses are in Bold in the Diagnosis column; the comments in regular font in the diagnosis column are not to be included as diagnosis All the possible diagnoses generated by the whole algorithm for a particular headache type will need to be displayed in "Summary" on Headache Website document Pg 42 Patient can only have one of the following diagnoses (Algorithm has been designed to identify one of this then to start looking for other possible diagnoses) Episodic Migraine Chronic Migraine Episodic Tension-Type headache Chronic Tension-Type headache Probable Migraine Probable Tension-Type Headache | | | | | | | |

## Claims

1. A system for diagnosing and treating headaches, including:
a generic characterisation module operable to log a plurality of characteristics relating to a patient (32) including personal details, generic headache factors including frequency and symptoms, possible triggers and relieving factors, previous treatments and medications, family history, and social factors, the generic characterisation module being non-specific of headache type and accessible outside of a headache event;
a differential diagnosis module including an algorithm for determining on the basis of a combination of a plurality of the characteristics input into the generic characterisation module a differential diagnosis of headache type, wherein in a first stage the algorithm applies a scoring to characteristics input into the generic characterisation module and generates values indicative of whether or not each of a plurality of headache types is exhibited by the patient, and in a second stage the algorithm receives from a clinician confirmation or alternative diagnosis from among the plurality of headache types;
a plurality of type specific log modules each operable to log and analyse a plurality of parameters relating to an associated headache type during the course of a headache, the parameters relating to one or more of headache symptoms, headache triggers, headache timing, or treatment taken;
at least one output module operable to output the log and/or analysis of each type specific log module, wherein at least the type specific log modules and the output module are included in a patient device (14; 16) the patient device including a graphical user input for inputting data into the type specific log modules,
wherein the system includes a database (24) of patient data and of diagnoses from the clinician relating to a plurality of patients (32), and a processing module operable to identify in the database patient data and diagnoses relating to other patients having at least one characteristic consistent with those of the patient and to reconfigure the algorithm on the basis of correlation with the stored patient data and diagnoses acquired over time.

2. A system according to claim 1, wherein the patient device is a computerised patient device, including a computer (14), a tablet (16) or a smart telephone (16).

3. A system according to claim 1 or 2, wherein the generic characterisation module and/or the differential diagnosis module are included in the patient device (14, 16).

4. A system according to claim 1, 2 or 3, wherein the at least one output module is operable to provide a patient indication of one or more of:
(i) headache history;
(ii) headache frequency;
(iii) treatment efficacy;
(iv) trigger frequency.

5. A system according to any preceding claim, wherein at least one of the patient indications is in graphical form, comprising:
a bar and/or
a pie chart, and/or
a graphical display of a person's head, subdivided into head and facial zones.

6. A system according to any preceding claim, wherein the patient indication is limited over a time period, optionally patient adjustable.

7. A system according to any preceding claim, wherein the system includes at least one sensing device (18) operable to sense one or more patient physiological parameters including at least one of:
(i) number of steps walked,
(ii) steps climbed,
(iii) distance travelled,
(iv) calories burned,
(v) active time,
(vi) heart rate,
(vii) sleep time,
(viii) sleep quality,
(ix) levels of phone use,
(x) sound levels, and
(xi) proportion of time spent indoors versus outdoors,
(xii) blood pressure,
(xiii) breath rate,
(xiv) mood sensor,
(xv) fatigue sensor,
(xvi) oxygen levels,
(xvii) blood sugar levels,
(xvii) temperature,
(xviii) alcohol levels in blood.

8. A system according to claim 6 or 7, wherein the sensing device (18) and type specific log modules are configured to communicate with one another automatically.

9. A system according to any preceding claim, wherein the system includes a generic headache log module for logging and processing data relating to a non-specific headache type.

10. A system according to any preceding claim, wherein the system includes a diary log module allowing a patient to input headache symptoms on time period basis.

11. A system according to any preceding claim, wherein the system includes provision for a patient to select one or more log modules for inputting and analysing headache symptoms.

12. A system according to any preceding claim, wherein the graphical user input includes at least a graphical display of a person's head, subdivided into head and facial zones.

## Patentansprüche

1. System zum Diagnostizieren und Behandeln von Kopfschmerzen, aufweisend:
ein Modul zur generischen Charakterisierung, das dazu befähigt ist, mehrere Merkmale zu protokollieren, die sich auf einen Patienten (32) beziehen, einschließlich persönliche Angaben, allgemeine Kopfschmerzfaktoren, aufweisend Häufigkeit und Symptome, mögliche Auslöser und lindernde Faktoren, frühere Behandlungen und Medikationen, Familienanamnese und soziale Faktoren, wobei das Modul zur generischen Charakterisierung nicht spezifisch für einen Kopfschmerztyp ist und außerhalb eines Kopfschmerzereignisses zugänglich ist;
ein Differenzialdiagnosemodul, das einen Algorithmus zum Bestimmen einer Differenzialdiagnose des Kopfschmerztyps auf der Grundlage einer Kombination der in das Modul zur generischen Charakterisierung eingegebenen Merkmale aufweist, wobei der Algorithmus in einer ersten Stufe eine Bewertung auf die in das Modul zur generischen Charakterisierung eingegebenen Merkmale anwendet und Werte generiert, die anzeigen, ob der Patient jeweils einen von mehreren Kopfschmerztypen aufweist oder nicht, und wobei der Algorithmus in einer zweiten Stufe von einem Kliniker eine Bestätigung oder eine alternative Diagnose aus den mehreren Kopfschmerztypen empfängt;
mehrere Module zur typspezifischen Protokollierung, die jeweils dazu befähigt sind, um mehrere Parameter, die während des Verlaufs eines Kopfschmerzes einen zugeordneten Kopfschmerztyp betreffen, zu protokollieren und zu analysieren, wobei die Parameter einen oder mehrere der folgenden Aspekte betreffen: Kopfschmerzsymptome, Kopfschmerzauslöser, Kopfschmerzzeitpunkt oder durchgeführte Behandlung;
mindestens ein Ausgabemodul, das dazu befähigt ist, die Protokollierung und/oder Analyse jedes Moduls zur typspezifischen Protokollierung auszugeben, wobei zumindest die Module zur typspezifischen Protokollierung und das Ausgabemodul in einer Patientenvorrichtung (14; 16) enthalten sind, wobei die Patientenvorrichtung eine grafische Benutzereingabe zum Eingeben von Daten in die typspezifischen Module zur typspezifischen Protokollierung umfasst,
wobei das System eine Datenbank (24) mit Patientendaten und mit Diagnosen von dem Kliniker, die mehrere Patienten (32) betreffen, sowie ein Verarbeitungsmodul umfasst, das dazu befähigt ist, in der Datenbank Patientendaten und Diagnosen zu identifizieren, die andere Patienten betreffen, die mindestens ein Merkmal aufweisen, das mit denjenigen des Patienten übereinstimmt, und den Algorithmus auf der Grundlage einer Korrelation mit den gespeicherten Patientendaten und im Laufe der Zeit erfassten Diagnosen gemäß neu zu konfigurieren.

2. System gemäß Anspruch 1, wobei die Patientenvorrichtung eine computergestützte Patientenvorrichtung ist, umfassend einen Computer (14), ein Tablet (16) oder ein Smartphone (16).

3. System gemäß Anspruch 1 oder 2, wobei das Modul zur generischen Charakterisierung und/oder das Differenzialdiagnosemodul in der Patientenvorrichtung (14, 16) enthalten sind.

4. System gemäß Anspruch 1, 2 oder 3, wobei das mindestens eine Ausgabemodul dazu befähigt ist, eine Patientenangabe zu einem oder mehreren der folgenden Aspekte bereitzustellen:
(i) Kopfschmerzanamnese;
(ii) Kopfschmerzfrequenz;
(iii) Behandlungswirksamkeit;
(iv) Auslösefrequenz.

5. System gemäß einem der vorangehenden Ansprüche, wobei mindestens eine der Patientenangaben in grafischer Form vorliegt, aufweisend:
ein Balken- und/oder
ein Tortendiagramm und/oder
eine grafische Darstellung eines menschlichen Kopfes, unterteilt in Kopf- und Gesichtszonen.

6. System gemäß einem der vorangehenden Ansprüche, wobei die Patientenangabe über einen Zeitraum begrenzt ist, optional durch den Patienten einstellbar.

7. System gemäß einem der vorangehenden Ansprüche, wobei das System mindestens eine Erfassungsvorrichtung (18) umfasst, die dazu befähigt ist, einen oder mehrere physiologische Parameter des Patienten zu erfassen, umfassend mindestens einen der folgenden:
(i) Anzahl der gegangenen Schritte,
(ii) erklommene Treppenstufen,
(iii) zurückgelegte Strecke,
(iv) verbrannte Kalorien,
(v) aktive Zeit,
(vi) Herzfrequenz,
(vii) Schlafdauer,
(viii) Schlafqualität,
(ix) Niveaus der Nutzung eines Telefons,
(x) Geräuschpegel, und
(xi) Anteil der Zeit, die drinnen verbracht wird und gegenüber der Zeit, die draußen verbracht wird,
(xii) Blutdruck,
(xiii) Atemfrequenz,
(xiv) Stimmungssensor,
(xv) Ermüdungssensor,
(xvi) Sauerstoffgehalte,
(xvii) Blutzuckerspiegel,
(xvii) Temperatur,
(xviii) Alkoholgehalte im Blut.

8. System gemäß Anspruch 6 oder 7, wobei die Erfassungsvorrichtung (18) und die Module zur typspezifischen Protokollierung dazu konfiguriert sind, automatisch miteinander zu kommunizieren.

9. System gemäß einem der vorangehenden Ansprüche, wobei das System ein generisches Kopfschmerz-Protokollierungsmodul zum Protokollieren und Verarbeiten von Daten, die einen nicht spezifischen Kopfschmerztyp betreffen, umfasst.

10. System gemäß einem der vorangehenden Ansprüche, wobei das System ein Tagebuch-Protokollierungsmodul umfasst, das es einem Patienten ermöglicht, Kopfschmerzsymptome auf Zeitraumbasis einzugeben.

11. System gemäß einem der vorangehenden Ansprüche, wobei das System eine Einrichtung für einen Patienten zum Auswählen eines oder mehrere Protokollierungsmodule zum Eingeben und Analysieren von Kopfschmerzsymptomen umfasst.

12. System gemäß einem der vorangehenden Ansprüche, wobei die grafische Benutzereingabe mindestens eine grafische Darstellung eines menschlichen Kopfes umfasst, unterteilt in Kopf- und Gesichtszonen.

## Revendications

1. - Système de diagnostic et de traitement de maux de tête, comprenant :
un module de caractérisation générique fonctionnel pour journaliser une pluralité de caractéristiques relatives à un patient (32), comprenant des détails personnels, des facteurs génériques de mal de tête comprenant la fréquence et les symptômes, des facteurs déclencheurs et de soulagement possibles, des traitements et médicaments antérieurs, des antécédents familiaux et des facteurs sociaux, ledit module de caractérisation générique étant non spécifique à un type de mal de tête et étant accessible en dehors d'un épisode de mal de tête ;
un module de diagnostic différentiel comprenant un algorithme pour déterminer, sur la base d'une combinaison d'une pluralité des caractéristiques entrées dans le module de caractérisation générique, un diagnostic différentiel du type de mal de tête, où, dans une première étape, l'algorithme applique une notation aux caractéristiques entrées dans le module de caractérisation générique et génère des valeurs indiquant si chacun d'une pluralité de types de mal de tête est présent ou non chez le patient, et dans une seconde étape, l'algorithme reçoit une confirmation ou un diagnostic alternatif de clinicien parmi la pluralité de types de mal de tête ;
une pluralité de modules de journalisation spécifiques au type, chacun fonctionnel pour journaliser et analyser une pluralité de paramètres relatifs à un type de mal de tête associé au cours de l'évolution d'un mal de tête, les paramètres se rapportant à un ou plusieurs parmi les symptômes du mal de tête, les facteurs déclencheurs du mal de tête, la temporisation du mal de tête ou le traitement suivi ;
au moins un module de sortie fonctionnel pour délivrer le journal et/ou l'analyse de chaque module de journalisation spécifique au type, au moins les modules de journalisation spécifiques au type et le module de sortie étant inclus dans un dispositif de patient (14 ; 16), le dispositif de patient comprenant une entrée d'utilisateur graphique pour entrer des données dans les modules de journalisation spécifiques au type,
dans lequel le système comprend une base de données (24) de données de patient et de diagnostics établis par le clinicien concernant une pluralité de patients (32), et un module de traitement fonctionnel pour identifier, dans la base de données, des données de patient et des diagnostics relatifs à d'autres patients présentant au moins une caractéristique concordant avec celles du patient et pour reconfigurer l'algorithme sur la base d'une corrélation avec les données de patient et les diagnostics stockés acquis au fil du temps.

2. - Système selon la revendication 1, dans lequel le dispositif de patient est un dispositif de patient informatisé, comprenant un ordinateur (14), une tablette (16) ou un téléphone intelligent (16).

3. - Système selon la revendication 1 ou 2, dans lequel le module de caractérisation générique et/ou le module de diagnostic différentiel sont inclus dans le dispositif de patient (14, 16).

4. - Système selon la revendication 1, 2 ou 3, dans lequel l'au moins un module de sortie est fonctionnel pour fournir une indication de patient concernant une ou plusieurs parmi :
(i) les antécédents de mal de tête ;
(ii) la fréquence de mal de tête ;
(iii) l'efficacité du traitement ;
(iv) la fréquence des facteurs déclencheurs.

5. - Système selon l'une quelconque des revendications précédentes, dans lequel au moins l'une des indications de patient se présente sous forme graphique, comprenant :
un diagramme en barre et/ou
un diagramme circulaire, et/ou
une représentation graphique de la tête d'une personne, subdivisée en zones de la tête et du visage.

6. - Système selon l'une quelconque des revendications précédentes, dans lequel l'indication de patient est limitée à une période de temps, facultativement réglable par le patient.

7. - Système selon l'une quelconque des revendications précédentes, dans lequel le système comprend au moins un dispositif de détection (18) fonctionnel pour détecter un ou plusieurs paramètres physiologiques de patient comprenant au moins l'un parmi :
(i) le nombre de pas effectués,
(ii) les marches montées,
(iii) la distance parcourue,
(iv) les calories brûlées,
(v) le temps d'activité,
(vi) la fréquence cardiaque,
(vii) le temps de sommeil,
(viii) la qualité du sommeil,
(ix) le niveau d'utilisation du téléphone,
(x) les niveaux sonores, et
(xi) la proportion de temps passé à l'intérieur par rapport à l'extérieur,
(xii) la pression artérielle,
(xiii) la fréquence respiratoire,
(xiv) le capteur d'humeur,
(xv) le capteur de fatigue,
(xvi) les taux d'oxygène,
(xvii) les taux de glycémie,
(xvii) la température,
(xviii) les taux d'alcool dans le sang.

8. - Système selon la revendication 6 ou 7, dans lequel le dispositif de détection (18) et les modules de journalisation spécifiques au type sont configurés pour communiquer automatiquement entre eux.

9. - Système selon l'une quelconque des revendications précédentes, dans lequel le système comprend un module de journalisation générique de mal de tête pour journaliser et traiter des données relatives à un type de mal de tête non spécifique.

10. - Système selon l'une quelconque des revendications précédentes, dans lequel le système comprend un module de journal de bord permettant à un patient d'entrer les symptômes de mal de tête sur une base de période de temps.

11. - Système selon l'une quelconque des revendications précédentes, dans lequel le système comprend la possibilité pour un patient de sélectionner un ou plusieurs modules de journalisation pour entrer et analyser les symptômes de mal de tête.

12. - Système selon l'une quelconque des revendications précédentes, dans lequel l'entrée d'utilisateur graphique comprend au moins un affichage graphique de la tête d'une personne, subdivisée en zones de la tête et du visage.
